(19) Europäisches Patentamt
European Patent Office
Office européen des brevets

(11) Publication number : 0 655 439 A2

# (12) EUROPEAN PATENT APPLICATION

(21) Application number : 94308232.1

(22) Date of filing : 09.11.94

(51) Int. Cl.[6] : **C07D 209/18**, C07D 209/14, C07D 307/16, A61K 31/405, A61K 31/34

(30) Priority : 12.11.93 US 151087

(43) Date of publication of application : 31.05.95 Bulletin 95/22

(84) Designated Contracting States : AT BE CH DE DK ES FR GB GR IE IT LI LU NL PT SE

(71) Applicant : ELI LILLY AND COMPANY
Lilly Corporate Center
Indianapolis Indiana 46285 (US)

(72) Inventor : Denney, Michael L.
Rural Route 2,
Box 135A2
Franklin, Indiana 46131 (US)
Inventor : Fisher, Matthew Joseph
4106 Armon Court
Carmel, Indiana 46033 (US)
Inventor : Happ, Anne Marie
5734 Broadway Street
Indianapolis, Indiana 46220 (US)
Inventor : Jakubowski, Joseph Anthony
3740 Governors Road
Indianapolis, Indiana 46208 (US)
Inventor : Kinnick, Michael Dean
343 Southcreek Drive
South Indianapolis, Indiana 46217 (US)
Inventor : McCowan, Jefferson Ray
2653 Crescent Hill Lane
Indianapolis, Indiana 46208 (US)
Inventor : Morin, John Michael, Jr.
9 Roselawn Avenue
Brownsburg, Indiana 46112 (US)
Inventor : Sall, Daniel Jon
376 Leisure Lane
Greenwood, Indiana 46142 (US)
Inventor : Vasileff, Robert Theodore
4651 Cherry Lane
Indianapolis, Indiana 46208 (US)

(74) Representative : Tapping, Kenneth George et al
Lilly Industries Limited
European Patent Operations
Erl Wood Manor
Windlesham Surrey GU20 6PH (GB)

## (54) 5,6-Bicyclic glycoprotein IIb IIIa antagonists useful in inhibition of platelet aggregation.

(57) This invention relates to 5,6 fused ring bicyclic compounds inclusive of indoles, benzofurans, and benzothiophenes, and corresponding to the formula (I)

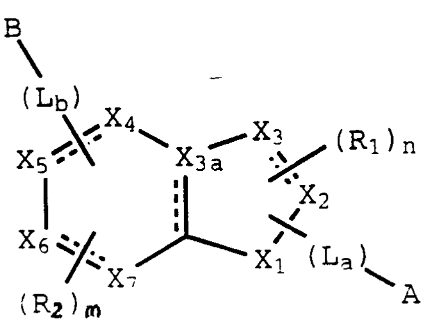

(I)

substituted with both basic (B) and acidic (A) functionality, which are useful in inhibition of platelet aggregation.

EP 0 655 439 A2

## Field of the Invention

This invention relates to 5,6 fused bicyclic ring compounds useful as glycoprotein IIb/IIIa antagonists for the prevention of thrombosis.

## Background of the Invention

The most prevalent ischemic arterial heart disease states are related to platelet dependent narrowing of the blood supply such as atherosclerosis and arteriosclerosis, acute myocardial infarction, chronic stable angina, unstable angina, transient ischemic attacks and strokes, peripheral vascular disease, arterial thrombosis, embolism, restenosis following angioplasty, carotid endarterectomy, anastomosis of vascular grafts, and etc. These conditions represent a variety of disorders thought to be initiated by platelet activation on vessel walls.

Platelet adhesion and aggregation is believed to be an important part of thrombus formation. This activity is mediated by a number of platelet adhesive glycoproteins. The binding sites for fibrinogen, fibronectin and other factors have been located on the platelet membrane glycoprotein complex IIb-IIIa. When platelets are activated by an agonist such as thrombin, the GPIIb-IIIa binding site becomes available to fibrinogen, leading to platelet aggregation eventually resulting in clot formation.

Heretofore it has been proposed to block these fibrinogen binding sites by the use of various therapeutic agents. For example, U.S. Patent No. 5,250,679 describes bicyclic nonpeptidyl platelet aggregation inhibitors having specificity for the GP IIb-IIIa receptor.

There is a need in the area of cardiovascular and cerebrovascular therapeutics for alternative agents to those currently in use for prevention and treatment of thrombi.

It is a discovery of this invention that certain novel bicyclic compounds block the GPIIb-IIIa fibrinogen receptor, thereby inhibiting platelet aggregation and subsequent thrombus formation. Pharmaceutical formulations containing the bicyclic compounds of this invention inhibit aggregation and are useful for the prophylaxis and treatment of thrombogenic diseases, such as myocardial infarction, stroke, and peripheral arterial disease.

## Summary of the Invention

The present invention relates to novel 5,6 bicyclic fused ring systems functionalized with both an acidic and basic component that serve as antagonists of the GPIIb/IIIa receptor where said compounds are represented by the formula (I), and all pharmaceutically acceptable salts, solvates, and prodrug derivatives thereof:

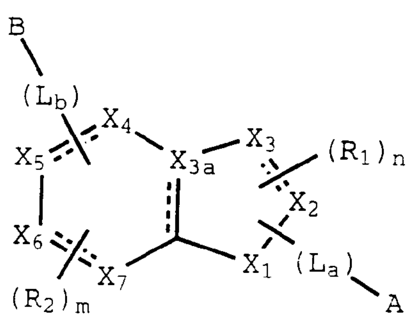

(I)

wherein:

$X_1$, $X_2$, and $X_3$ of the 5 membered ring are independently selected from carbon, nitrogen, sulfur, and oxygen, with the proviso that at least one of $X_1$, $X_2$, and $X_3$ is carbon;

$X_4$, $X_5$, $X_6$, and $X_7$ are independently selected from carbon, nitrogen, sulfur, and oxygen, with the proviso that at least two of $X_4$, $X_5$, $X_6$, and $X_7$ are carbon;

$X_{3a}$, the bridging atom, is selected from the group consisting of carbon and nitrogen;

B is a basic group linked to the six membered ring by linking group -($L_b$)-, where -($L_b$)- is;

(i) a bond, or

(ii) a divalent substituted or unsubstituted chain of from 1 to 15 atoms;

A is an acidic group linked to the five membered ring by linking group -($L_a$)-, where -($L_a$) is;

(i) a bond, or

(ii) a divalent substituted or unsubstituted chain of from 1 to 15 atoms;

n is an integer selected from 1 to 5;

$(R_1)_n$ are organic radicals attached to one or more of the atoms $X_1$, $X_2$, and $X_3$ of the five membered ring and where $R_1$ are independently selected from A, A-$(L_a)$-, hydrogen, alkyl, halosubstituted alkyl, hydroxylalkyl, alkenyl, alkynyl, cycloalkyl, aryl, aryloxy, aralkyl, hydroxy, alkoxy, aralkoxy, carbamyl, carboxy, acyl, cyano, halo, nitro, sulfo, and when any of $X_1$, $X_2$, and $X_3$ have two sites for substitution, then $R_1$ may also be =O;

m is an integer selected from 1 to 7;

$(R_2)_m$ are organic radicals attached to one or more of the atoms $X_4$, $X_5$, $X_6$, and $X_7$ of the six membered ring and where $R_2$ are independently selected from B, B-$(L_b)$-, hydrogen, alkyl, hydroxyalkyl, halosubstituted alkyl, alkenyl, alkynyl, cycloalkyl, aryl, aryloxy, aralkyl, hydroxy, alkoxy, aralkoxy, carbamyl, amino, substituted amino, acyl, cyano, halo, nitro, sulfo, and when any of $X_4$, $X_6$, $X_6$, and $X_7$ have two sites for substitution, then $R_2$ may also be =O.

Another aspect of the invention are pharmaceutical formulations containing the novel 5,6 bicyclic compounds of the invention.

Another aspect of the invention is a method of preventing thrombosis by administering to a mammal the novel 5,6 bicyclic compounds of the invention.

**Detailed Description of the Invention**

Definitions:

The term "5,6 bicyclic compound", refers to a chemical compound having a nucleus formed from fused 6 and 5 membered rings as represented by the formula:

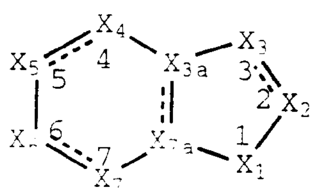

where the dashed lines indicate optional unsaturation. The term "alkyl" used herein refers to a monovalent straight or branched chain radical of from one to fifteen carbon atoms, including, but not limited to methyl, ethyl, n-propyl, isopropyl, n-butyl, isobutyl, tert-butyl, n-hexyl, and the like.

The term, "halosubstituted alkyl" as used herein refers to an alkyl group as just defined, substituted by one, two or three halogen atoms selected from fluorine, chlorine, bromine, and iodine. Examples of such groups include chloromethyl, bromoethyl, trifluoromethyl, and the like.

The term, "aryl" as used herein refers to an organic radical derived from an aromatic hydrocarbon by removal of one atom; e.g., phenyl, $C_{1-4}$ alkylphenyl, naphthyl, toluenyl, benzyl, and methylbenzoyl.

The term "alkenyl" as used herein refers to a monovalent straight or branched chain radical of from two to six carbon atoms containing a carbon to carbon double bond including, but not limited to, 1-propenyl, 2-propenyl, 2-methyl-1-propenyl, 1-butenyl, 2-butenyl and the like.

The term, "alkylene" as used herein refers to a divalent straight or branched chain group of from one to ten carbon atoms, including but not limited to, $-CH_2-$, $-(CH_2)_2-$, $-(CH_2)_3-$, $-CH(CH_3)-$, $-CH(C_2H_5)-$, $-CH(CH_3)CH_2-$, and the like.

The term "alkenylene" as used herein refers to a divalent straight or branched chain group of from two to ten carbon atoms containing a carbon-carbon double bond, including but not limited to, $-CH=CH-$, $-C(CH_3)=CH-$, $CH=CH-CH_2-$, $-CH=C(CH_3)-CH_2-$, $-CH_2CH(CH=CH_2)CH_2$, and the like.

The term, "alkynylene" as used herein refers to a divalent straight or branched chain group of from two to ten carbon atoms containing a carbon-carbon triple bond, including but not limited to,

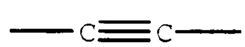 ,

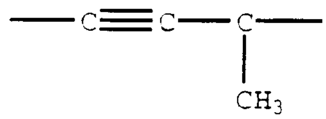

and the like.

The term, "amidino" refers to the radical having the structural formula;

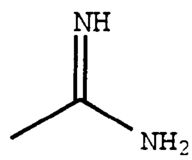

The term, "amino" refers to radicals derived from primary, secondary or tertiary amines.

The term, "bond" refers a linkage between atoms consisting of an electron pair.

The term, "acidic group" refers to an organic radical which is a proton donor.

The term, "basic group" refers to an organic radical which is a proton acceptor.

The words, "pharmaceutically acceptable salts" incluse both acid and base addition salts.

<u>Compounds of the Invention:</u>

Compounds of this invention have the general formula (I) shown below:

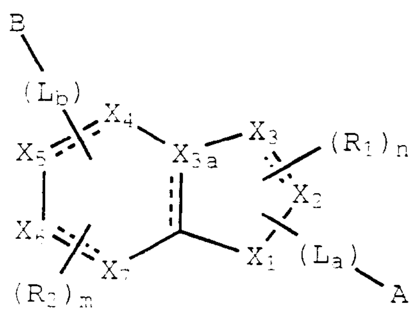

(I)

and all pharmaceutically acceptable salts, solvates and prodrug derivatives thereof.

wherein:

$X_1$, $X_2$, and $X_3$ of the 5 membered ring are independently selected from carbon, nitrogen, sulfur, and oxygen, with the proviso that at least one of $X_1$, $X_2$, and $X_3$ is carbon;

$X_4$, $X_5$, $X_6$, and $X_7$ are independently selected from carbon, nitrogen, sulfur, and oxygen, with the proviso that at least two of $X_4$, $X_5$, $X_6$, and $X_7$ are carbon;

$X_{3a}$, the bridging atom, is selected from the group consisting of carbon and nitrogen;

B is a basic group linked to the six membered ring by linking group -($L_b$)-,

where -($L_b$)- is;

(i) a bond, or

(ii) a divalent substituted or unsubstituted chain of from 1 to 15 atoms;

A is an acidic group linked to the five membered ring by linking group -($L_a$)-, where -($L_a$)- is;

(i) a bond, or

(ii) a divalent substituted or unsubstituted chain of from 1 to 15 atoms;

n is an integer selected from 1 to 5;

$(R_1)_n$ are organic radicals attached to one or more of the atoms $X_1$, $X_2$, and $X_3$ of the five membered ring, where each $R_1$ is independently selected from A, A-($L_a$)-, hydrogen, alkyl, halosubstituted alkyl, hydroxylalkyl, alkenyl, alkynyl, cycloalkyl, aryl, aryloxy, aralkyl, hydroxy, alkoxy, aralkoxy, carbamyl, carboxy, acyl, cyano, halo, nitro, sulfo, and when any of $X_1$, $X_2$, and $X_3$ have two sites for substitution, then $R_1$ may also be =O;

m is an integer selected from 1 to 7;

$(R_2)_m$ are organic radicals attached to one or more of the atoms $X_4$, $X_5$, $X_6$, and $X_7$ of the six membered ring, where each $R_2$ is independently selected from B, B-$(L_b)$-, hydrogen, alkyl, hydroxyalkyl, halosubstituted alkyl, alkenyl, alkynyl, cycloalkyl, aryl, aryloxy, aralkyl, hydroxy, alkoxy, aralkoxy, carbamyl, amino, substituted amino, acyl, cyano, halo, nitro, sulfo, and when any of $X_4$, $X_6$, $X_6$, and $X_7$ have two sites for substitution, then $R_2$ may also be =O.

The dashed lines in the structural formula (I) signify the optional presence of an additional bond, that is, unsaturation that will lend aromatic character to the ring structure. It will be understood that the bridging atoms will either be unsubstituted or substituted (with hydrogen) depending on the degree of unsaturation in the bicyclic ring system. Thus, the bicyclic nucleus of the compounds of the invention may be formed from ring systems inclusive of, but not limited to, any of the nuclei represented by the following forty structural formulae:

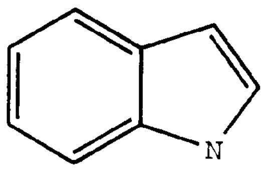 , 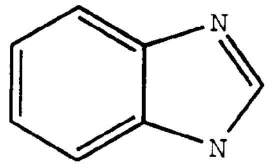 ,

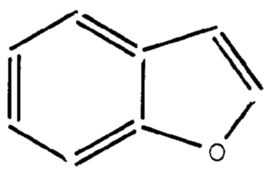 , 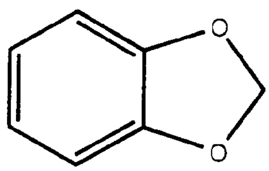 ,

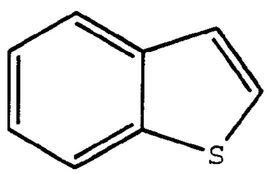 , 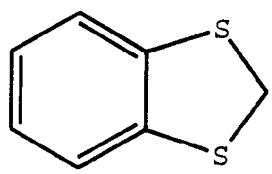 ,

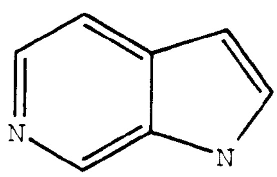 , 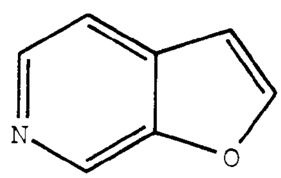 ,

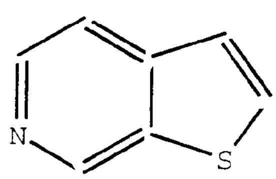 , 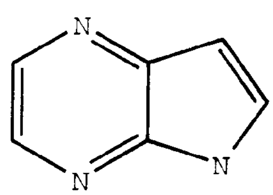 ,

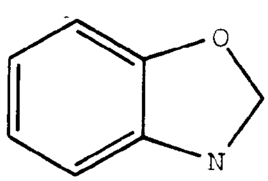 , 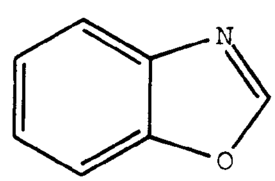 ,

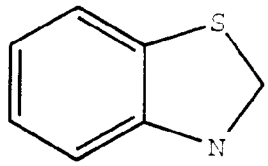 , 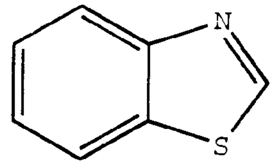 ,

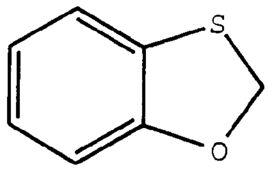 , 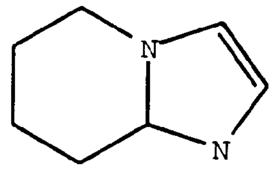 ,

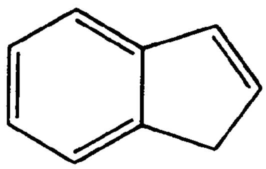 , 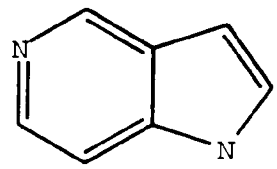 ,

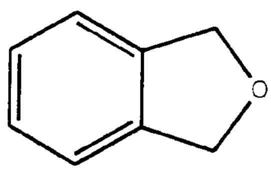 , 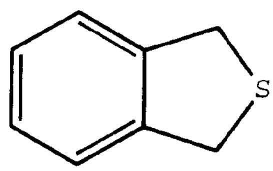 ,

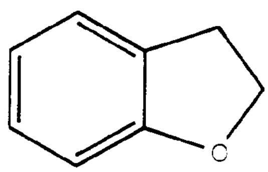 , 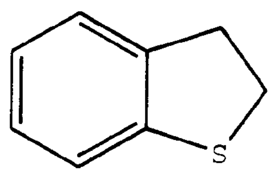 ,

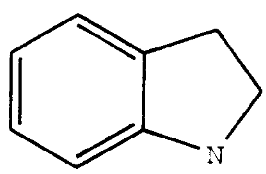 , 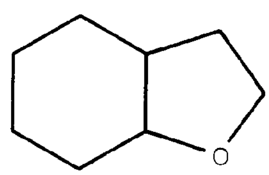 ,

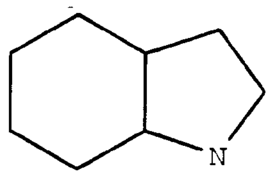 , 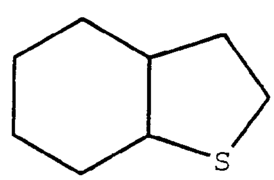 ,

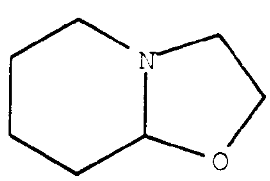 , 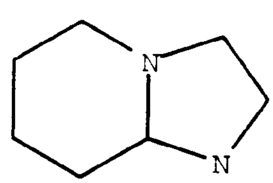 ,

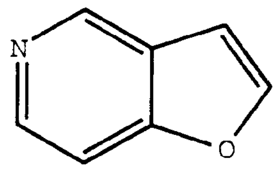 , 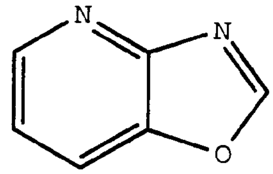 ,

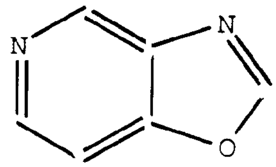 , 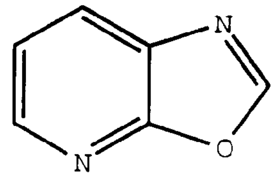 ,

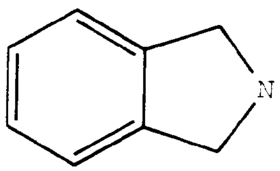 , 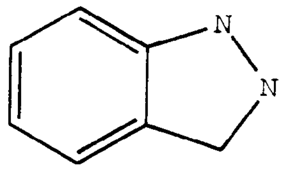 ,

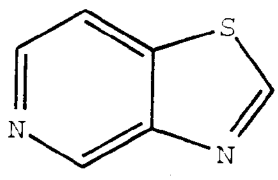 , 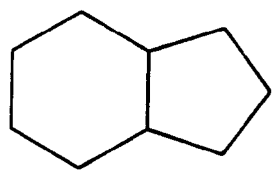 ,

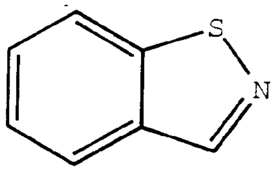 , 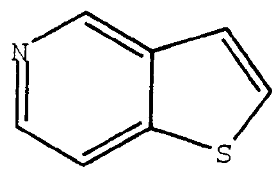 ,

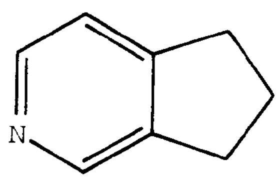 , and 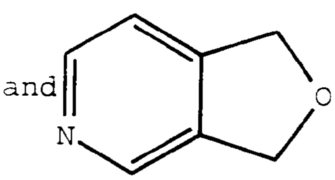 .

The acidic group A is a proton donor illustrated by, but not limited to the following groups:
- 5-tetrazolyl,
- $SO_3H$,
- carboxyl,

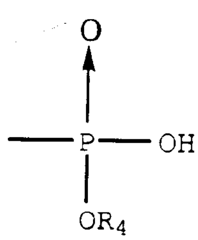 ,

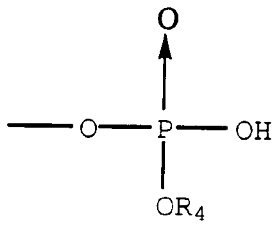

,

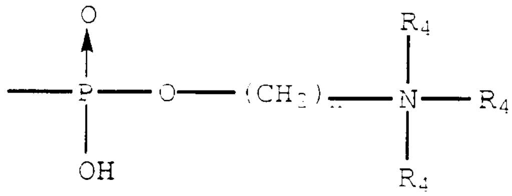

,

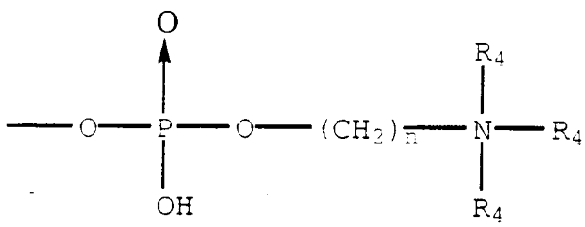

,

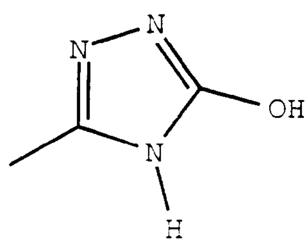

,

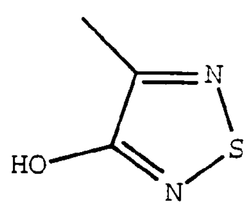

;

where n is an integer from 1 to 12 and $R_4$ is independently selected from:

—H ,

—$(C_1-C_2$ alkyl$)$ ,

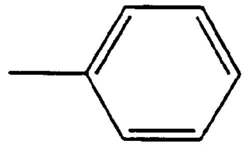

,

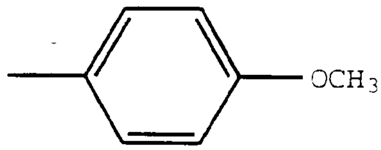

,

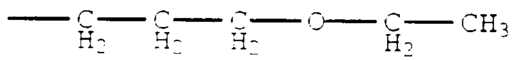

,

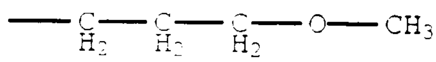

,

$-CONR'$ ,

—(pyridinyl) ,

and

$-SO_2R'$ ,

and where R' is H or $C_1$-$C_{12}$ alkyl.

Particularly preferred acidic groups are those which contain carboxyl functionality.

The basic group B is a proton acceptor illustrated by, but not limited to, the following groups:

amino,

guanidino,

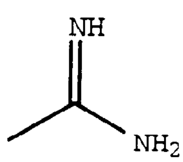

,

and

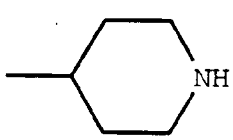

.

The linking groups -($L_b$)- and ($L_a$)-, which attach groups B and A, respectively, to the bicyclic nucleus may both be independently selected from

(i) a bond, or

(ii) a divalent substituted or unsubstituted chain of from 1 to 15 atoms;

Alkylene, alkenylene, and alkynylene groups are useful as linking groups -($L_b$)- and -($L_a$)-.

Preferred linking groups have 4 to 8 chain atoms. It is particularly preferred that the sum of the chain atoms in -($L_b$)- and -($L_a$)- of formula be from 6 to 16.

Linking groups and unsaturated ring structures containing parts such as;

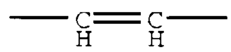

have cis and trans forms and both such forms and their mixtures in all proportions are within the scope of this invention.

Preferred linking groups -($L_a$)- for the acid group A are selected from the group consisting of the following formulae:

(bond),

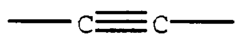 ,

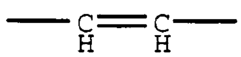 ,

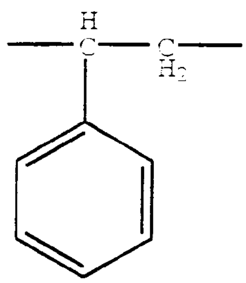 , 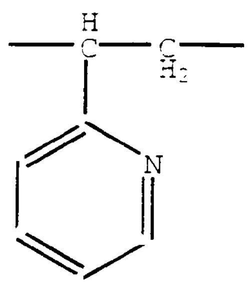 ,

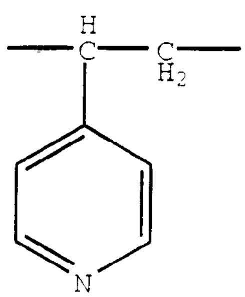 , 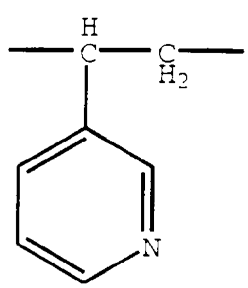 ,

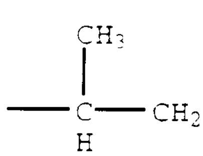 ,

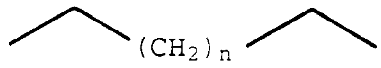

,

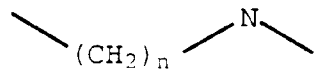

,

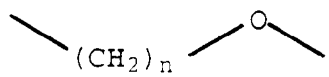

,

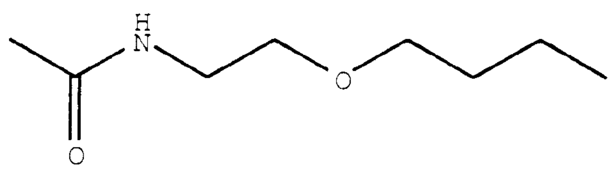

,

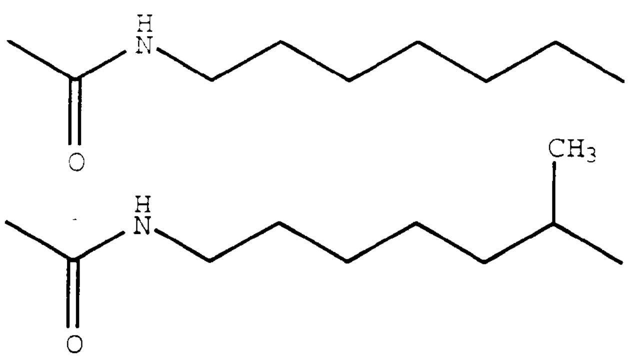

,

,

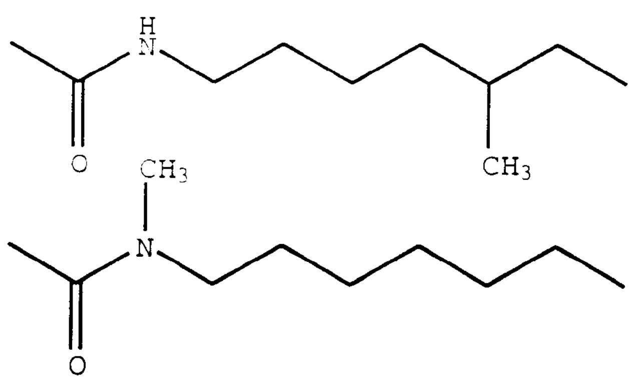

,

and

,

where n is an integer from zero to 8.

Illustrative A-($L_a$)- carboxyl functional acid groups in combination with their linking groups are represented by the formulae shown below:

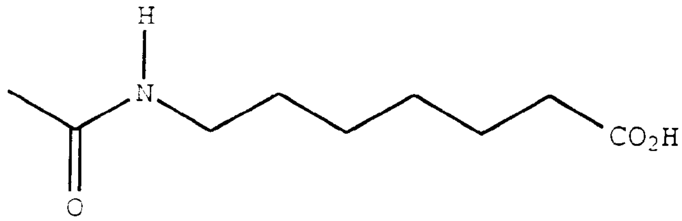

,

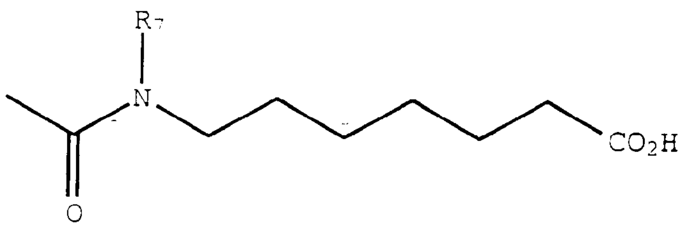

,

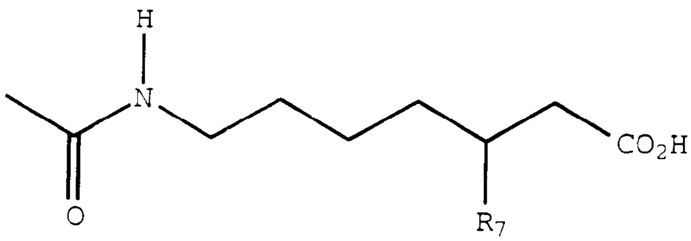

,

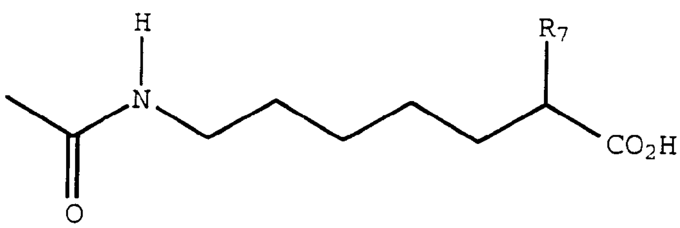

,

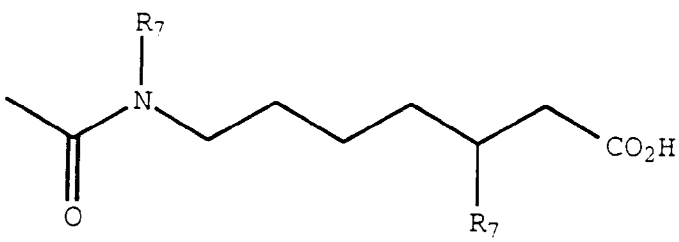

,

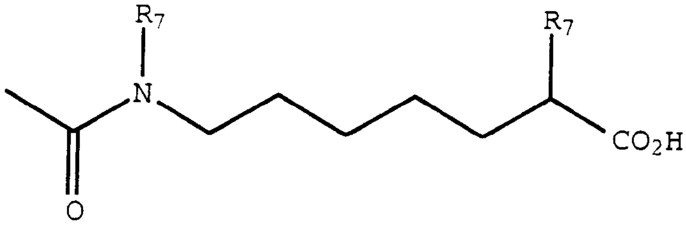

,

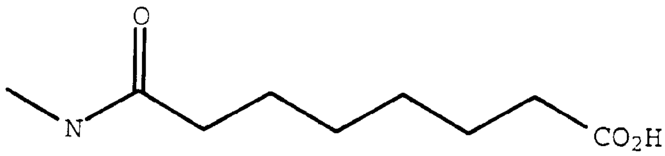 ,

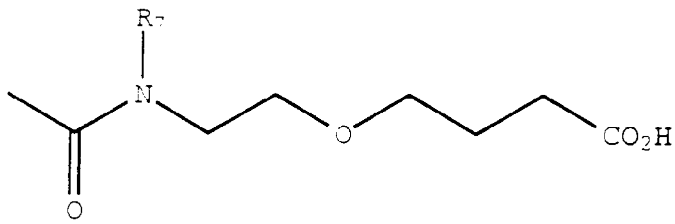 ,

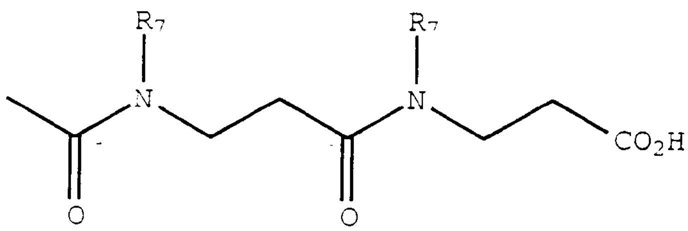 ,

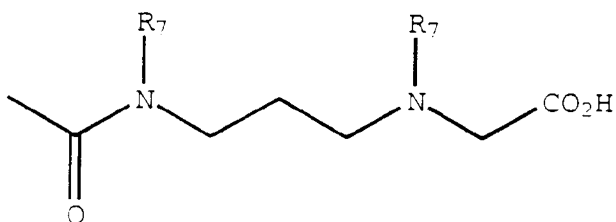 ,

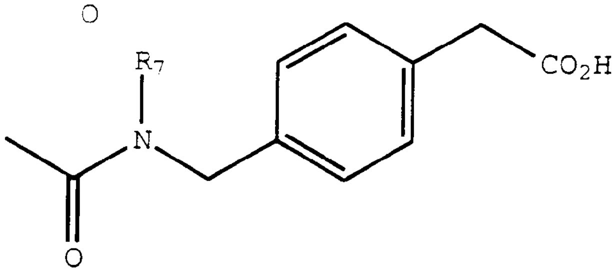 ,

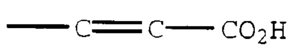 ,

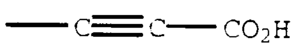 ,

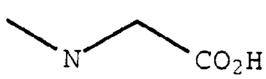 ,

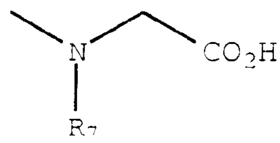

,

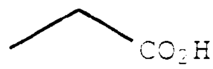

,

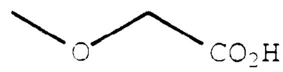

,

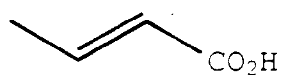

,

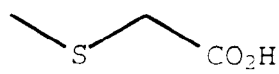

,

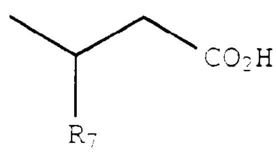

,

and where $R_7$ is independently selected from hydrogen, $C_1$-$C_{12}$ alkyl, phenyl, $C_1$-$C_{12}$ alkenyl, $C_1$-$C_{12}$ alkynyl, and $C_4$-$C_8$ cycloalkyl groups.

Preferred linking groups -($L_b$)- for the base B are selected from the group represented by the following formulae:

(bond)

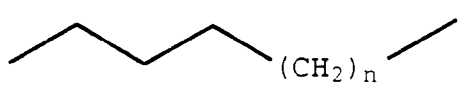

,

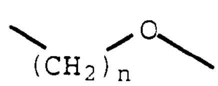

,

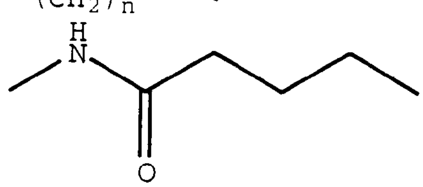

,

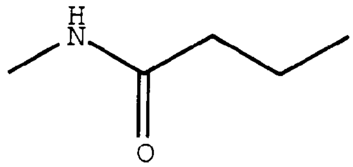

,

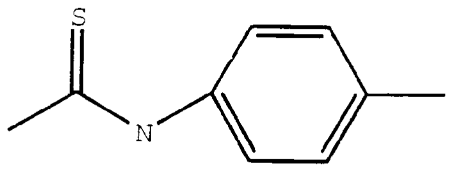

,

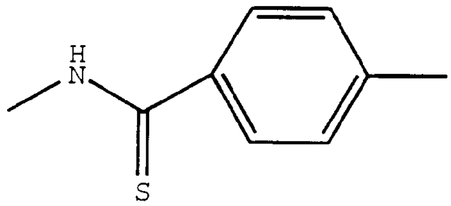

,

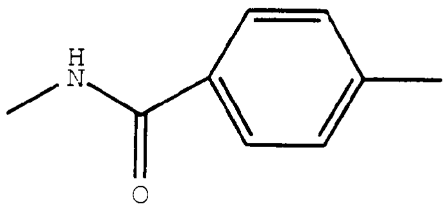

,

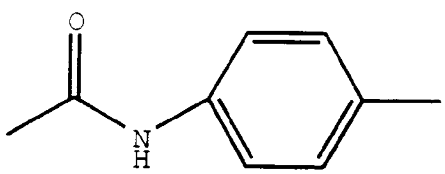

,

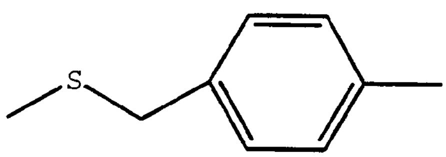

,

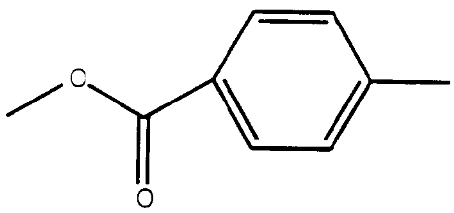

,

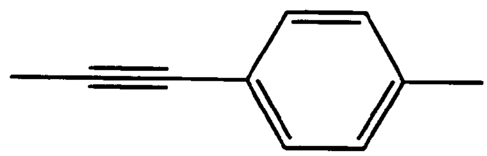 ,

and

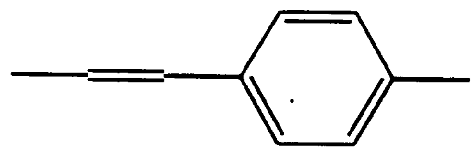 ;

where n is an integer from zero to 8.

Illustrative B-($L_b$)- basic groups in combination with their linking group are represented by the formulae shown below:

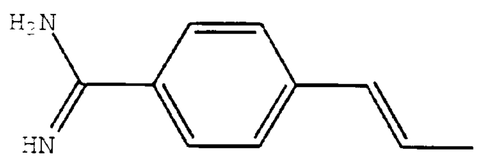 ,

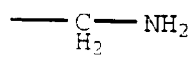

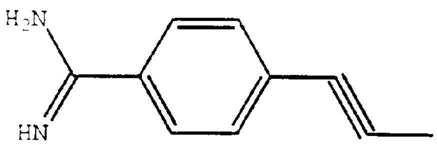 ,

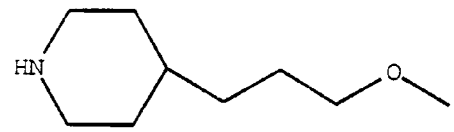 ,

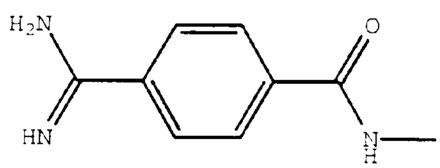 ,

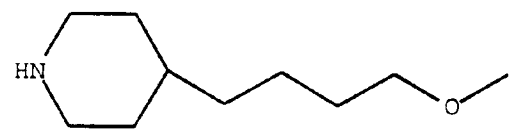 ,

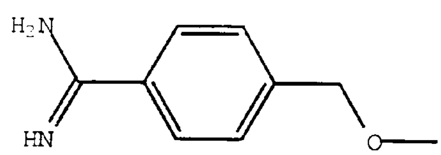 ,

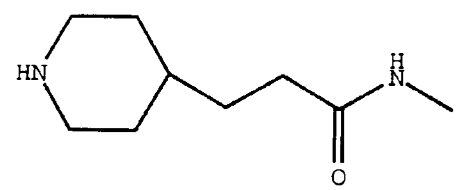 ,

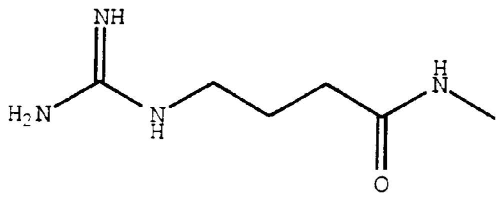 , 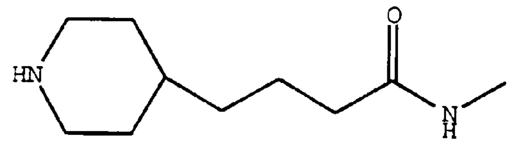 ,

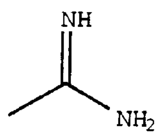 , and 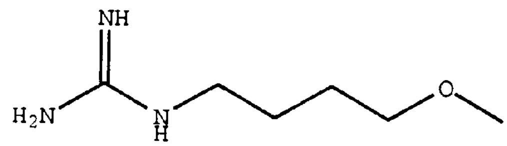 .

Preferred compounds of formula (I) are those wherein $X_1$ and $X_2$ are selected from carbon and nitrogen and the acidic group with its linking group, A-($L_a$)-, is substituted on either ring atom $X_1$ or $X_2$.

Other preferred compounds of formula I are those wherein $X_5$ and $X_6$ are carbon and the basic group with its linking group, B-($L_b$)-, is substituted on either ring atom $X_5$ or $X_6$.

A particularly preferred compound (and all pharmaceutically acceptable salts, solvates, and prodrug derivatives thereof) of the invention is shown in formula II below:

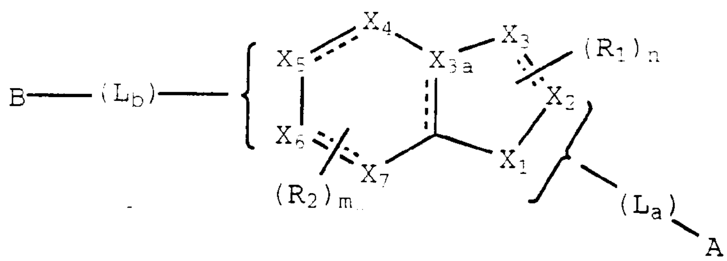 (II)

where all substituents are as previously defined.

In formula II the placement of the A, B, and their respective linking groups is limited, that is, B-($L_b$)- may only be substituted on ring atoms $X_5$ and $X_6$, and A-($L_a$)- may only be substituted on ring atoms $X_1$ or $X_2$.

Preferred compounds corresponding to formula II are those wherein the 5,6 bicyclic nucleus and the position of the A-($L_a$)-and B-($L_b$)- substituents are selected from the group consisting of the substituted nuclei represented by the following thirty structural formulae:

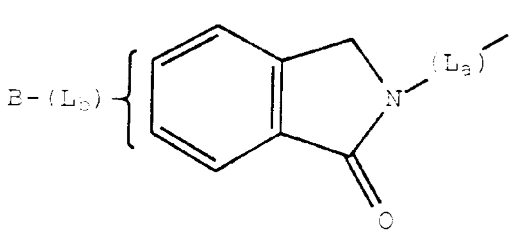 , 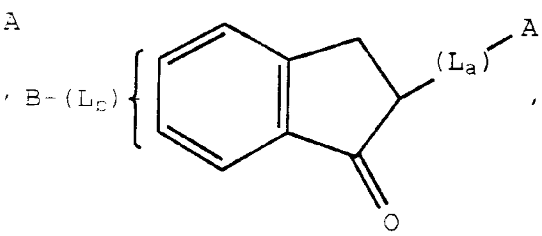 ,

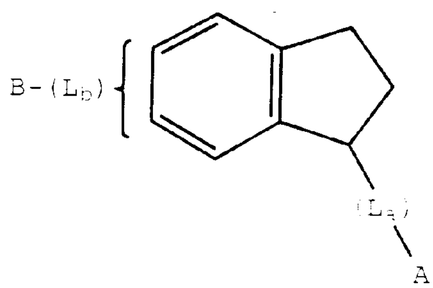 , 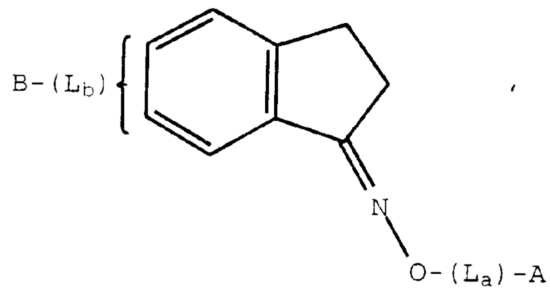 ,

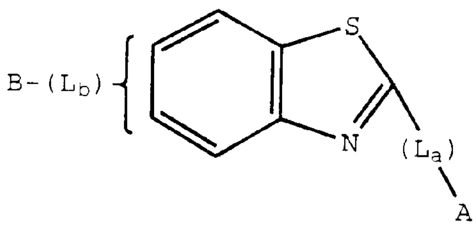 , 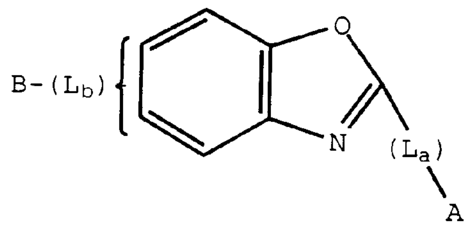 ,

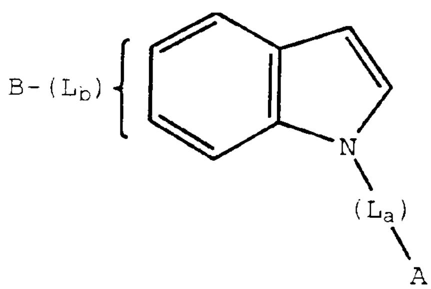 , 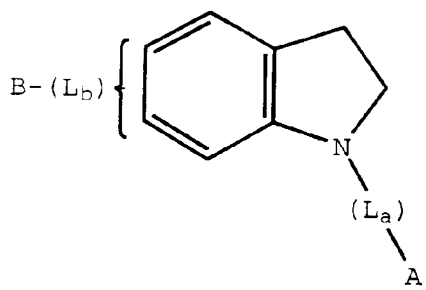 ,

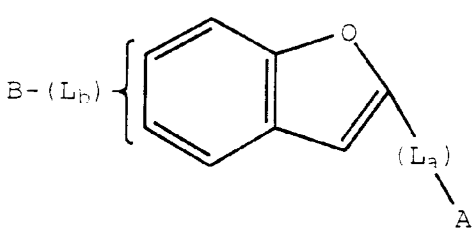 , 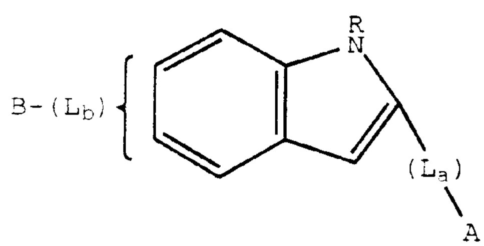 ,

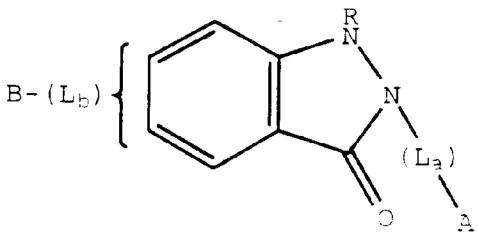 , 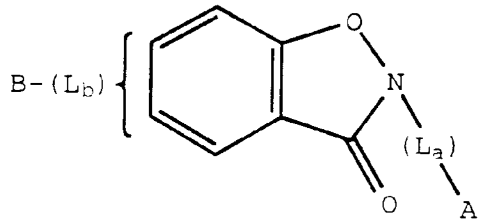 ,

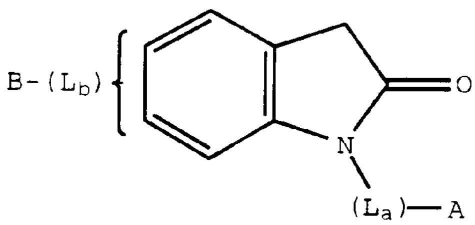 , 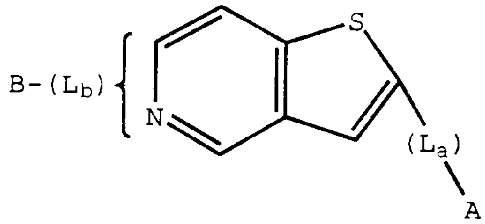 ,

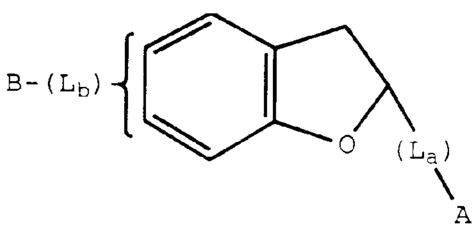 , 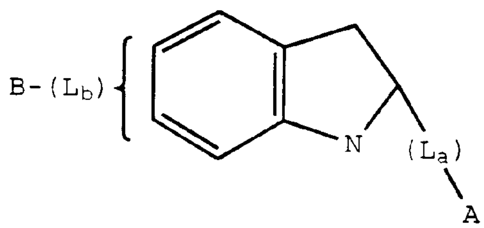 ,

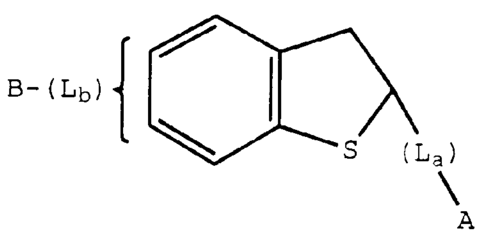 , 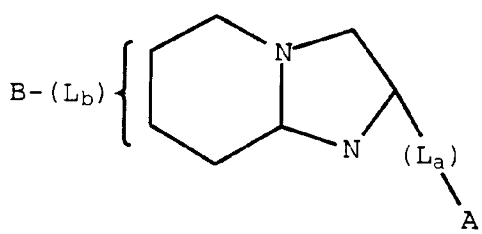 ,

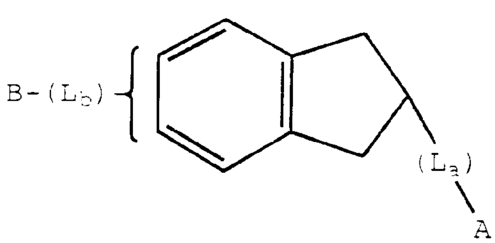 , 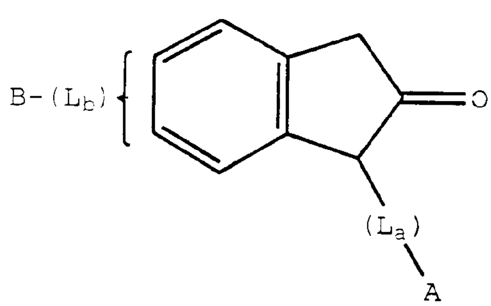 ,

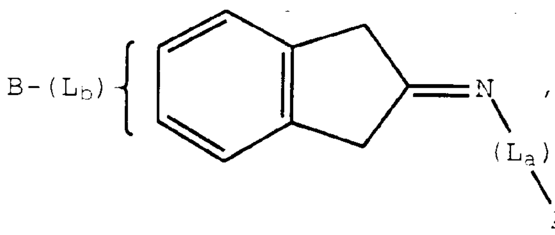 , 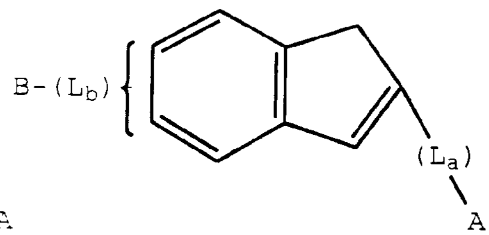 ,

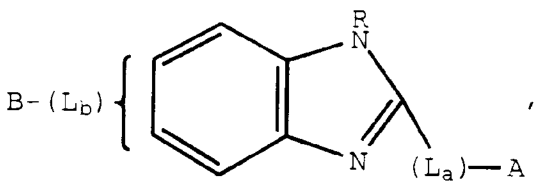 , 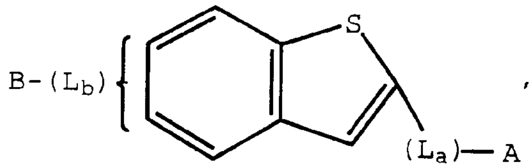 ,

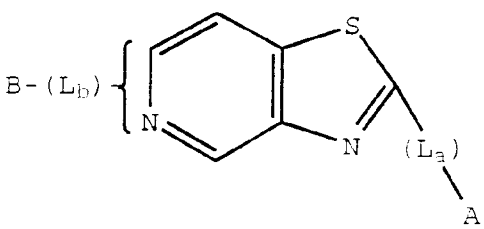 , 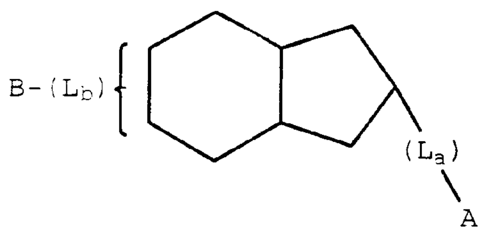 ,

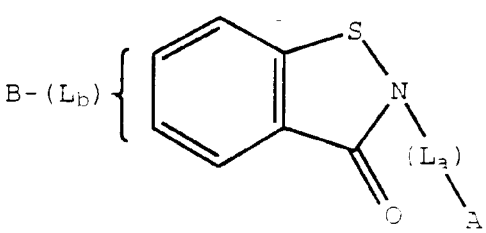 , 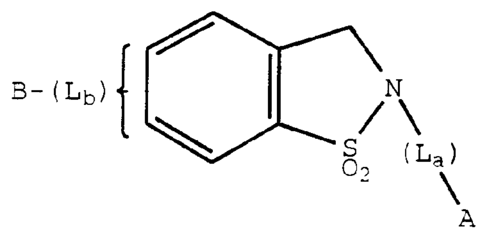 ,

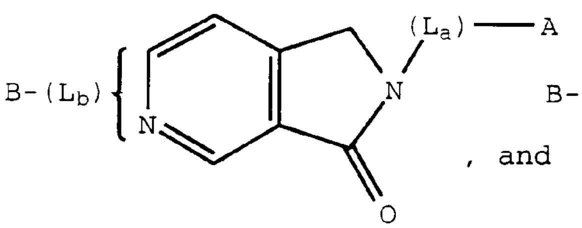 , and 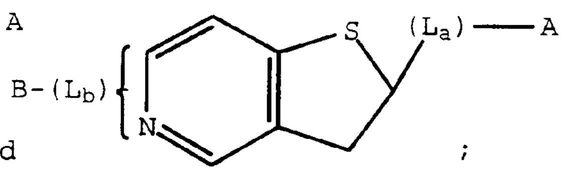 ;

where R is $C_1$-$C_4$ alkyl.

Particularly preferred compounds of formula II are those wherein the 5,6 bicyclic nucleus and the position of its substituents B-($L_b$)- and A-($L_a$)- are selected from the group consisting of the substituted nuclei represented by the following nine structural formulae:

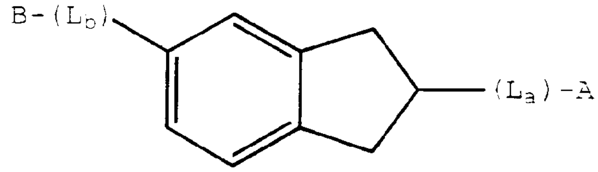

,

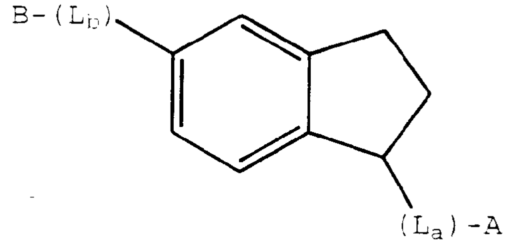

,

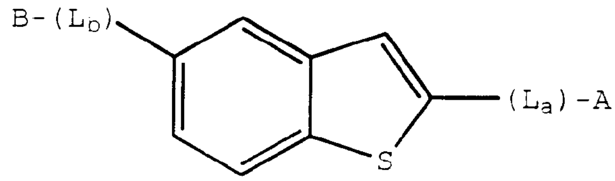

,

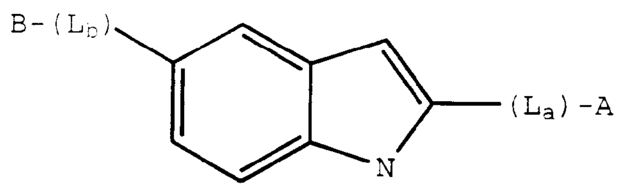

,

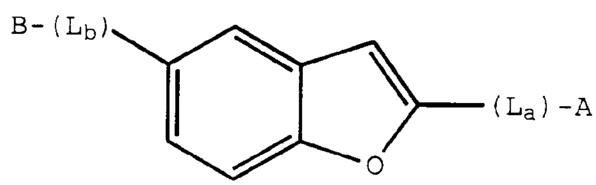

,

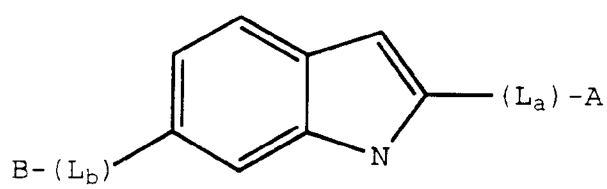

,

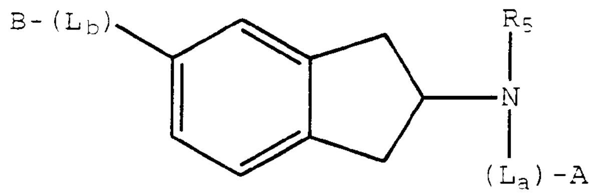

,

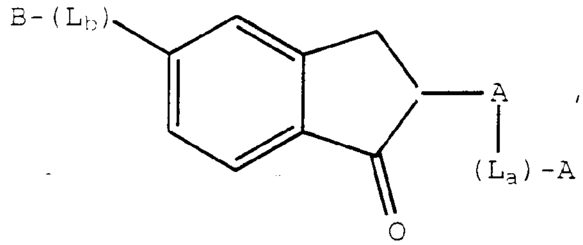

,

and

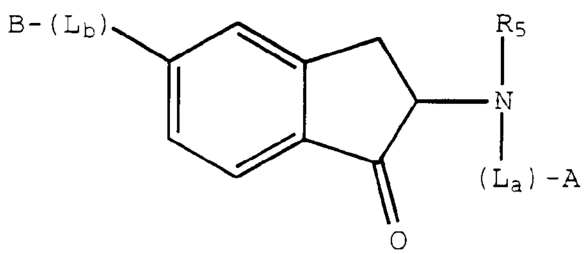

;

where $R_5$ =
-C(O)R', -$SO_2$R', -R'
where R' is H or $C_1$-$C_{12}$ alkyl.

A still more preferred subset of compounds of formula II are represented by formula III which are 5,6 bicyclic indole-type compounds (and all pharmaceutically acceptable salts, solvates, and prodrug derivatives thereof) having a basic group substituted on the $X_5$ ring atom as shown below:

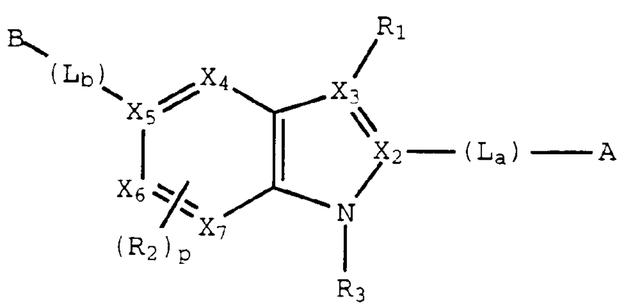

(III)

wherein:

$X_2$ and $X_5$ are carbon;

$X_3$ is independently selected from carbon and nitrogen;

$X_4$, $X_5$, and $X_7$ are independently selected from carbon and nitrogen, with the proviso that at least two of $X_4$, $X_6$, and $X_7$ are carbon;

B is a basic group linked to the six membered ring by linking group -($L_b$)-, where -($L_b$)- is;

(i) a bond, or

(ii) a divalent substituted or unsubstituted chain of from 1 to 15 atoms;

A is an acidic group linked to the five membered ring by linking group -($L_a$)-, where -($L_a$)- is;

(i) a bond, or

(ii) a divalent substituted or unsubstituted chain of from 1 to 15 atoms;

($R_1$) is selected from A, A-($L_a$)-, hydrogen, alkyl, hydroxyalkyl, halosubstituted alkyl, alkenyl, alkynyl, cycloalkyl, aryl, aryloxy, aralkyl, hydroxy, alkoxy, aralkoxy, carbamyl, carboxy, acyl, cyano, halo, nitro, sulfo;

p is an integer selected from 1 to 3;

$(R_2)_p$ are organic radicals attached to one or more of the atoms $X_4$, $X_6$, and $X_7$ of the six membered ring and where $R_2$ are independently selected from B, B-($L_b$)-, hydrogen, alkyl, halosubstituted alkyl, hydroxyalkyl, alkenyl, alkynyl, cycloalkyl, aryl, aryloxy, aralkyl, hydroxy, alkoxy, aralkoxy, carbamyl, amino, substituted amino, acyl, cyano, halo, nitro, and sulfo; and

$R_3$ is hydrogen, halo, $C_1$-$C_4$ alkyl, $C_2$-$C_4$ alkenyl, $C_2$-$C_4$ alkynyl, $C_1$-$C_4$ alkoxy, $C_1$-$C_4$ haloalkyl, aralkyl, alkaryl, hydroxyalkyl and acyl.

A still more preferred subset of compounds of formula II are represented by formula IV which are 5,6 bicyclic indole-type compounds (and all pharmaceutically acceptable salts, solvates, and prodrug derivatives thereof) having a basic group substituted on the $X_6$ ring atom as shown below:

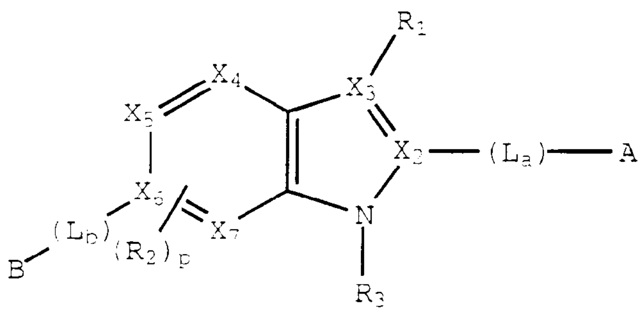

(IV)

wherein:

$X_2$ and $X_6$ are carbon;

$X_3$ is independently selected from carbon and nitrogen;

$X_4$, $X_6$, and $X_7$ are independently selected from carbon and nitrogen, with the proviso that at least two of $X_4$, $X_5$, and $X_7$ are carbon;

B is a basic group linked to the six membered ring by linking group -($L_b$)-, where -($L_b$)- is;

(i) a bond, or

(ii) a divalent substituted or unsubstituted chain of from 1 to 15 atoms;

A is an acidic group linked to the five membered ring by linking group -($L_a$)-, where -($L_a$)- is;

(i) a bond, or

(ii) a divalent substituted or unsubstituted chain of from 1 to 15 atoms;

$R_1$ is selected from A, A-($L_a$)-, hydrogen, alkyl, hydroxyalkyl, halosubstituted alkyl, alkenyl, alkynyl, cycloalkyl, aryl, aryloxy, aralkyl, hydroxy, alkoxy, aralkoxy, carbamyl, carboxy, acyl, cyano, halo, nitro, sulfo;

p is an integer selected from 1 to 3;

$(R_2)_p$ are organic radicals attached to one or more of the atoms $X_4$, $X_6$, and $X_7$ of the six membered ring and where $R_2$ are independently selected from B, B-($L_b$)-, hydrogen, alkyl, halosubstituted alkyl, hydroxyalkyl, alkenyl, alkynyl, cycloalkyl, aryl, aryloxy, aralkyl, hydroxy, alkoxy, aralkoxy, carbamyl, amino, substituted amino, acyl, cyano, halo, nitro, and sulfo; and

$R_3$ is hydrogen, halo, $C_1$-$C_4$ alkyl, $C_2$-$C_4$ alkenyl, $C_2$-$C_4$ alkynyl, $C_1$-$C_4$ alkoxy, $C_1$-$C_4$ haloalkyl, aralkyl, alkaryl, hydroxyalkyl and acyl.

Another highly preferred subset of compounds of formula II are represented by formula V which are 5,6 bicyclic benzofuran-type compounds and all pharmaceutically acceptable salts, solvates, and prodrug derivatives thereof) as shown below:

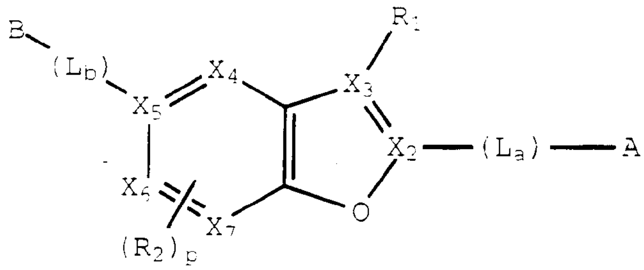

(V)

wherein:

$X_2$ and $X_5$ are carbon;

$X_3$ is independently selected from carbon and nitrogen;

$X_4$, $X_6$, and $X_7$ are independently selected from carbon and nitrogen, with the proviso that at least two of $X_4$, $X_6$, and $X_7$ are carbon;

B is a basic group linked to the six membered ring by linking group $-(L_b)-$, where $-(L_b)-$ is;

(i) a bond, or

(ii) a divalent substituted or unsubstituted chain of from 1 to 15 atoms;

A is an acidic group linked to the five membered ring by linking group $-(L_a)-$, where $-(L_a)$ is;

(i) a bond, or

(ii) a divalent substituted or unsubstituted chain of from 1 to 15 atoms;

$R_1$ is selected from A, A-$(L_a)$-, hydrogen, alkyl, hydroxyalkyl, halosubstituted alkyl, alkenyl, alkynyl, cycloalkyl, aryl, aryloxy, aralkyl, hydroxy, alkoxy, aralkoxy, carbamyl, carboxy, acyl, cyano, halo, nitro, sulfo;

p is an integer selected from 1 to 3;

$(R_2)_p$ are organic radicals attached to one or more of the atoms $X_4$, $X_6$, and $X_7$ of the six membered ring and where $R_2$ are independently selected from B, B-$(L_b)$-, hydrogen, alkyl, halosubstituted alkyl, hydroxyalkyl, alkenyl, alkynyl, cycloalkyl, aryl, aryloxy, aralkyl, hydroxy, alkoxy, aralkoxy, carbamyl, amino, substituted amino, acyl, cyano, halo, nitro, and sulfo.

Another preferred subset of compounds of formula II are represented by formula VI which are 5,6 bicyclic benzothiophene-type compounds and all pharmaceutically acceptable salts, solvates, and prodrug derivatives thereof) as shown below:

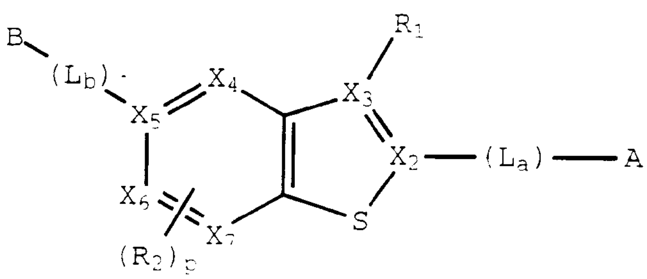

(VI)

where the substituents of formula VI are as previously defined for formula V.

Specific compounds of the invention which are highly preferred are represented by the following structural formulae X to XXIV (and mixtures thereof) and all pharmaceutically acceptable salts, solvates and prodrug derivatives thereof:

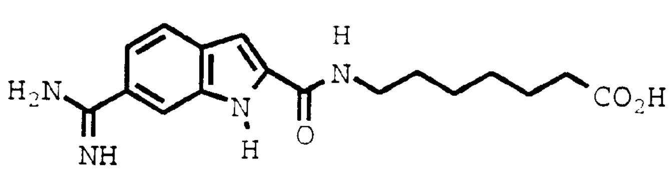

, (X)

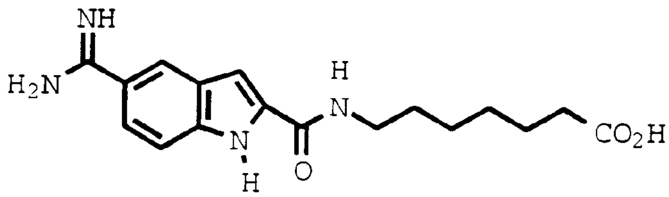

, (XI)

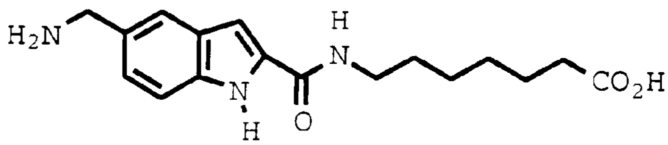

, (XII)

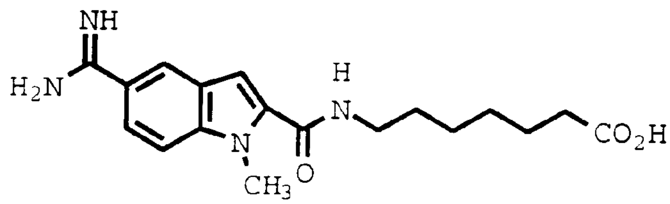

, (XIII)

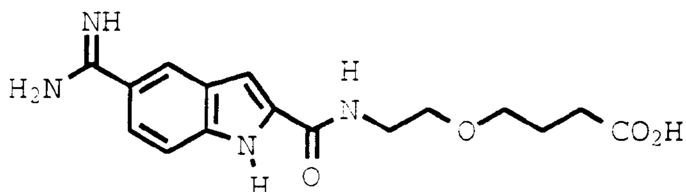

, (XIV)

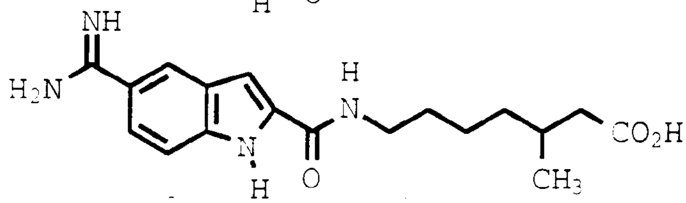

, (XV)

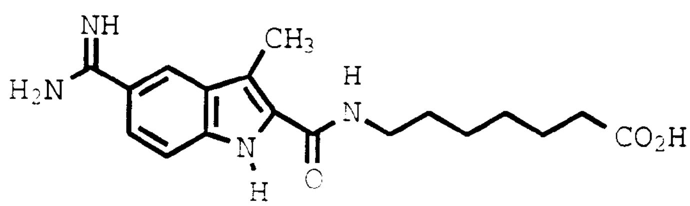

, (XVI)

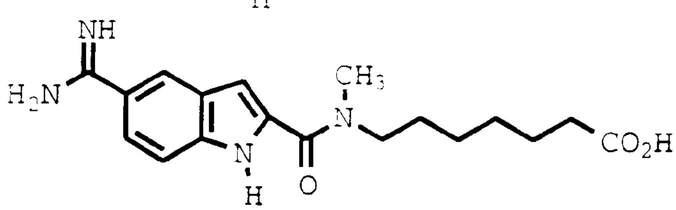

, (XVII)

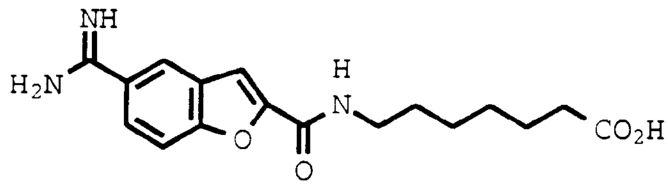

, (XVIII)

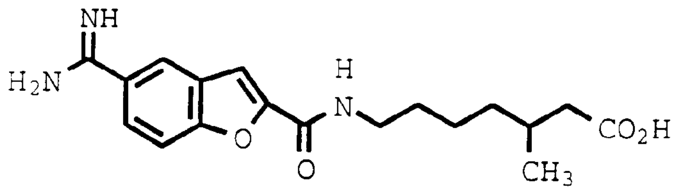

, (XIX)

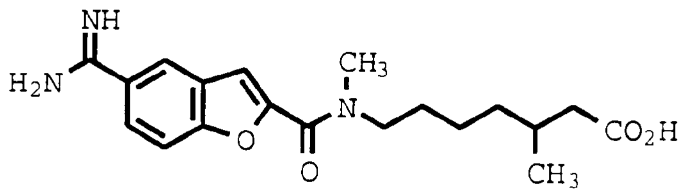

, (XX)

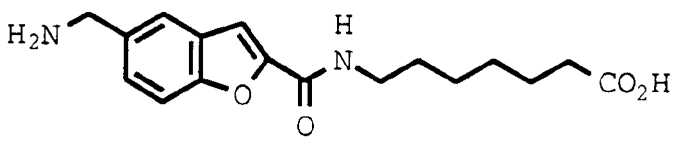

, (XXI)

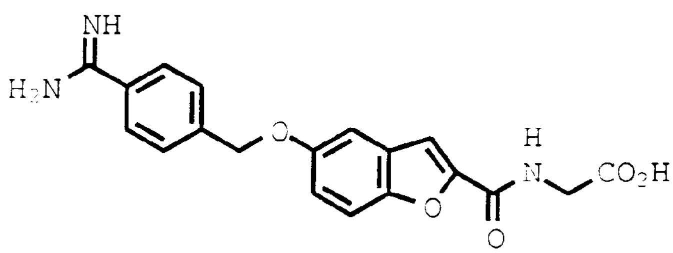

, (XXII)

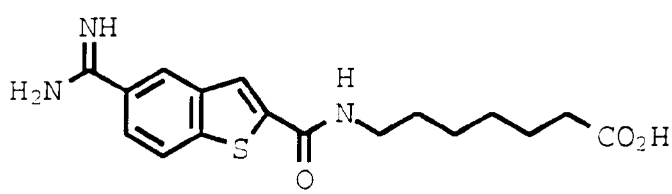

, (XXIII)

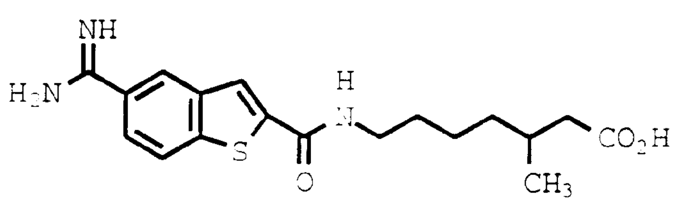

. (XXIV)

Other specific compounds of the invention are represented by the following structural formulae XXVI to XXXVIII and all pharmaceutically acceptable salts, solvates and prodrug derivatives thereof:

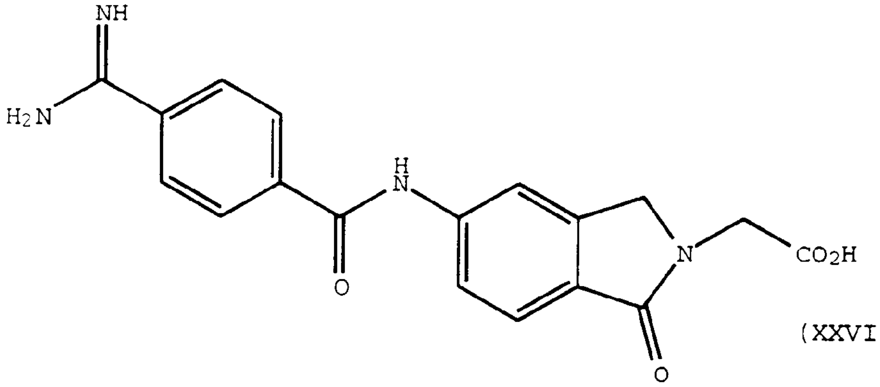

(XXVI) ,

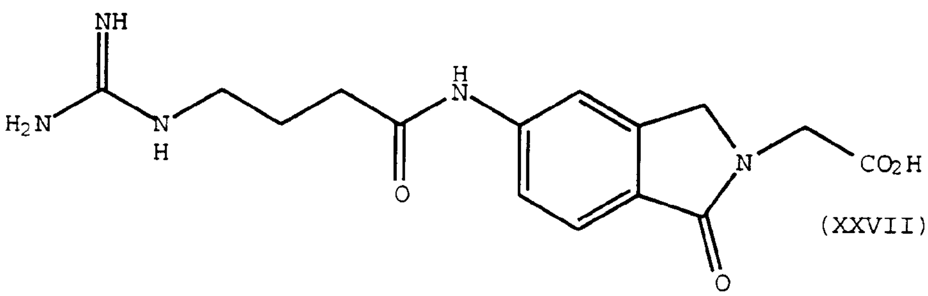

(XXVII) ,

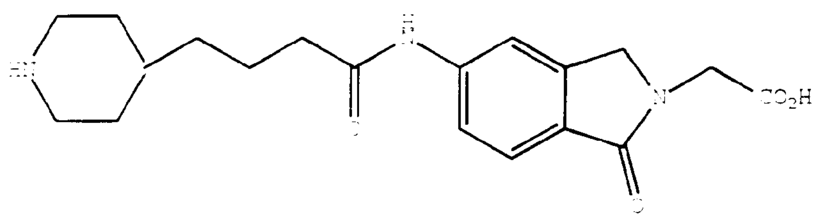

, (XXVIII)

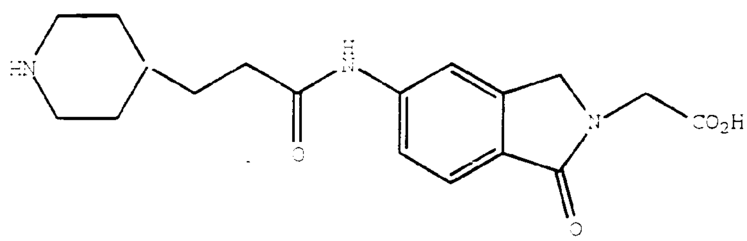

, (XXIX)

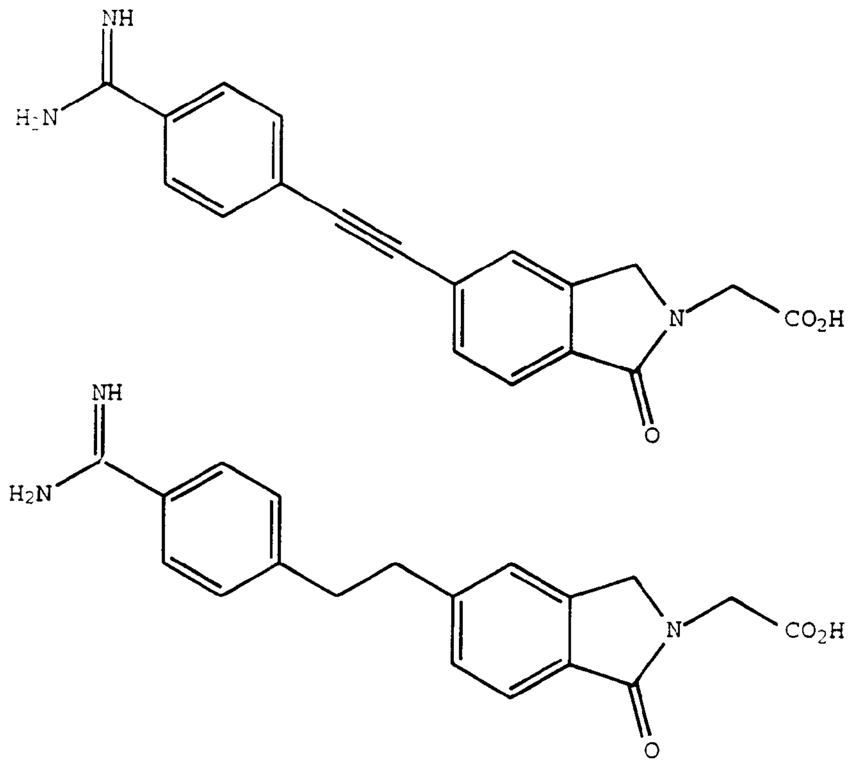

, (XXX)

, (XXXI)

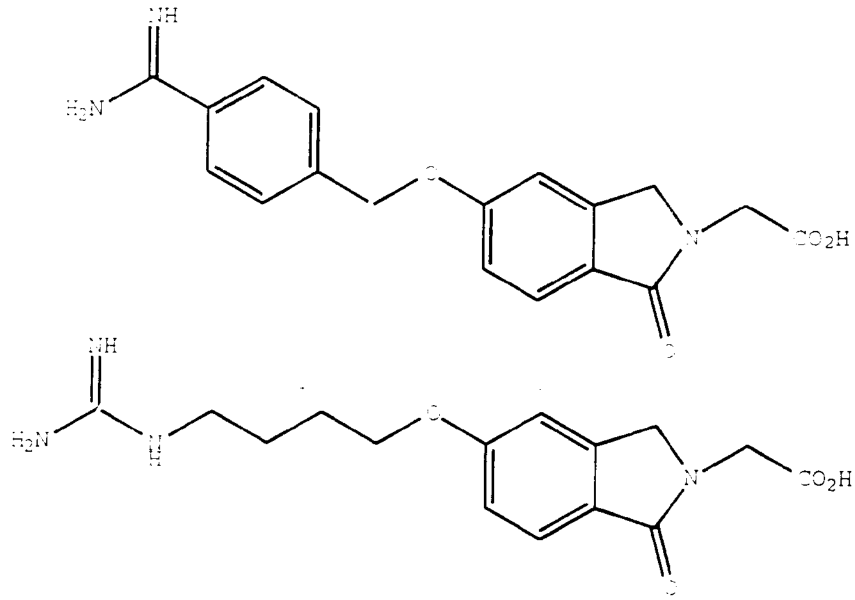

, (XXXII)

, (XXXIII)

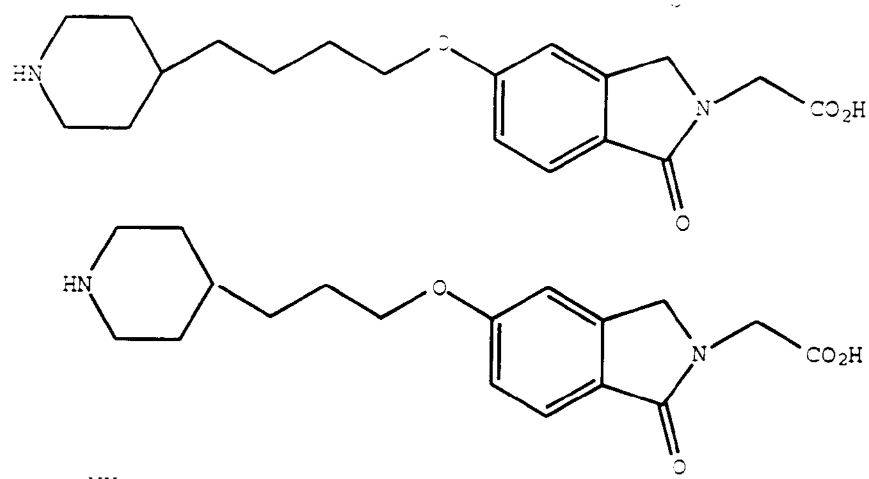

, (XXXIV)

, (XXXV)

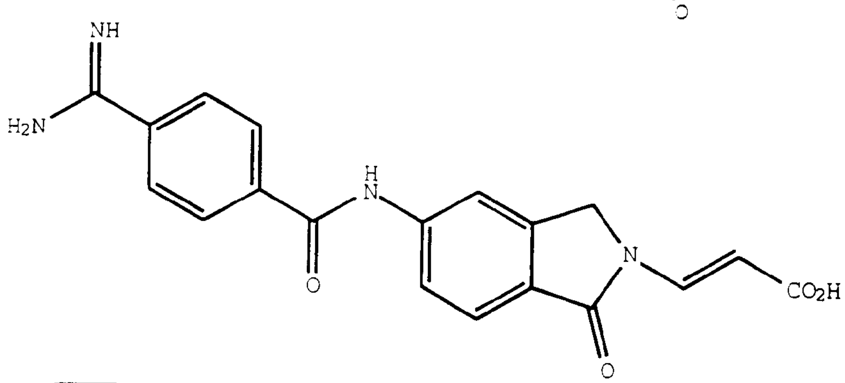

, (XXXVI)

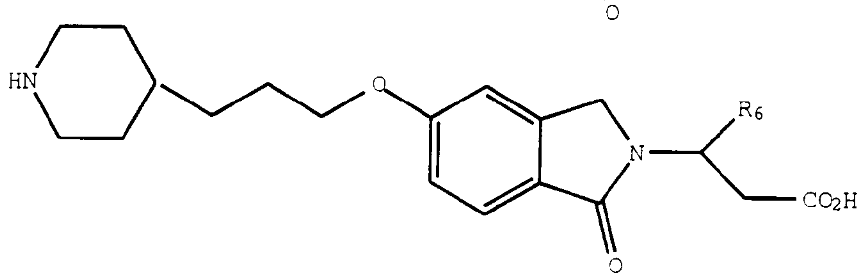

, (XXXVII)

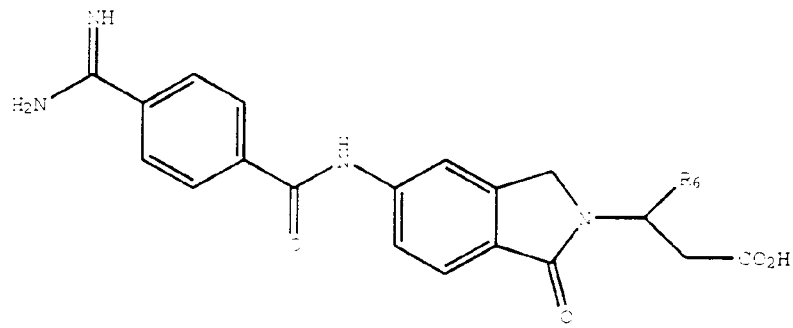

, (XXXVIII)

and mixtures of XXVI to XXXVIII;

where

$R_6$ =

$CH_3$, $CH_2CH_3$, $CH_2CH_3CH_3$,

$CH_2CH_2CH_2CH_3$, $CH_2CH_2CH_2CH_2CH_3$

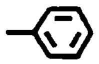, 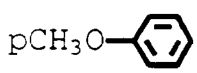, 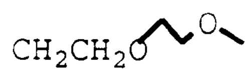

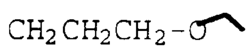, $CH_2CH_2CH_2OCH_3$

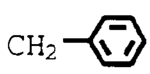, 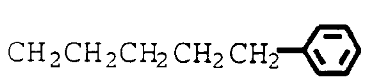,

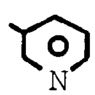, 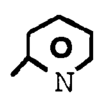, and 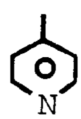.

The compounds of the invention possess at least one acidic functional substituent (viz., A of Formula I) and, as such, are capable of forming salts. Representative pharmaceutically acceptable salts, include but are not limited to, the alkali and alkaline earth salts such as lithium, sodium, potassium, calcium, magnesium, aluminum and the like. Salts are conveniently prepared from the free acid by treating the acid in solution with a base or by exposing the acid to an anion exchange resin on the salt cycle.

Included within the definition of pharmaceutically acceptable salts are the relatively non-toxic, inorganic and organic base addition salts of compounds of the present invention, for example, ammonium, quaternary ammonium, and amine actions, derived from nitrogenous bases of sufficient basicity to form salts with the compounds of this invention (see, for example, S. M. Berge, et. al., "Pharmaceutical Salts," J. Phar. Sci., 66: 1-19 (1977)).

The basic portion of the compounds of the invention (viz., group B of formula I) may be reacted with suitable organic or inorganic acids to form salts of the invention. Representative salts include; acetate, benzenesulfonate, benzoate, bicarbonate, bisulfate, bitartrate, borate, bromide, camsylate, carbonate, chloride, clavulanate, citrate, dihydrochloride, edetate, edisylate, estolate, esylate, fumarate, gluceptate, gluconate, glutamate, glycollylarsanllate, hexylresorcinate, hydrabamine, hydrobromide, hydrochloride, hydroxynaphthoate, iodide, isothionate, lactate, lactobionate, laurate, malate, malseate, mandelate, mesylate, methylbromide, methylnitrate, methylsulfate, mucate, napsylate, nitrate, oleate, oxalate, palmitate, pantothenate, phosphate, polyga-

lacturonate, salicylate, stearate, subacetate, succinate, tannate, tartrate, tosylate, trifluoroacetate, trifluoromethane sulfonate, and valerate.

The compounds of the formula (I) can also be in the form of zwitterions, since they contain both acidic and basic functionality and are capable of self-protonation.

Certain compounds of the invention possess one or more chiral centers and may thus exist in optically active forms. Likewise, when the compounds contain an alkenyl or alkenylene group there exists the possibility of *cis*- and *trans*- isomeric forms of the compounds. The *R*- and *S*- isomers and mixtures thereof, including racemic mixtures as well as mixtures of *cis*- and *trans*- isomers, are contemplated by this invention. Additional asymmetric carbon atoms can be present in a substituent group such as an alkyl group. All such isomers as well as the mixtures thereof are intended to be included in the invention. If a particular stereoisomer is desired, it can be prepared by methods well known in the art by using stereospecific reactions with starting materials which contain the asymmetric centers and are already resolved or, alternatively by methods which lead to mixtures of the stereoisomers and subsequent resolution by known methods. Prodrugs are derivatives of the compounds of the invention which have chemically or metabolically cleavable groups and become by solvolysis or under physiological conditions the compounds of the invention which are pharmaceutically active *in vivo*. Derivatives of the compounds of this invention have activity in both their acid and base derivative forms, but the acid derivative form often offers advantages of solubility, tissue compatibility, or delayed release in a mammalian organism (see, Bundgard, H., Design of Prodrugs, pp. 7-9, 21-24, Elsevier, Ansterdam 1985). Prodrugs include acid derivatives well known to practitioners of the art, such as, for example, esters prepared by reaction of the parent acidic compound with a suitable alcohol, or amides prepared by reaction of the parent acid compound with a suitable amine. Simple aliphatic or aromatic esters derived from acidic groups pendent on the compounds of this invention are preferred prodrugs. In some cases it is desirable to prepare double ester type prodrugs such as (acyloxy) alkyl esters or ((alkoxycarbonyl)oxy)alkyl esters.

Method of Making Compounds of the Invention

General synthetic schemes 1 through 14 shown below are used to prepare the compounds of the invention.

The following abbreviations are used throughout the synthetic schemes and examples:

| | |
|---|---|
| Boc | tertiary-butyloxy carbonyl |
| DMF | dimethyl formamide |
| TFA | trifluoroacetic acid |
| Cbz | benzyloxycarbonyl |
| EDCI | 1-(3-dimethylaminopropyl)-3-ethyl carbodiimide hydrochloride |
| DMAP | 4-dimethylaminopyridine |
| THF | tetrahydrofuran |
| $Boc_2O$ | di-tert-butyl dicarbonate |
| HMDS | 1,1,1,3,3,3-hexamethyl disilazane |
| DIEA | diisopropylethylamine |

The compounds of the invention may be prepared by the general reaction schemes set out below:

Reaction schemes 1 and 2 describe methodology for incorporating the basic group (B) into compounds having the general formula (I):

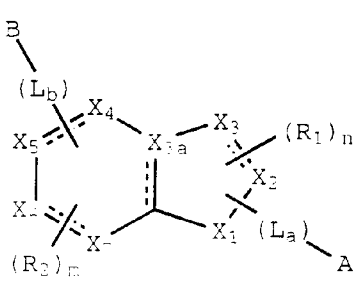

(I)

However, for the sake of illustration, Schemes 1 and 2 depict a subclass of compounds of formula I having the formula (Ia) as follows:

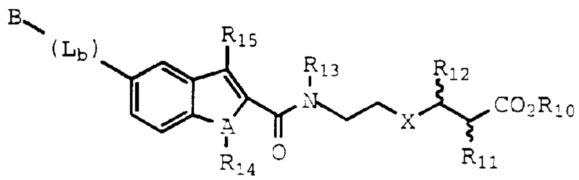

(Ia)

where;

$R_{10}$, $R_{11}$, $R_{12}$, $R_{13}$, and $R_{15}$ are independently selected from hydrogen, methyl, ethyl, n-butyl, or tert-butyl;

$R_{14}$ is absent or selected from hydrogen, methyl, ethyl, n-butyl, or tert-butyl;

A is N, O, or S, and the ring atoms adjacent A are carbon (as shown);

$-(L_b)-$ is a divalent group attached to the 5 or 6 position of the six membered ring and selected from a bond, methylene, or benxyloxy;

B is a basic group selected from amine or amidine; and

X is a divalent group selected from $-(CH_2)n-$, (where n is an integer from 1 to 4) $-(CH_2)_p-O-(CH_2)_q-$ (where p and q are integers from zero to 4, with the proviso that p plus q equals n.

Scheme 1

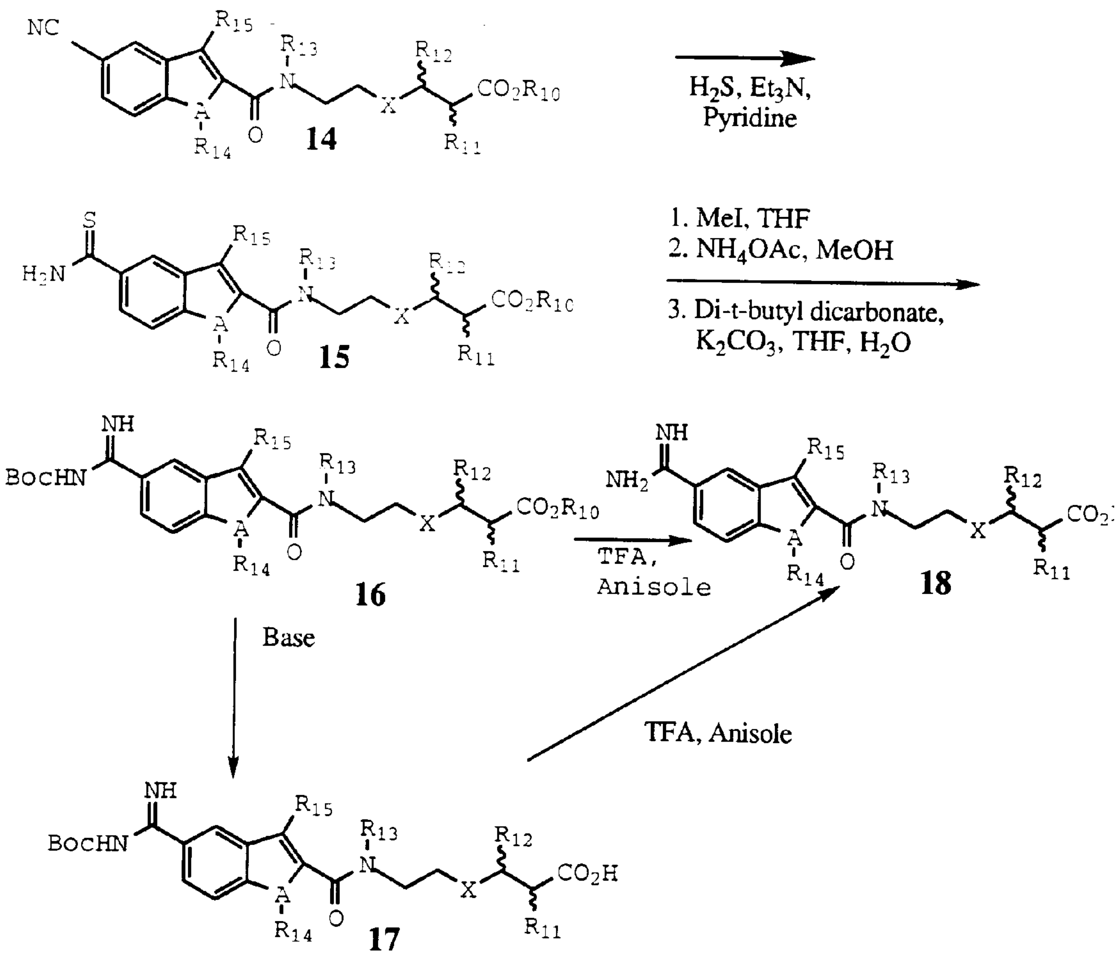


A basic (amidine) 5,6 bicyclic compound is prepared from the corresponding nitrile by the process of

Scheme 1. The starting material, nitrile (14), is converted to a thioamide compound (15). Thereafter, the thioamide compound (15) is converted to a thioimidate with methyl iodide. The thioimidate is not isolated and is thereafter converted to amidine, then again reacted with di-tert butyl dicarbonate to yield the protected amidino ester (16) which can be purified. Compound (16) is converted to product (18) by removing the carboxyl ($R_{10}$) and amidino protecting groups (Boc). If the group $R_{10}$ on the right hand side is not tert-butyl, (e.g., methyl, ethyl) then the conversion of compound (16) to (18) may be accomplished by proceeding through compound (17) using a base as shown in Scheme 1. If the group, $R_{10}$, on the right hand side is t-butyl, the Boc protected amidino ester (16) can be converted directly to product (18) as shown in Scheme 1.

Another variation of the general method of going from intermediate (14) to compounds of the invention (18) is to use the conditions of Scheme 2 which require no protection/deprotection steps as shown below:

Scheme 2

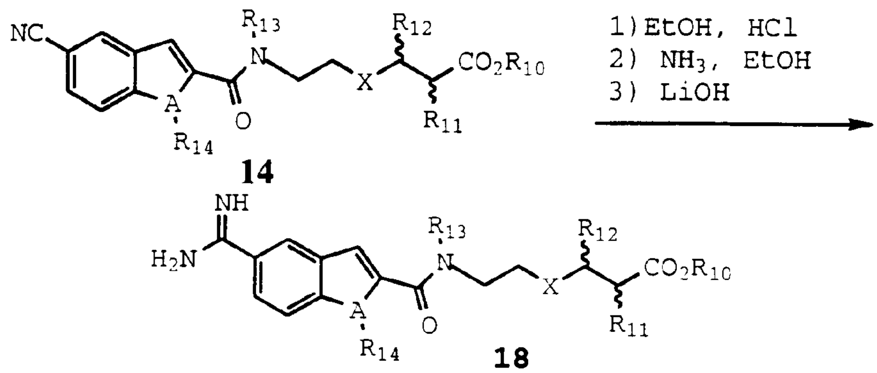


14

18

In Scheme 2, product (18) is derived from (14) by initial conversion of (14) to the corresponding imidate using EtOH and HCl. Ammonia is then used to convert the imidate to the corresponding amidine, which is hydrolyzed to the acidic product (18) using lithium hydroxide.

The previous reaction Schemes 1 and 2 are generic for the preparation of compounds of the invention having a variety of heteroatoms, designated "A." The following Scheme 3 depicts the preparation of hetero-nitrogen containing compounds (A = N) of the invention (indole-types) represented by formula (3a):

Scheme 3

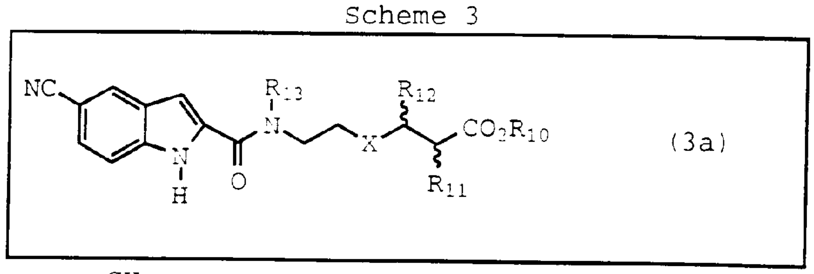

(3a)

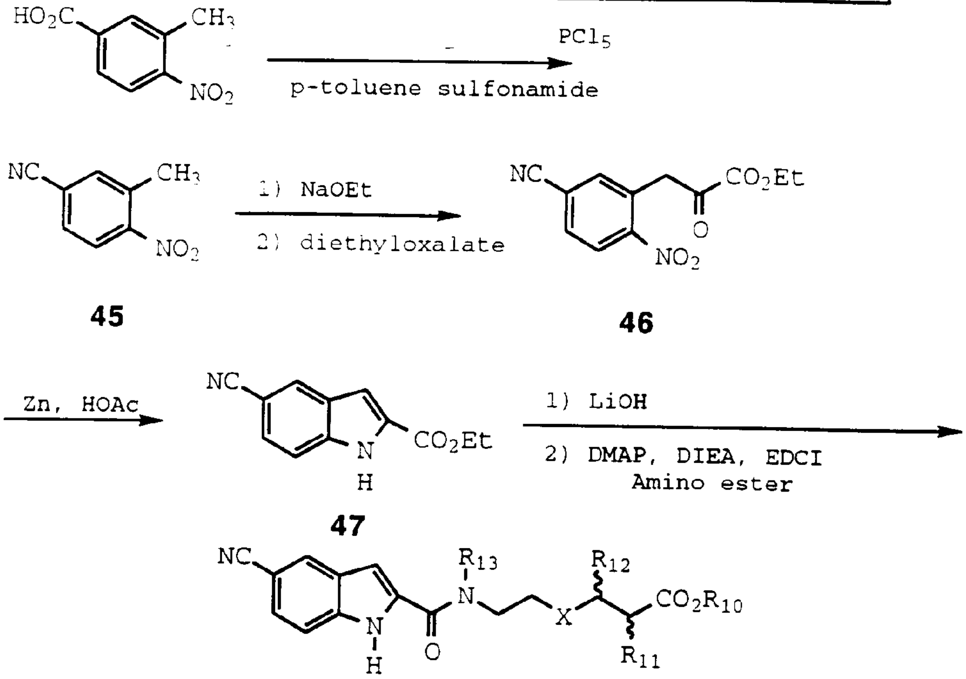


45

46

47

14

The 3-methyl-4-nitrobenzoic acid starting material is reacted with phosphorus pentachloride and p-toluene sulfonamide to convert it to the corresponding nitrile (45). Compound 45 is deprotonated with a suitable base to generate a benzylic anion which is reacted with diethyloxalate, affording the α-keto ester (46). Reduction of the nitro group in compound (46) followed by in situ cyclization gives indole (47), which is thererafter reacted with LiOH to hydrolyze the compound to its acid form.

The acid form of (47) is used to couple to a suitable amino ester by the use of the three reagents, DMAP, DIEA and EDCI, thereby yielding amide (14). The series of amides (14), are converted to the amidino acid products (18) of the invention by using the reaction conditions outlined in Schemes 1 and 2.

Reaction scheme 3a outlines the synthesis of heteronitrogen containing intermediates (A = N) of the invention (indole-types) represented by formula (14p). These intermediates are used in the preparation of compounds of Formula I in which the basic group is attached to the 6 position of the six membered ring:

Scheme 3a

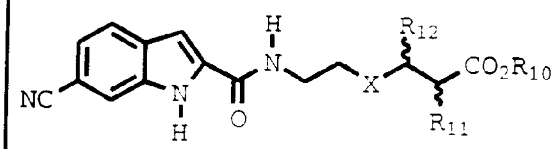

(14p)

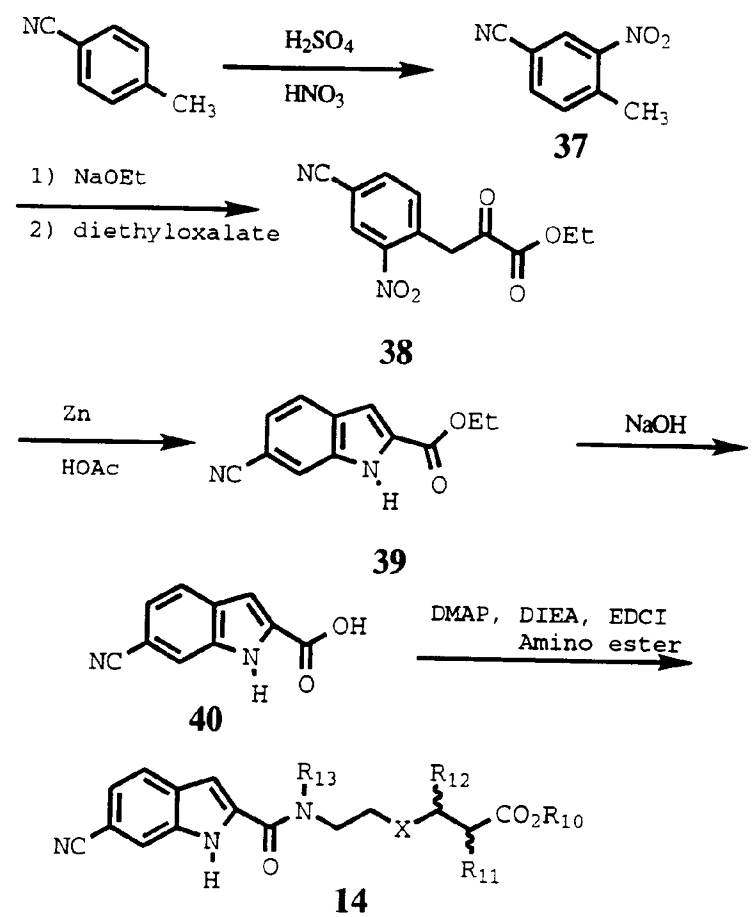


37

38

39

40

14

The synthesis of compound 14p begins with nitration of the starting material, p-tolunitrile, using nitric acid/sulfuric acid to afford 2-nitro-4-cyanotoluene (37). Deprotonation at the benzylic position of (37) using a suitable base followed by reaction of the resulting anion with diethyloxalate affords the α-ketoester (38). Reduction of the nitro group of compound (38) using zinc in acetic acid followed by in situ cyclization affords ethyl 6-cyanoindole-2-carboxylate (39) which is thereafter subjected to base hydrolysis (NaOH) to give 6-cyanoindole-2-carboxylate (40). The acid (40) is used to couple a suitable amino ester by the use of the three reagents, DMAP and DIEA and EDCI thereby yielding amide (14p) which is converted to the amidino acid product (18p) by using the reaction conditions outlined in Scheme 1.

Scheme 4 is a subset of Scheme 3 and illustrates a method of putting an organic radical (viz.,$R_{15}$, a non-hydrogen) at the 3 position of the five membered ring in the bicyclic nucleus. Starting with compound (46) from Scheme 3, deprotonation and subsequent alkylation at the benzylic position affords compound (61). The remainder of the synthesis proceeds according to the reaction conditions outlined in Scheme 3.

Scheme 4

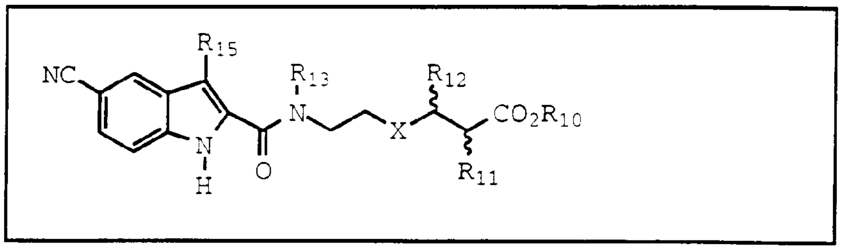

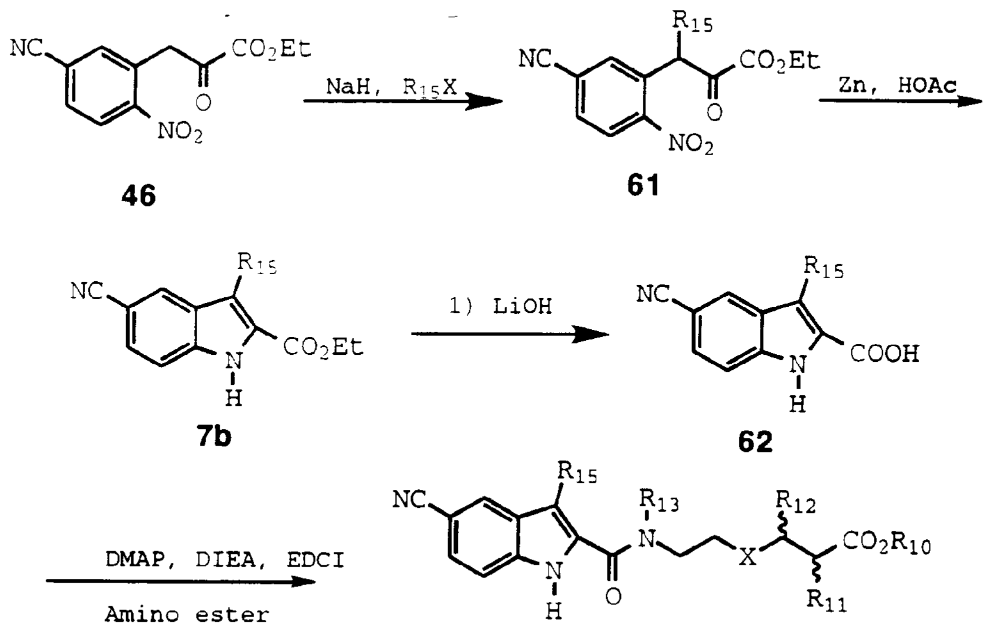


46

61

7b

62

14

Scheme 5 depicts the preparation of 5-cyanobenzofuran-type (A = O) intermediates represented by formula (5a) used to prepare the compounds of the invention:

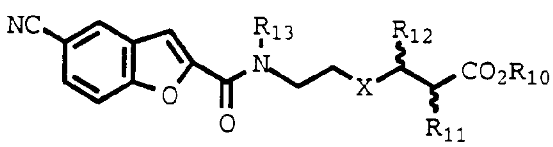

(5a)

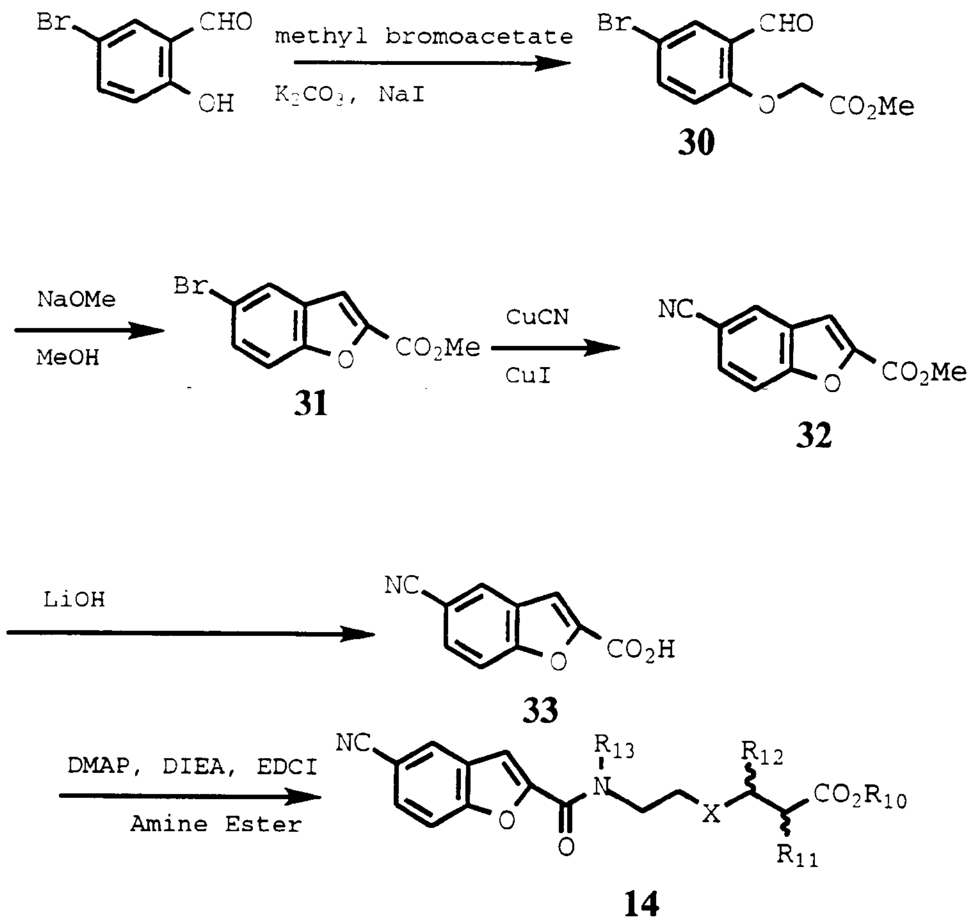


30

31

32

33

14

In Scheme 5, 2-hydroxy-5-bromobenzaldehyde is alkylated with methyl bromoacetate to give the corresponding ester (30) which is thereafter cyclized to give benzofuran (31). The aryl bromide of compound (31) is displaced with cyanide, then LiOH is used to hydrolyze the methyl ester to afford the corresponding acid (33). Acid (33) is then coupled with the desired amino ester by the use of the three reagents, DMAP and DIEA and EDCI. Nitrile ester 14 is converted to the final product (18), a compound of the invention, by following the reaction conditions outlined in Scheme 1 above.

Scheme 6 depicts the preparation of 5-cyanobenzothiophene-type intermediates (A = S) represented by formula (6a) used to prepare the compounds of the invention:

Scheme 6

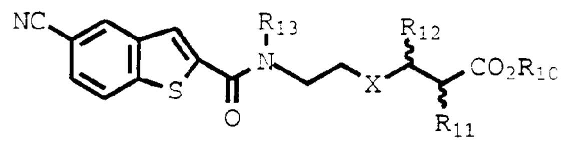

(6a)

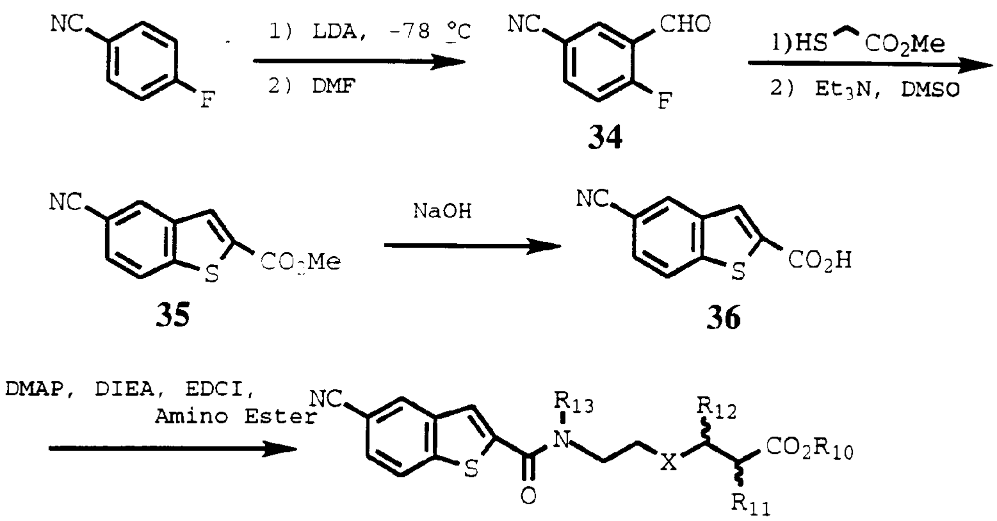


34

35

36

14

In Scheme 6, 4-fluorobenzonitrile is deprotonated using lithium diisopropylamide and the anion is quenched with DMF to afford aldehyde (34), which is further reacted with methylmercaptoacetate followed by triethylamine to effect addition and cyclization to give methyl-5-cyanobenzothiophene-2-carboxylate (35). Base hydrolysis (NaOH) of ester (35) gives the corresponding acid (36). Amide (14) is obtained by coupling acid (36) with the desired amino ester by using the three reagents, DMAP, DIEA and EDCI as described in the preceding Scheme.

In the preceding schemes, the final coupling step attaches an amino ester to a 5,6-bicyclic nucleus to give an intermediate used in preparing the compounds of the invention. The following reaction schemes give general methods for preparing the amino esters used in the preparation of the compounds of the invention.

Scheme 7 describes the synthesis of amino ester intermediates having oxygen substitution in the amine chain as represented by formula (7a) below:

Scheme 7

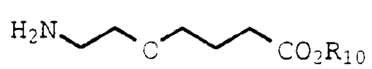

(7a)

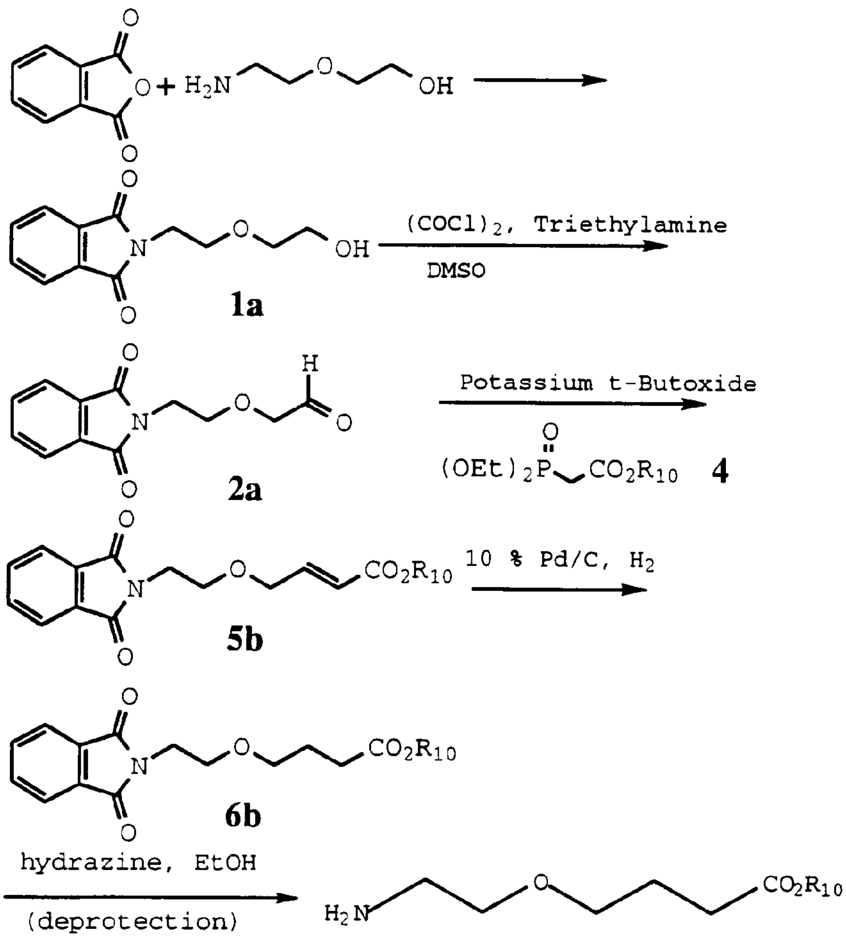


In Scheme 7, phthalic anhydride is reacted with 2-(2-aminoethoxy)ethanol to give the phthalimide (1a), which is then oxidized with oxalyl chloride, triethylamine, and DMSO (a Swern oxidation) to give aldehyde (2a). Product (2a) is converted to an α,β-unsaturated ester (5b) by a Horner-Emmons reaction. Ester (5b) is reduced by hydrogenation to the saturated ester (6b) which is thereafter deprotected with hydrazine to yield the amino ester reagent used for preparation of compound (14d - Scheme 3).

Scheme 8 describes the method of synthesizing amino esters with substituents alpha to the carboxyl group as represented by formula (8a) below, for use in the coupling reactions used in the final steps of preparing the compounds of the invention.

Scheme 8

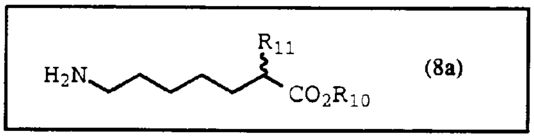

(8a)

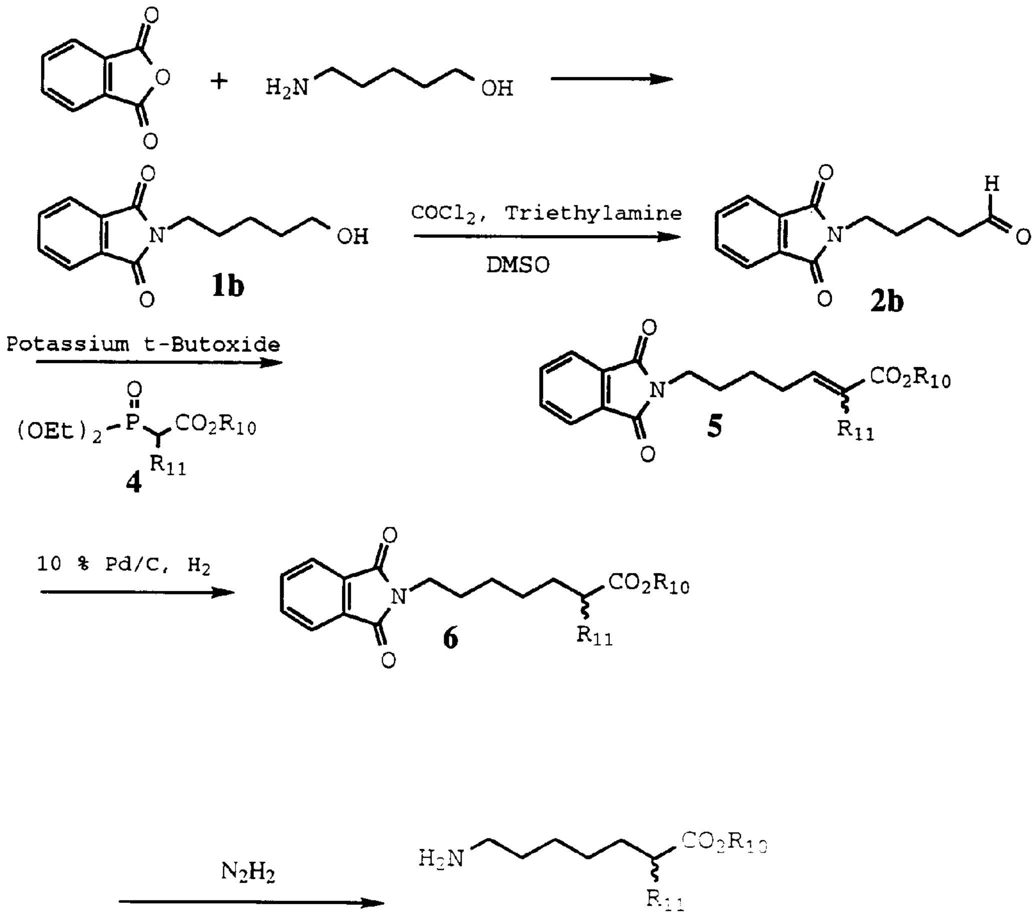


1b 2b 4 5 6 13

In Scheme 8, phthalic anhydride is reacted with 5-aminopentane-1-ol to give the phthalimide (1), which is oxidized with oxalyl chloride, triethylamine and DMSO (Swern oxidation) to give aldehyde (2). Compound (2) is converted to an α,β-unsacurated ester (5) by a Horner-Emmons reaction. Unsaturated ester (5) is reduced by hydrogenation to give the saturated ester (6) which is then deprotected with hydrazine to yield the amino ester product used for preparation of compounds (14).

Scheme 9 is a subscheme to Scheme 8 and provides details for the preparation of phosphonates (4) from the corresponding bromides as shown below:

Scheme 9

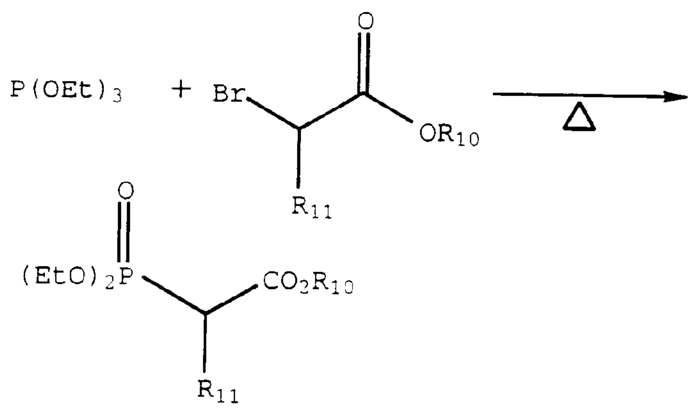

Scheme 10 describes the method of synthesizing amino esters with substituents beta to the carboxyl group as represented by formula 10a below used in the preparation of the compounds of the invention.

Scheme 10

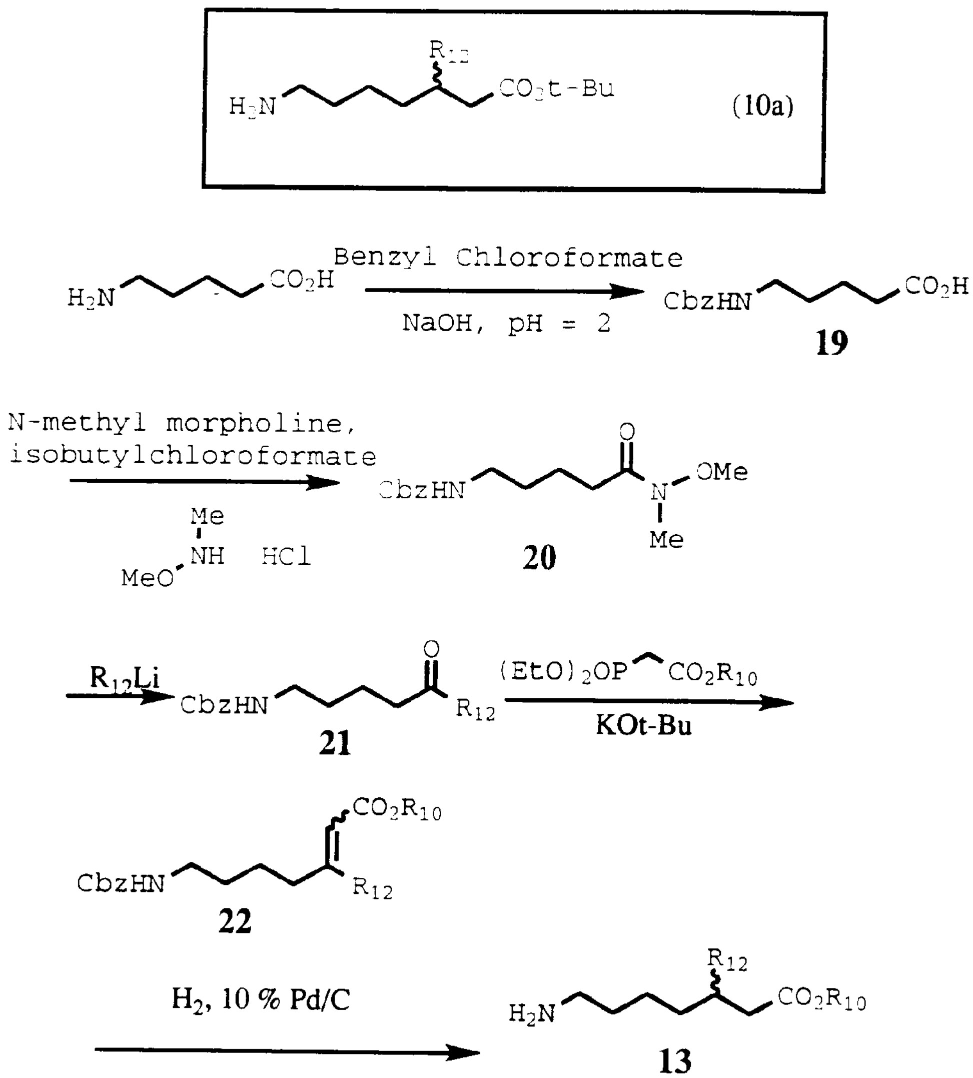


In Scheme 10, 5-aminopentanoic acid is reacted with benzyl chloroformate (to protect the amine functionality) and the resultant protected amine (19) is then converted to the Weinreb amide (20) which is thereafter reacted with an organolithium reagent to give a ketone (21). Ketone (21) is converted (by a Horner-Emmons reaction) to an α,β-unsaturated ester (22), which is then reduced by hydrogenation to yield the saturated β-substituted amino ester (13). Amino esters 13 are not purified but are used as isolated to couple to a suitable 6,5-nuclei as desribed in the previous schemes.

Scheme 11 shows a method of making the amino ester reactants in which the nitrogen is alkylated with an organic radical (non-hydrogen) for use in the coupling reactions used in the preparation of the compounds of the invention.

Scheme 11

N-Alkylated, b-Substituted Amino Esters

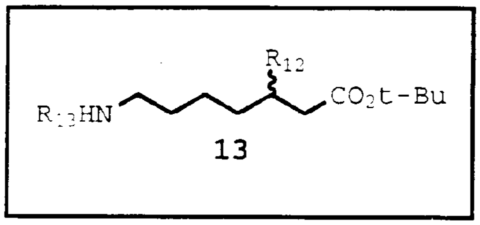

13

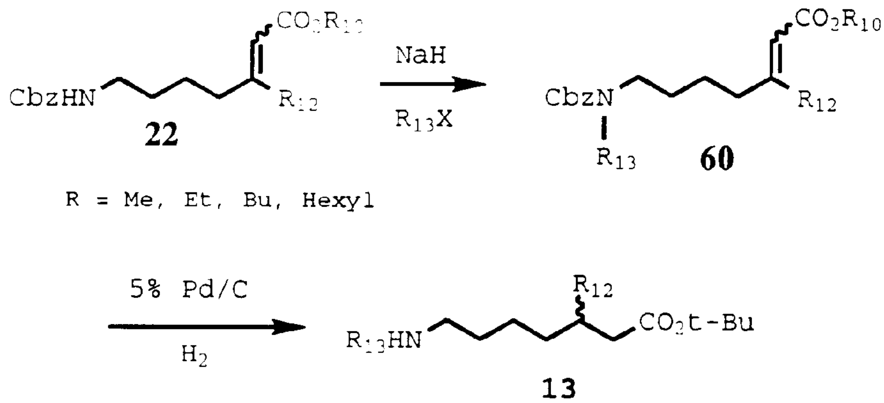


22 60 13

R = Me, Et, Bu, Hexyl

Protected amino ester (22) (from Scheme 10) is deprotonated using a suitable base and the resultant anion alkylated with an appropriate electrophile ($R_{13}X$) to afford the unsaturated ester (60) which is thereafter hydrogenated to give the saturated N-alkylated amino esters. Amino esters (13) are not purified but are used as isolated to couple to a suitable 6,5-nuclei as described in the previous schemes.

Scheme 12 describes a method of preparing benzyl- and cyclohexyl-containing amino esters, as represented by formula (12a) below, for use in the coupling reactions used in the final steps of preparing the compounds of the invention.

Scheme 12

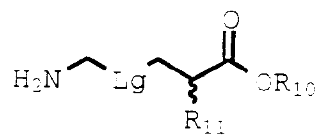

(12a)

Lg = phenyl or cyclohexyl

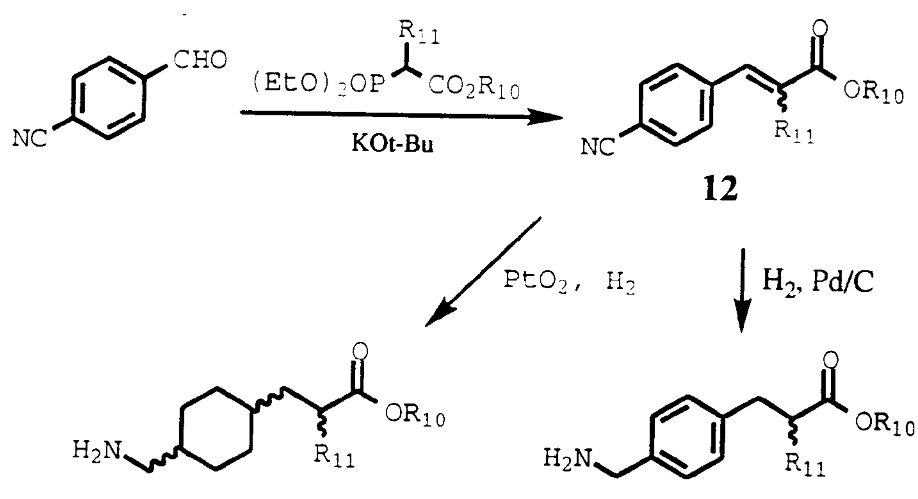


**13**

In Scheme 12, the starting material, 4-cyanobenzaldehyde, is converted (by a Horner-Emmons reaction) to give an α,β-unsaturated ester (12), which is then reduced by hydrogenation either over Pd/C or over $PtO_2$ to give the benzylamino ester or cyclohexylamino ester respectively. Amines (13) are not purified but are used as isolated to couple to a suitable 6,5-nuclei as described in the previous schemes.

Scheme 13 illustrates the preparation of compounds represented by formula (12a) which are used in the preparation of compounds having a modified B-($L_b$)- basic functionality, specifically, a benzyloxyamidino, as shown below:

Scheme 13

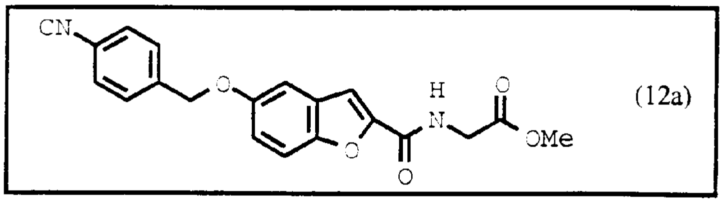

(12a)

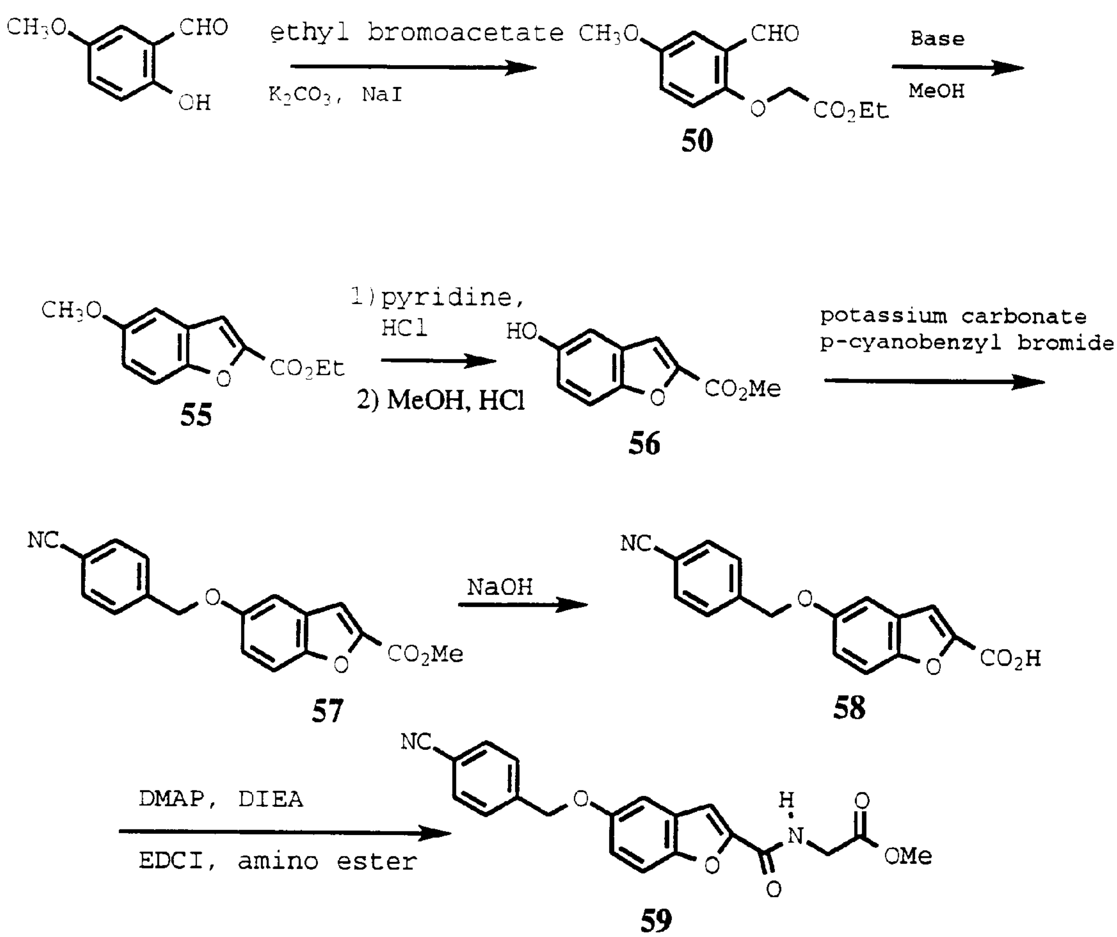


The starting material, 2-hydroxy-5-methoxybenzaldehyde, is alkylated with ethyl bromoacetate to give ester (50), which is thereafter subjected to base catalyzed cyclization to give ethyl- 5-methoxybenzofuran-2-carboxylate (55). This step is followed by deprotection of the methyl ester (55) with pyridine hydrochloride and reesterification of the crude product using methanoic HCl to give the hydroxybenzofuran (56), which is thereafter alkylated with p-cyanobenzylbromide to give cyanobenzyloxy substituted benzofuran (57). Benzofuran ester 57) is thereafter subjected to base hydrolysis to give the corresponding acid (58) which is then coupled with an amino ester (glycine) as previously shown. The nitrile ester (59) is converted to the amidino acid, a compound of the invention, by using the reaction conditions outlined in Scheme 1.

Scheme 14 illustrates the preparation of a compound having an amine functionality as its basic component (rather than the amidine group illustrated in the preceding Schemes).

Scheme 14

Aminomethyl Compounds

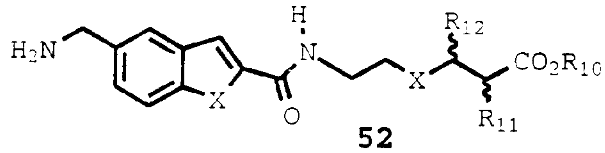

52

X = $(CH_2)_{0-2}$, $-OCH_2-$

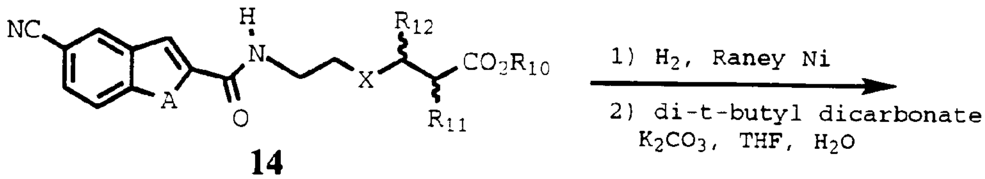


14

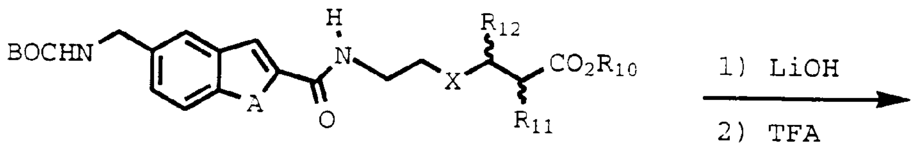


51

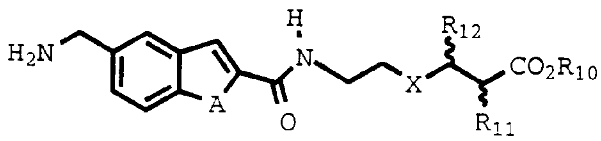

52

Synthesis begins with hydrogenation of nitrile (14) and subsequent protection of the primary amine using di-t-butyldicarbonate to afford a protected amine (51). The protected amino ester is hydrolyzed using LiOH and the Boc protecting group thereafter removed under acidic conditions to give a final product (52). The thioamide preparation step employed in the previous syntheses is not present since the amidine functionality is not formed.

Scheme 15 illustrates a synthesis where an organic radical (non-hydrogen) is placed on the indole nitrogen of the 5,6-bicyclic nucleus.

Scheme 15

N-Substituted Indoles

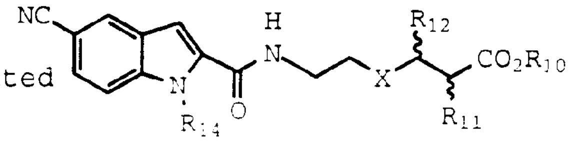

(14)

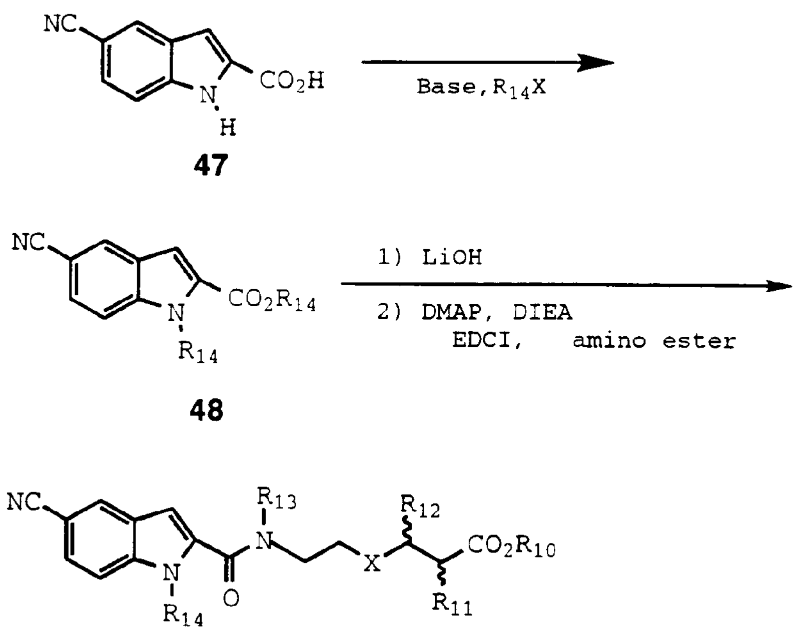


47

48

14

Scheme 15 begins with 5-cyanoindole-2-carboxylate (47) which is N-alkylated and esterified in a single step to give compound (48). Compound (48) is converted to the intermediate (14) and ultimately taken on to the final product as shown in previous schemes.

The following Examples (Nos. 1 to 36, excluding Nos. 7, 8, and 24) illustrate the practice of the invention, but are not intended to limit its scope.

Example 1

This Example teaches the preparation of a 5,6-bicyclic nucleus of the indole type for formula I with $X_1$ = N and which is appropriately functionalized to allow incorporation of B-($L_b$)- and A-($L_a$)- at positions $X_5$ and $X_2$ respectively:

Part A Preparation of the intermediate 4-nitro-3-tolunitrile, represented by the formula:

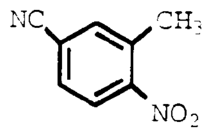

(45)

A mixture of $PCl_5$ (200 g, 0.96 mole) and p-toluene sulfonamide (90 g, 0.52 mole) was treated with 3-methyl-4-nitrobenzoic acid (82 g, 0.44 mole). The reaction was stirred at room temperature for 1 hour at which time it was heated to 140°C and the $POCl_3$ (150 ml) distilled from the mixture. The reaction was cooled to 5°C, pyridine (200 ml) was carefully added and the mixture allowed to stand overnight. The reaction was carefully diluted to 1 L with $H_2O$ and the mixture stirred for 3 hours. The brown solid was filtered, washed with $H_2O$ and triturated with 5 N aqueous NaOH. The remaining solid was filtered, washed with $H_2O$ and dried to afford 60.0 g of a powder. The solid was stirred in 1200 ml $CH_2Cl_2$ and filtered. The supernatant was evaporated in vacuo and the residue recrystallized from EtOH to give 35.9 g (23 %) of 4-nitro-3-tolunitrile.
MS(FD), m/e 162 (M+).

| Anal Calcd for $C_8H_6N_2O_2$: | | | |
|---|---|---|---|
| Calculated | C 59.26, | H 3.73, | N 17.28; |
| Found | C 59.25, | H 3.71, | N 17.41. |

Part B Preparation of the intermediate, ethyl-3-cyano-6-nitrotoluyloxalate (46) :

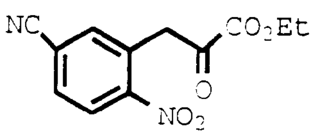

(46)

A solution of NaOEt, which was prepared by dissolving Na metal (7.7 g; 0.33 mole) in 400 ml EtOH, was added all at once to a solution of the 4-nitro-3-tolunitrile (32.0 g, 0.20 mole) (prepared in Part A) and freshly distilled diethyl oxalate (200 ml) in EtOH (240 ml). The reaction was stirred at room temperature for 20 hours, poured into 500 ml of 1 N aq. HCl, and diluted with 500 ml $H_2O$. This mixture was extracted twice with $CH_2Cl_2$ and the combined organic layers were washed once each with 1 N aqueous HCl and $H_2O$. The organic phase was dried over $Na_2SO_4$ and evaporated in vacuo to give 52.4 g of a solid. Column chromatography ($SiO_2$; $CH_2Cl_2$) afforded 42 g of crude product which was recrystallized from hexanes/EtOAc to give 22.1 g (43 %) of ethyl-3-cyano-6-nitrotoluyloxalate.
MS(FD), m/e 262 (M+).

| Anal Calcd for $C_{12}H_{10}N_2O_5$: | | | |
|---|---|---|---|
| Calculated | C 54.97, | H 3.84, | N 10.68; |
| Found | C 55.00, | H 3.77, | N 10.74. |

Part C Preparation of the intermediate, ethyl-5-cyanoindole-2-carboxylate (47):

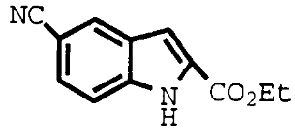

(47)

A suspension of ethyl-3-cyano-6-nitrotoluyloxalate (10.0 g, 38 mmol) (prepared in Part B) in 110 ml glacial acetic acid was treated with Zn dust (25.0 g, 38 mmol) in portions. After complete addition, the reaction was kept at 90°C for 15 minutes. The hot reaction was filtered and the supernatant allowed to cool. The precipitated product was collected by filtration to give 5.90 g (73 %) of the title compound. An analytical sample was obtained by recrystallization from EtOAc.
$^1H$ NMR (300 MHz; $CDCl_3$) d 9.30 (s, 1H), 8.08 (s, 1H), 7.53 (m, $^2H$), 7.30 (d, J = 2 Hz, 1H), 4.44 (q, J = 7 Hz, 2H), 1.43 (t, J = 7Hz, 3H).
MS(FD), m/e 214 (M+).

| Anal Calcd for $C_{12}H_{10}N_2O_2$: | | | |
|---|---|---|---|
| Calculated | C 67.28, | H 4.70, | N 13.08; |
| Found | C 67.28, | H 4.61, | N 13.01. |

Part D Preparation of 5-cyanolndole-2-carboxylic acid:

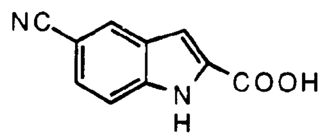

Ethyl 5-cyanoindole-2-carboxylate (16.5 g; 77.1 mmol) (prepared in Part C) was added to a solution of solid KOH (11.0 g, 200 mmol) in 110 ml $H_2O$ and 440 ml ethanol. The mixture was stirred for 5 hours at ambient temperature and filtered. The filter cake was resuspended in 1 N aqueous HCl and the mixture was stirred for 1 hour. The solid was filtered and dried to afford 8.45 g of 5-cyanoindole-2-carboxylic acid which was used without further purification.
FDMS 186 (M+)

Example 2

This example teaches the preparation of a 5,6-bicyclic nucleus of the indole type of formula I with $X_1$ = N and which is appropriately functionalized to allow for incorporation of B-($L_b$)- and A-($L_a$)- at positions $X_6$ and $X_2$ respectively:

Part A Preparation of the intermediate 2-nitro-4-toluinitrile (37):

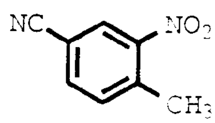

(37)

Compound (37) was prepared according to the method of JACS vol.99, page 6721 (1977). Concentrated

sulfuric acid (51 ml), cooled in an ice bath to 5°C, was treated dropwise with concentrated nitric acid (21 ml) while keeping the temperature below 15°C. To this was added p-tolunitrile (24 ml, .2 M) dropwise while keeping the temperature below 40°C. After stirring 10 minutes, a solid mass formed. Ice was added and the resulting mixture was extracted with $CH_2Cl_2$. The extracts were dried with $MgSO_4$ and concentrated to 32.13 g (99%) of a solid.

Part B Preparation of the intermediate ethyl-2-nitro-4-cyanotoluyl-oxalate (38):

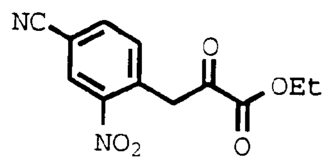

(38)

A mixture of 2-nitro-4-toluinitrile (prepared in Part A) (30.6 g, 188 mmol), diethyl oxalate (200 g) and EtOH (300 ml) was treated portionwise with a solution of sodium ethoxide freshly prepared from NaH (11.3 g, 283 mmol) and EtOH (300 ml). The reaction was stirred overnight at ambient temperature, neutralized with 5 N aqueous HCl (56.6 ml) and concentrated under reduced pressure. The resulting liquid was partitioned between $H_2O$ and $CH_2Cl_2$. After washing the organic layer with $H_2O$, it was dried with $MgSO_4$ and concentrated in vacuo to a solid
MS(FD), m/e 262 (M+).

Part C Preparation of the intermediate 6-cyanoindole-2-carboxylate (40):

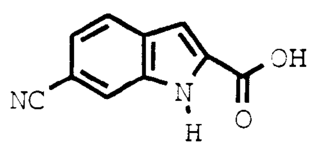

(40)

Nitro compound (38) (25g, 95 mmol) (prepared in Part B) was mixed with HOAc (275 ml) and heated to get homogenous solution. After adding $H_2O$ (275 ml) it was refluxed while adding zinc powder (62.4 g, 954 mmol) portionwise. Heating was continued for 1 hour and the mixture was filtered while hot. The filtrate was concentrated to a solid which was partitioned between EtOAc and $H_2O$. The organic layer was washed with saturated solution of $NaHCO_3$ several times, dried with $MgSO_4$ then concentrated to a solid which was purified by chromatrography ($SiO_2$, 5-10% v/v EtOAc/Hexane). 8.4 g of the material was recovered which by TLC ($SiO_2$, 30% v/v EtOAC/Hexanes) was two spots. The material was used as isolated.

The above solid material (7.9 g) was mixed with EtOH (200 ml) and 5 N aq. NaOH (22 ml). The reaction was refluxed for 3 hours, neutralized with 5 N aqueous HCl (22 ml) and concentrated in vacuo. The residue was partitioned between EtOAc and $H_2O$. After washing the organic layer with brine, it was dried with $MgSO_4$ and concentrated to dryness. The product was isolated by recrystallization from toluene/MeOH to give 3.1 g. MS(FD), m/e 186 (M+).

Example 3

This example teaches the preparation of 5,6-bicyclic nuclei of the indole type of formula I with $X_1$ = N,with a non-hydrogen substitution at the $X_3$ position, and which is appropriately functionalized to allow incorporation of B-($L_b$)- and A-($L_a$)- at positions $X_5$ and $X_2$ respectively:

Part A Preparation of the intermediate (61):

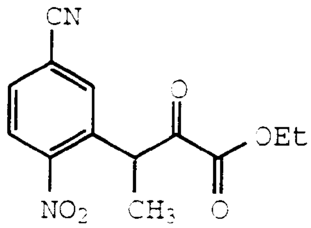

(61)

Sodium hydride (60% dispersion in oil; 3.44 g, 86 mmol) was washed with hexanes and dried under vacuum. Dry THF (250 ml) was added and cooled to 0°C. To this was added a solution of (46) (22.58 g, 86 mmol) (prepared in Part B, Example 1) in dry DMF portionwise. The reaction mixture was stirred at 0°C for 1 hour then methyl iodide (12.2 g, 86 mmol) added. This mixture was stirred at 0°C for 1 hour then at ambient temperature overnight. The reaction was acidified with 1 N aqueous HCl and diluted with EtOAc. The layers were separated and the organic layer washed with brine several times and dried over $MgSO_4$. The material was then concentrated under vacuum and purified by chromatography ($SiO_2$, 15 % v/v EtoAc-Hexanes) to recover 17.6 g (74%) of a yellow solid.

| Analysis, calculated for $C_{13}H_{12}N_2O_5$: | | | |
|---|---|---|---|
| Calculated: | C, 56.52 | H, 4.38 | N, 10.14 |
| Found: | C, 56.80 | H, 4.61, | N 10.32 |

Part B Preparation of the intermediate ethyl-3-methyl-5-cyanoindole-2-carboxylate (7b):

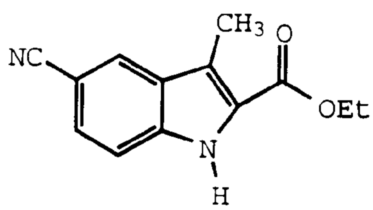

(7b)

The keto ester (61) (8.5 g,31 mmol) (prepared in Part A) was dissolved in glacial acetic acid (100 ml). Water was added (100 ml) and the mixture warmed to 75°C. Thereafter, zinc dust (20 g, .31 mol) was added portionwise. This mixture was stirred at 75°C for several hours, poured onto ice and extracted with EtOAC. The extracts were washed with brine several times, dried over $MgSO_4$ and concentrated under vacuum to dryness to obtain 6.7 g solid. The solid was recrystallized from a small amount of EtOAc to obtain an analytical sample.

| Analysis, calculated for $C_{13}H_{12}N_2O_2$ | | | |
|---|---|---|---|
| Calculated: | C, 68.41 | H, 5.30 | N, 12.27 |
| Found: | C, 68.42 | H, 5.04 | N 11.11 |

Part C Preparation of the intermediate 3-methyl-5-cyanoindole-2-carboxylate (62):

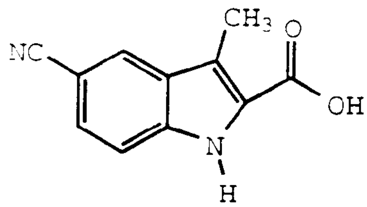

(62)

Ethyl-3-methyl-5-cyanoindole-2-carboxylate (prepared in Part B) (4.8g, 21 mmol) was mixed with MeOH (400 ml) and 1 M aqueous LiOH (52 ml). The mixture stirred overnight at ambient temperature and an additional 24 hours at 60°C before being neutralized with 5 N HCl (10.4 ml). The product was concentrated under vacuum and extracted with EtOAc. Extracts were dried over $MgSO_4$ and concentrated to dryness to recover 3.93 g (94%) of a solid.

| Analysis, calculated for $C_{11}H_8N_2O_2$ | | | |
|---|---|---|---|
| Calculated: | C, 66.00 | H, 4.03 | N, 13.99 |
| Found: | C, 65.75 | H, 4.18 | N 13.82 |

Example 4

This Example teaches the preparation of a 5,6-bicyclic nuclei of the indole type from formula I with $X_1$ = N with a non hydrogen substitution on the indole nitrogen and which is appropriately functionalized to allow incorporation of B-($L_b$)-and A-($L_a$)- at positions $X_5$ and $X_2$ respectively:

Part A Preparation of the intermediate methyl-1-methyl-5-cyanoindole-2-carboxylate (48):

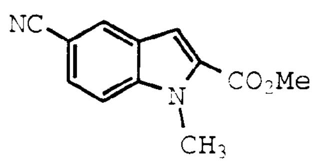

(48)

Powdered KOH (6.03 g, 107.5 mmol) was added to 40 ml DMSO and the mixture stirred for 0.5 hours. 5-Cyanoindole-2-carboxylate (5.0 g; 27 mmol) (prepared in Example 1 Part D) was added and the mixture stirred for 15 minutes before MeI (7.0 ml, 112 mmol) was added dropwise. The reaction was stirred at room temperature for 16 hours and was added to 250 ml $H_2O$. The precipitate was filtered, dried and recrystallized to give 4.50 g (78 %) of the title compound as a white solid.
MS(FD), m/e 214 (M+).

| Anal Calcd for $C_{12}H_{10}N_2O_2$: | | | |
|---|---|---|---|
| Calculated | C 67.28, | H 4.70, | N 13.08; |
| Found | C 67.38, | H 4.79, | N 13.33. |

Example 5

This Example teaches the preparation of 5,6-bicyclic nuclei of the benzothiophene type of formula I with

$X_1$ = S and which is appropriately functionalized to allow incorporation of B-($L_b$)-and A-($L_a$)- in positions $X_5$ and $X_2$ respectively:

Part A Preparation of methyl-5-cyanobenzothiophene-2-carboxylate (35):

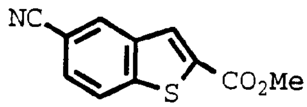

(35)

The compound was prepared according to the procedure described in (Tetrahedron Letters Volume 33, Number 49, pg 7499, 1992.

To a mixture of freshly distilled diisopropylamine (11.6 g, 0.114 M) and dry THF (200 ml) at -10°C under an atmosphere of argon was added 2.5 M n-BuLi (42 ml) dropwise keeping the temperature below -5°C. The mixture was stirred at -10°C for an additional 10 minutes. After recooling to -78°C it was treated with a solution of 4-fluorobenzonitrile in THF (30 ml). The resultant mixture was stirred for an additional 75 minutes at -78°C before adding DMF (7.6 g, 0.103 M). After stirring at -78°C for 15 minutes, the reaction was quenched with acetic acid 20 ml), and diluted with ethyl ether (200 ml). The organic layer was washed with 1 N aq. HCl twice then water before drying with $MgSO_4$ and concentrating in vacuo to 15.7 g of an oil.

The above oil (13.3g, 89 mmol) was combined with methyl thioglycolate (11.4 g, 107 mmol) and dry DMSO (50 ml). The mixture was stirred under $N_2$ at ambient temperature while adding triethylamine (19.9 g, 196 mmol) dropwise to obtain a dark solution. The reaction was heated at 95°C for 2 hours, poured into a mixture of $H_2O$ and celite, extracted with EtOAc, washed with $H_2O$, dried with $MgSO_4$ and decolorized with activated charcoal. Concentration gave 8.02 g (41 %) of a tan solid.

| Analysis, Calculated for $C_{11}H_7NSO_2$ | | | |
|---|---|---|---|
| Calculated: | C, 60.82 | H, 3.23 | N, 6.45 |
| Found: | C, 60.58 | H, 3.23 | N, 6.23 |

Part B Preparation of 5-cyanobenzothiophene-2-carboxylic acid (36):

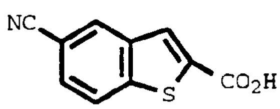

(36)

Combined the benzothiophene ester (35) (8.42 g, 39 mmol), EtOH (75 ml) and 5 N aq. NaOH (16 ml). Stirred overnight at ambient temperature then quenched with 5 N aq. HCl (16 ml). Concentrated under reduced pressure to a solid which was rinsed with $H_2O$ to give 7.8 g (98%) of a tan solid.

FDMS m/e 203 ($M^{+}$)

Example 6

This Example teaches the preparation of 5,6-bicyclic nuclei of the benzofuran type in formula I with $X_1$ = O and which is appropriately substituted to allow for incorporation of B-($L_b$)-and A-($L_a$)- at positions $X_5$ and $X_2$, respectively:

Part A Preparation of the intermediate methyl 4'-bromo-2'-formyl phenoxy) acetate (30):

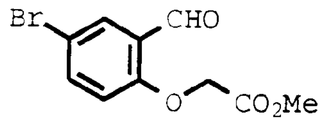

(30)

A slurry of $K_2CO_3$ (1.52 g, 11.0 mmol) and NaI (0.30 g, 2.0 mmol) in 3 ml of DMF was treated with 5-bromosalicylaldehyde (2.01 g, 10.0 mmol) followed by ethyl bromoacetate (1.84 g., 11.0 mmol). The reaction was stirred for 15 hours and was concentrated in vacuo. The residue was taken up in 50 ml EtOAc and was washed with $H_2O$ (3 x 25 ml). The organic layer was dried over $Na_2SO_4$ and evaporated in vacuo to give 3.25 g of a viscous oil which was purified by flash chromatography ($SiO_2$; 10 % EtOAc/Hexane) to afford 2.90 g (99 %) of the title compound (30).
MS(FD), m/e 286 (M+), 288.

| Anal Calcd for $C_{11}H_{11}BrO_4$: | | |
|---|---|---|
| Calculated | C 46.02, | H 3.86; |
| Found | C 46.21, | H 3.91. |

Part B Preparation of the intermediate methyl 5-bromobenzofuran-2-carboxylate (31):

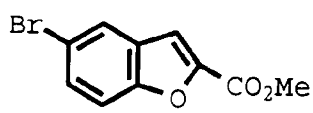

(31)

A solution of (4-bromo-2-formylphenoxy)-acetic ester (300 mg, 1.04 mmol) (prepared in Part A) in 8 ml of MeOH at 0°C was treated with of NaOMe (75 mg, 1.4 mmol) and the mixture heated to reflux for 15 hours. The reaction was cooled and treated with 5 ml of 1 N aq citric acid. The organic solvent was evaporated in vacuo, and the aqueous mixture extracted with EtOAc (3 x 20 ml). The combined organic extracts were dried over $Na_2SO_4$ and evaporated to give 285 mg of a white solid. The solid was taken up in MeOH and the solution treated with a stream of HCl(g). The reaction was stirred at room temperature for 2 hours and was evaporated in vacuo to give 230 mg of a solid which was recrystallized from Hexane/EtOAc to afford 195 mg (74 %) of the title compound.
MS(FD), m/e 254 (M+), 256.

| Anal Calcd. for $C_{10}H_7BrO_3$: | | |
|---|---|---|
| Calculated | C 47.09, | H 2.77; |
| Found | C 47.24, | H 2.73. |

Part C Preparation of the intermediate methyl 5-cyanobenzofuran-2-carboxylate:

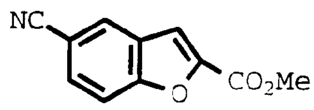

(32)

A slurry of methyl-5-bromobenzofuran-2-carboxylate (700 mg, 2.74 mmol) (prepared in Part B), CuI (525 mg, 2.76 mmol) and of CuCN (500 mg, 5.6 mmol) in 10 ml DMF was heated to 155°C for 16 hours. The reaction was cooled, filtered through celite and evaporated *in vacuo*. The residue was partitioned between $H_2O$ and EtOAc (25 ml each). The organic layer was separated, dried over $Na_2SO_4$ and evaporated to give 755 mg of a yellow solid. The solid was recrystallized from Hexane/EtOAc to afford 495 mg (90 %) of the title compound.

Part D Preparation the intermediate 5-cyanobenzofuran-2-carboxylate (33):

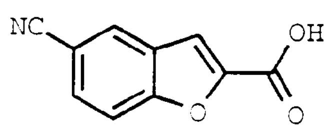

(33)

Methyl ester (32) (6.6 g, 33 mmol) was mixed with MeOH (500 ml). To this was added a solution of LiOH (1.6 g, 66 mmol) in $H_2O$ (160 ml). The mixture was stirred overnight at ambient temperature, then neutralized with 5 N aqueous HCl (13.2 ml). The resultant solid was concentrated under reduced pressure and partitioned between $H_2O$ and EtOAc. The organic layer was dried with $MgSO_4$ and concentrated to solid which was recrystallized from MeOH to recover 3.2 g (52%) solid.

Example 9

Preparation of the compound of the invention represented by the formula (18a):

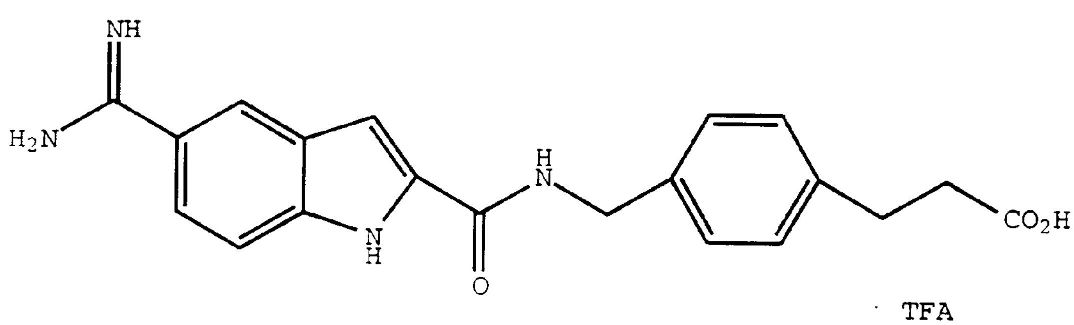

(18a)

Part A Preparation of the nitrile intermediate (12a):

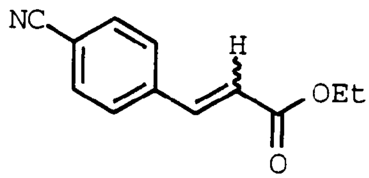

(12a)

A solution of 4-cyanobenzaldehyde (14.62 g, 111.5 mmol) and triethylphosphonoacetate (25 g, 111.5 mmol) in 300 ml of dry THF was cooled to 0°C. To this solution was added potassium tert-butoxide (12.5 g, 111.5 mmol) portionwise while continuing to cool. The cooling bath was removed and the reaction stirred at ambient temperature for 16 hours. The reaction was quenched with brine and extracted with EtOAc. The extracts were washed with $H_2O$ and dried with $MgSO_4$. After concentration in vacuo to an amber oil, the product was isolated by chromatography ($SiO_2$, 2 % v/v EtOAc/Hexane) to yield 14.52 g (65 %) solid.

Part B Preparation of the amino ester intermediate, (13a):

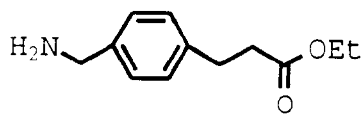

(13a)

A mixture of unsaturated ester (12a) (5.0 g, 24.9 mmol) (prepared in Part A), EtOH (100 ml) and HOAc (25 ml) was hydrogenated with Pd/C 5% (.8 g) for 2 hours. The reaction was filtered through celite and concentrated. The resulting oil was partitioned between saturated aqueous $NaHCO_3$ and EtOAc to obtain a precipitate which was filtered to give 2.5 g (48%) of a white solid.

Part C Preparation of the nitrile ester intermediate (14a):

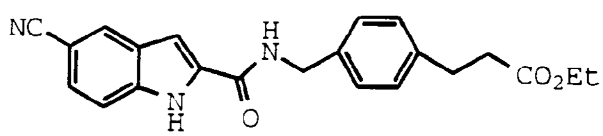

(14a)

A mixture of the amino ester (13a) (2.4 g, 11.6 mmol) (prepared in Part B), 4-dimethylaminopyridine (2.1 g, 17.4 mmol), diisopropylethylamine (4.5 g, 34.8 mmol), 5-cyano-2-indolecarboxylic acid (2.2 g, 11.6 mmol) (Part D, Example 1) and $CH_2Cl_2$ (100 ml) was stirred at ambient temperature. To this was added 1-(3-dimethylaminopropyl)-3-ethylcarbodiimide hydrochloride (3.3 g, 17.4 mmol). The reaction was stirred at ambient temperature overnight (16 hours). After adding THF (50 ml) the mixture was washed with 1M aqueous citric acid in $H_2O$ and saturated aqueous $NaHCO_3$. The organic layer was dried with $MgSO_4$ and concentrated to a white solid which was purified by chromatography ($SiO_2$, 1 % v/v MeOH/$CHCl_3$) to yield 2.97 g (68 %) white solid.

m.p. 191-192°C FDMS 375 (M+)

| Analysis, Calculated for $C_{22}H_{21}N_3O_3$ | | | |
|---|---|---|---|
| Calculated: | C, 70.38 | H, 5.64 | N, 11.19 |
| Found: | C, 70.65 | H, 5.69 | N, 11.12 |

Part D Preparation of the thioamide intermediate (15a):

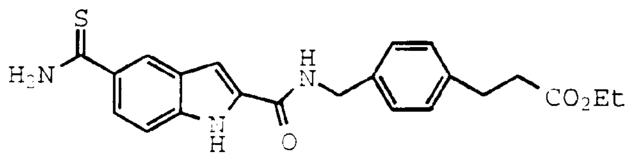

(15a)

A solution of (14a) (1.0 g, 2.7 mmol) (product of Part C) in dry pyridine (30 ml) and $Et_3N$ (3.2 ml) was stirred at ambient temperature while bubbling $H_2S$ gas into it for 15 minutes. Stirring was continued for 3 days. The product was concentrated to a solid, mixed with toluene and reconcentrated to a solid.

Part E Preparation of the protected aminine intermediate (16a):

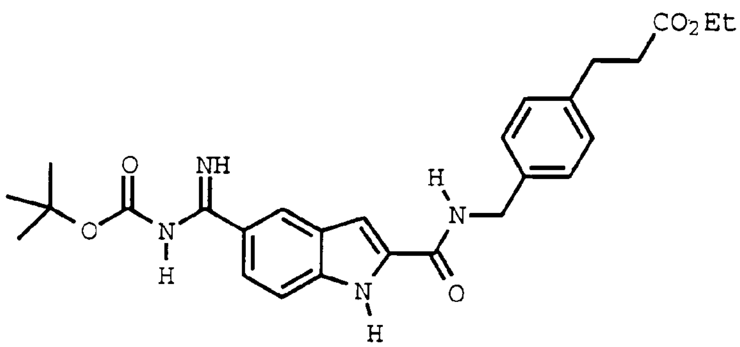

(16a)

Compound (15a) (2.7 mmol) (prepared in Part D) was mixed with acetone (30 ml) and methyl iodide (excess). The reaction mixture was refluxed for 30 minutes, then concentrated to dryness under reduced pressure. Anhydrous ammonium acetate (.9 g, 11.7 mmol) and MeOH (20 ml) was added and the mixture refluxed overnight (16 hours.). The reaction mixture was concentrated in vacuo, then treated with di-tert-butyl dicarbonate (7 g, 32 mmol), potassium carbonate (4.4 g, 32 mmol), THF, (15) and $H_2O$ (15 ml). The mixture was stirred at ambient temperature for 2 hours. The reaction mixture was partitioned between water and EtOAc. The organic layer was dried with $MgSO_4$ and concentrated under reduced pressure to yield an oil which was purified by chromatography ($SiO_2$, 5 % v/v MeOH/$CHCl_3$) to yield 1 g (75 %) yellow oil.

Part F Preparation of the acid intermediate (17a):

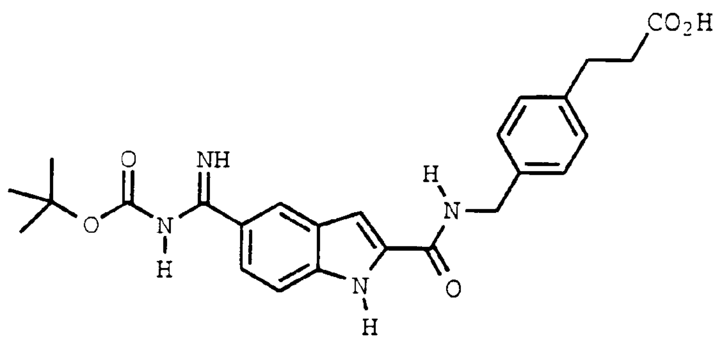

(17a)

Compound (16a) (1.0 g, 2 mmol) was mixed with EtOH (40 ml) and 5 N aqueous NaOH (2 ml). The reaction was stirred at ambient temperature overnight (16 hours.). Glacial acetic acid (10 mmol) was added and the reaction mixture was concentrated to solid which was mixed with $H_2O$ and EtOAc. The mixture was filtered to obtain .5 g (54 %) solid.

Part G Preparation of amidino acid product (18a):

The product of part F, compound (17a), (0.1 g, 0.2 mmol) was mixed with anhydrous anisole (1 ml) and trifluoroacetic acid (2 ml). The reaction was stirred at ambient temperature for 1 hour. After concentrating under reduced pressure the resulting solid was triturated with a mixture of hot EtOAc and MeOH (3:1) to recover 96 mg (95 %) of the title compound as the trifluoroacetate salt.

$^1$H NMR (DMSO/TMS): ∂ = 2.2-2.4 (m,2H), 2.7-2.8 (m, 2H), 4.5 (d,2H) 7.2-7.3 (m, 4H), 7.4 (s, 1H), 7.5-7.7 (m, 2H), 8.2 (s, 1H), 8.8 (s,2H), 9.1-9.2 (m, 3H), 12.1-12.2 (m, 2H)

| Analysis, Calculated for $C_{22}H_{21}F_3N_4O_5$• 0.6 $H_2O$, 0.2 EtOAc | | | |
|---|---|---|---|
| Calculated: | C, 54.03 | H, 4.73 | N, 11.05 |
| Found: | C, 53.88 | H, 4.38 | N, 10.68 |

Example 10

Preparation of the indole-type compound of the invention represented by the formula (18b):

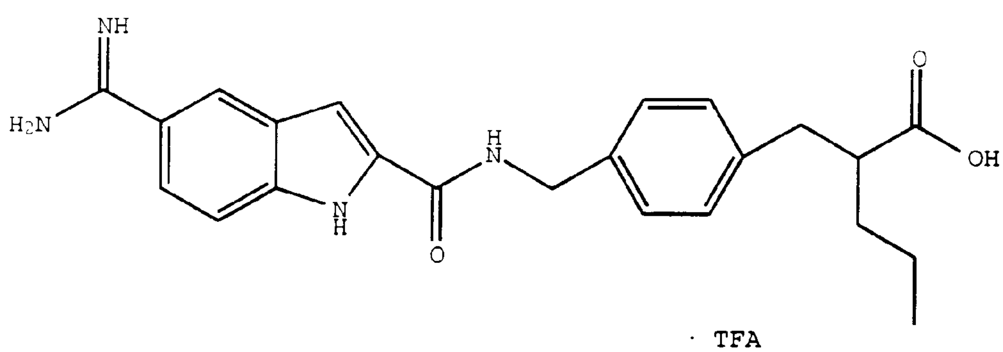

· TFA

(18b)

Part A Preparation of the intermediate (12b):

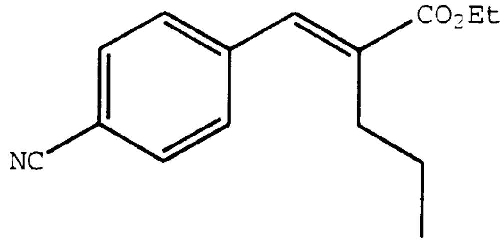

(12b)

The intermediate (12b) was prepared by the method of (12a) (from example 9, Part A) using triethyl-2-phosphonopentanoate in place of triethylphosphonoacetate.

Part B Preparation of the intermediate (13b):

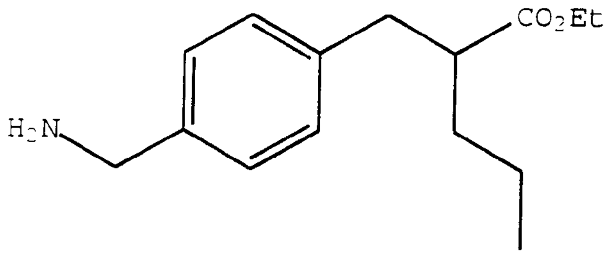

(13b)

This intermediate was prepared according to the method of Example 9, Part B (13a) using as starting material the product of Part A of this Example. The product was purified by chromatography ($SiO_2$, 5-10% v/v $CHCl_3$/MeOH) to recover a 61% yield of analytically pure material.
FDMS 250 ($M^{+1}$)

| Analysis, Calculated for $C_{15}H_{23}NO_2$• .1 $CHCl_3$ | | | |
|---|---|---|---|
| Calculated: | C, 69.41 | H, 8.91 | N, 5.36 |
| Found: | C, 69.64 | H, 8.64 | N, 5.50 |

Part C Preparation of the intermediate (14b):

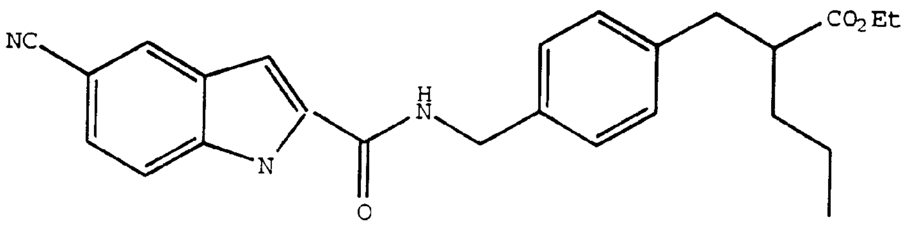

(14b)

The intermediate was prepared using the method of (14a) (per Example 9, Part C) starting from (13b) (prepared in Part B of this Example) in a 57% yield. An analytically pure sample was obtained by recrystallization from MeOH. m.p. 141-142°C. FDMS 417 (M+)

| Analysis, Calculated for $C_{25}H_{27}N_3O_3$ | | | |
|---|---|---|---|
| Calculated: | C, 71.92 | H, 6.52 | N, 10.06 |
| Found: | C, 71.86 | H, 6.53 | N, 10.26 |

Part D Preparation of the intermediate (15b):

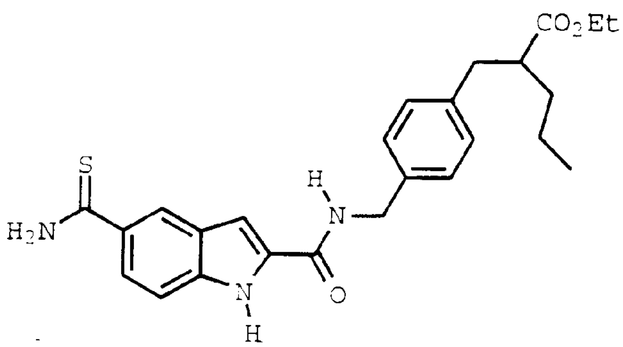

(15b)

This intermediate was prepared by the method of Example 9, Part D (15a) in a quantitative yield using (14b) (prepared in Part C) as starting material in place of (14a). FDMS 451 (M+)

| Analysis, Calculated for $C_{25}H_{29}N_3SO_3$ | | | |
|---|---|---|---|
| Calculated: | C, 66.49 | H, 6.47 | N, 9.31 |
| Found: | C, 66.28 | H, 6.69 | N, 9.39 |

Part E Preparation of the intermediate (16b):

This intermediate was prepared by the method of Example 9, Part E (16a) in a 43% yield using (15b) as starting material in place of (15a).
FDMS 535 (M+1)

| Analysis, Calculated for $C_{30}H_{38}N_4O_5$•0.14 $CHCl_3$ | | | |
|---|---|---|---|
| Calculated: | C, 65.66 | H, 6.97 | N, 10.16 |
| Found: | C, 65.71 | H, 6.72 | N, 10.10 |

Part F Preparation of the intermediate (17b):

This intermediate was prepared by the method of Example 9, Part F (17a) using as starting material the product of Part E (16b) in place of (16a). The yield was 71% after purification by chromatography ($SiO_2$, 10 v/v MeOH/$CHCl_3$).

Part G Preparation of the title compound (18b):

The title compound of the invention was prepared using the method of Example 9, Part G (18a) using as starting material the product of (17b) in place of (17a) to give a 70% yield of analytically pure material as the trifluoroacetate salt. FDMS 365 $^{(M+1)}$
$^1$H NMR (DMSO/TMS) : ∂ = 0.8-0.9 (m,3H), 1.2-1.5 (m, 4H), 2.4-2.6 (m, 1H), 2.6-2.9 (m,2H), 4.0 (d, 2H), 7.1-7.3 (m, 4H), 7.4 (s, 1H), 7.5-7.7 (m, 2H), 8.2 (s,1H) , 8.8 (bs, 2H), 9.1-9.2 (m, 3H), 12.0-12.2 (m, 2H)

| Analysis, Calculated for $C_{25}H_{27}F_3N_4O_5$ | | | |
|---|---|---|---|
| Calculated: | C, 57.69 | H, 5.23 | N, 10.76 |
| Found: | C, 57.95 | H, 5.28 | N, 10.92 |

Example 11

Preparation of Compound (18c):

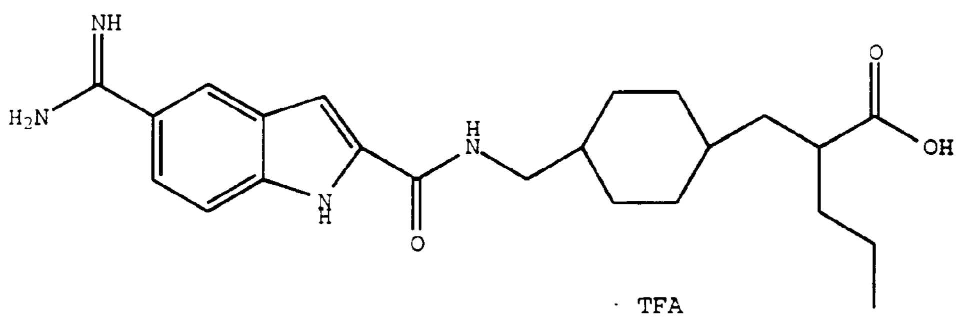

(18c)

Part A Preparation of the intermediate (13c):

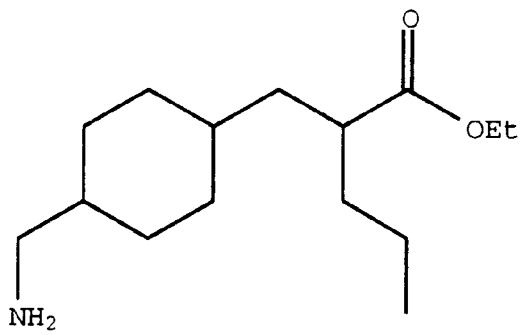

(13c)

Intermediate 13c was prepared using the method described for the synthesis of 13a, (Example 9, Part B) except that hydrogenation of the starting material (12b) (of Example 10, Part A) was conducted using $PtO_2$ catalyst for 16 hours.

Part B Preparation of the Intermediate (14c):

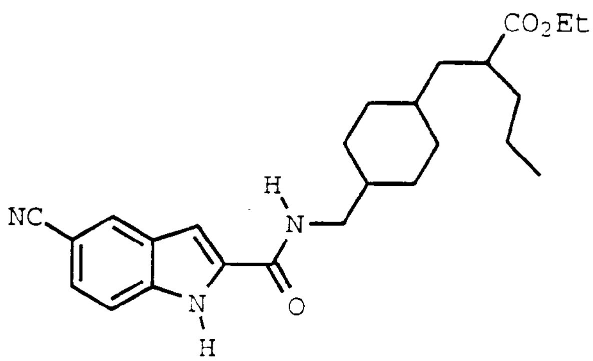

(14c)

Intermediate (14c) was prepared in 59% yield starting from (13c) (Part A of this Example) by using the method described in Example 9, Part C.

Part C Preparation of the intermediate (15c):

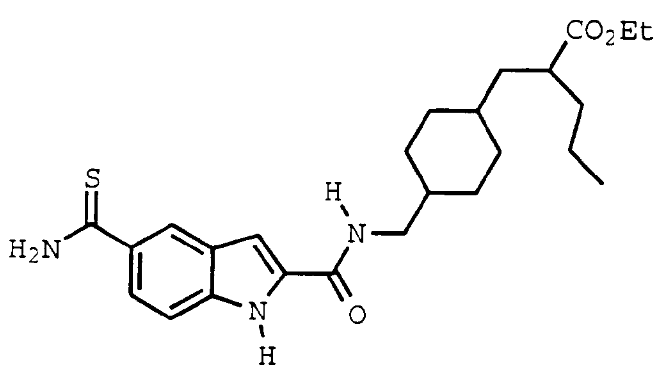

(15c)

Intermediate (15c) was prepared in 98% yield starting from (14c) (from Part B of this Example) by using the method described in Example 9, Part D.
FDMS 457 (M+)

Part D Preparation of the intermediate (16c):

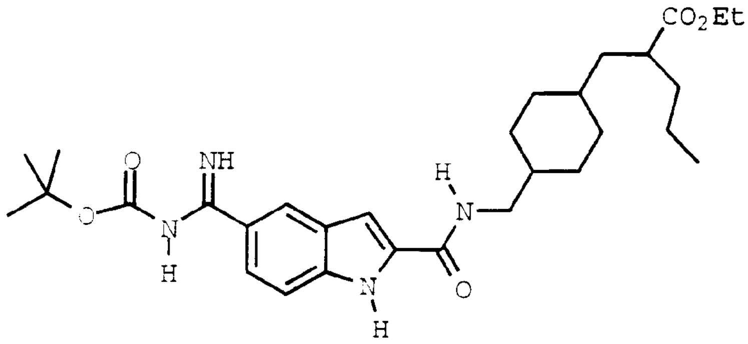

(16c)

Intermediate (16c) was prepared according to the method of Example 9, Part E (16a) in a 43% yield using as starting material (15c) (prepared in Part C of this Example) in place of (15a) . FDMS 541 (M+1)

| Analysis, Calculated for $C_{30}H_{44}N_4O_5$•.1 $CHCl_3$ | | | |
|---|---|---|---|
| Calculated: | C, 65.42 | H, 8.04 | N, 10.14 |
| Found: | C, 65.61 | H, 8.34 | N, 10.09 |

Part E Preparation of the intermediate (17c):

The intermediate was prepared using the method of Example 9, Part F (17a) using as starting material (16c) (prepared in Part D this Example). Yield was 14% after purification by chromatography ($SiO_2$, 5:1 v/v MeOH/HOAc/$CHCl_3$).

Part F Preparation of the title compound (18c):

The title compound was prepared as the trifluoroacetate salt in a 39% yield by the method of Example 9, part G (18a) using as starting material the product of Part E (17c) of this Example in place of (17a).
$^1$H NMR (DMSO/TMS): ∂ = 0.7-0.9 (m,3H), 1.1-1.5 (m, 14H), 1.6-1.8 (m, 2H), 2.2-2.3 (m, 1H), 3.0-3.3 (m,2H), 7.3 (s, 1H),7.5-7.6 (m, 2H), 8.2 (s, 1H), 8.6 (t, 1H), 8.9 (bs,2H), 9.1 (bs, 2H), 12.0-12.2 (m, 2H)

Example 12

Preparation of amidino acid (18d):

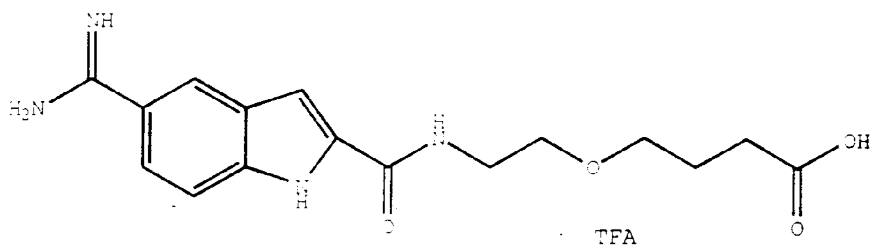

(18d)

Part A Preparation of pthaloimido ether (1a):

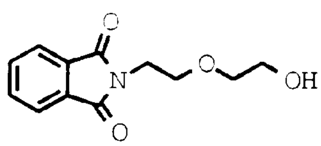

(1a)

Phthalic anhydride (10 g, 67.5 mmol) was combined with 2(2-aminoethoxy)ethanol and heated at 130°C overnight (16 hours). The resulting oil was treated with toluene, concentrated under reduced pressure to remove water and used as isolated.

Part B Preparation of the intermediate represented by the formula (2a):

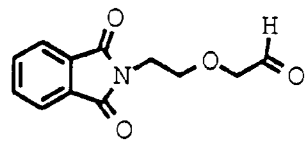

(2a)

A cooled mixture of oxalyl chloride (12.85 g, 101 mmol) and $CH_2Cl_2$ (100 ml) at -78°C under an atmosphere of $N_2$ was treated with a solution of DMSO ( 13.18 g, 169 mmol) in $CH_2Cl_2$ (50 ml) dropwise. The reaction was stirred at -78°C for 15 min. before adding a solution of alcohol (1a) (15.9 g, 67.5 mmol) (prepared in Part A) in $CH_2Cl_2$ (50 ml) dropwise. Stirring was continued for an additional 10 minutes before adding $Et_3N$ (34 g, 337.5 mmol) in one portion. The cooling bach was removed and the temperature was allowed to reach 0°C. Water was added and the organic layer was washed with a solution of NaOCl, then $H_2O$. The reaction mixture was dried over $MgSO_4$ and concentrated under reduced pressure to give 12.45 g (81 %) of an oil.

Part C Preparation of intermediate (5b):

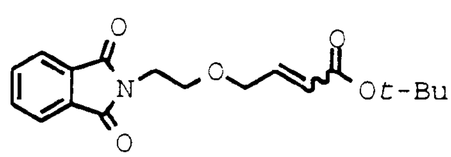

(5b)

Sodium hydride (60 % oil dispersion; 3.4 g, 85 mmol) was washed with hexane under an atmosphere of argon, mixed with dry THF (150 ml) and cooled to 0°C. The resulting suspension was treated with t-butyl diethylphosphonoacetate (21.4 g, 85 mmol) portion wise while keeping the temperature below 10°C. The reaction was stirred at 0°C until gas evolution stopped. The reaction mixture was then treated with a solution of aldehyde (2a) (85 mmol) (prepared in Part B of this Example) in THF (50 ml) while keeping the temperature below 10%. The cooling bath was removed and the mixture was stirred at ambient temperature until the reaction was over as evident by TLC ($SiO_2$, 30 % v/v EtOAc-Hexane). The reaction mixture was quenched with a saturated aqueous $NH_4Cl$ solution and extracted with EtOAc. The organic layer was dried with $MgSO_4$ and concentrated under reduced pressure to an oil which was purified by chromatography ($SiO_2$, 15% v/v EtOAc/Hexane) to obtain 15 g of a solid.

Part D Preparation of the intermediate (6b)

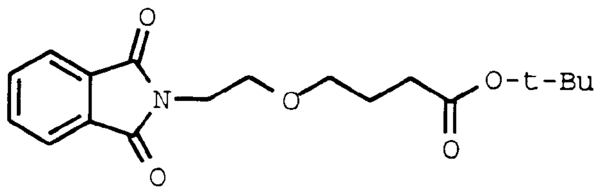

(6b)

Unsaturated tert-butyl ester (5b) (15 g, 45 mmol) (prepared in Part C of this Example) was mixed with EtOAc (100 ml) and hydrogenated with 5.5 g 10% Pd/C at an initial pressure of 40 psi (2.81 $Kg/cm^2$) for 8 hours. The reaction mixture was filtered through a pad of celite and concentrated under reduced pressure to yield (6b).

Part E Preparation of the intermediate (14d):

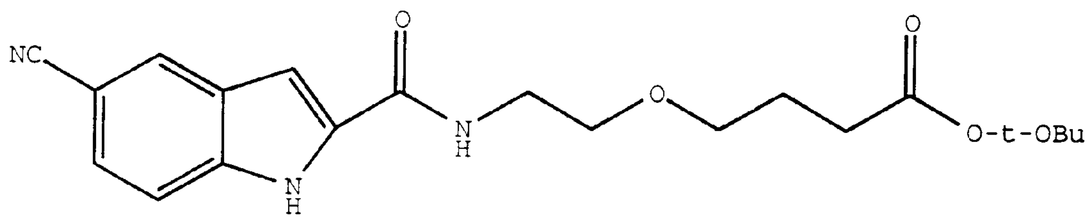

(14d)

Ester (6b) (3.0 g, 9.0 mmol) (prepared in Part D this Example) was mixed with EtOH (100 ml), hydrazine monohydrate (3 ml) and celite (10 g). The mixture was refluxed for 1 hour, cooled to 0°C and filtered through a pad of celite. The residue after concentration in vacuo reduced pressure was mixed with $CH_2Cl_2$, refiltered through celite and reconcentrated to a colorless oil (1.88 g) which was directly coupled to 5-cyanoindole-2-carboxylate in 75% yield (2 steps) by following the procedure described in Example 9, Part C. FDMS 371 (M+)

| Analysis, Calculated for $C_{20}H_{25}N_3O_4$ | | | |
|---|---|---|---|
| Calculated: | C, 64.67 | H, 6.78 | N, 11.31 |
| Found: | C, 64.47 | H, 6.88 | N, 11.31 |

Part F Preparation of the intermediate (15d):

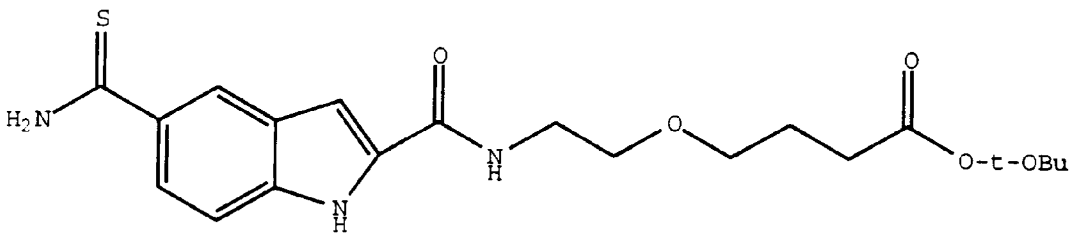

(15d)

A solution of (14d) (2.4 g, 6.5 mmol) (prepared in Part D) in dry pyridine (90 ml) and $Et_3N$ (10 ml) was stirred at ambient temperature while bubbling $H_2S$ gas into it for 10 minutes. Stirring was continued for 6 days. The reaction mixture was concentrated to a solid, mixed with toluene and reconcentrated to a solid. The product was then triturated with hot EtOAc and the remaining solid recrystallized from EtOH to recover 1.65 g (62%) yellow crystals.
FDMS 405 (M+)

| Analysis, Calculated for $C_{20}H_{27}N_3SO_4$ | | | |
|---|---|---|---|
| Calculated: | C, 59.24 | H, 6.71 | N, 10.36 |
| Found: | C, 59.02 | H, 6.73 | N, 10.28 |

Part G Preparation of the intermediate, (16d)

The product of Part F, (15d) (1.4 g, 3.4 mmol) was combined with methyl iodide (4.9 g, 34.5 mmol) and acetone (50 ml), refluxed for 1 hour, then concentrated to dryness under reduced pressure. Anhydrous ammonium acetate (1.1 g, 13.8 mmol) and dry MeOH (30 ml) were added. The resulting mixture was refluxed for an additional hour, then concentrated in vacuo to an oil which was mixed with THF and concentrated to a solid. Potassium carbonate (3.8 g, 27.6 mmol), di-tert-butyl dicarbonate (6 g, 27.6 mmol), THF (25) and $H_2O$ (25 ml) were added. The mixture was stirred at ambient temperature for 2 hours, quenched with $H_2O$ and extracted with EtOAc. The organic layer was dried with $MgSO_4$ and concentrated to an oil which was purified by chromatography ($SiO_2$, 2 % v/v MeOH/$CHCl_3$) to yield 0.833 g (50 %) of a foam. FDMS 489 $^{(M+1)}$

| Analysis, Calculated for $C_{25}H_{36}N_4O_6$ | | | |
|---|---|---|---|
| Calculated: | C, 61.46 | H, 7.43 | N, 11.47 |
| Found: | C, 61.25 | H, 7.43 | N, 11.19 |

Part H Preparation of the title compound (18d):

Compound (16d) (0.65 g, 1.33 mmol) (prepared in Part G of this Example) was treated with anhydrous anisole (5 ml) and trifluoroacetic acid (5 ml) at ambient temperature overnight (16 hours). The solution was concentrated under reduced pressure to afford 559 mg of the title compound as the trifluoroacetate salt.
FDMS 333 (M+1)
$^1H$ NMR (DMSO/TMS): $\partial$ = 1.6-1.8 (m,2H), 4-2.5 (t, 2H), 3.3-3.6 (m, 6H), 7.3 (m, 1H),7.5-7.7 (m,2H), 8.2 (s, 1H),8.6-8.8 (t, 1H), 8.9 (bs, 2H), 9.2 (bs, 2H), 11.9-12.2 (m, 2H)

| Analysis, calculated for $C_{18}H_{21}F_3N_4O_6$ | | | |
|---|---|---|---|
| Calculated: | C, 48.43 | H, 4.74 | N, 12.55 |
| Found: | C, 48.16 | H, 4.46 | N, 12.64 |

Example 13

Preparation of Compound (18e) having α-methyl substitution in the acid chain:

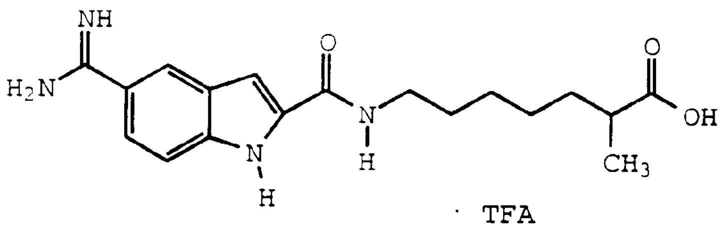

(18e)

Part A Preparation of intermediate (1b) represented by the formula (1b):

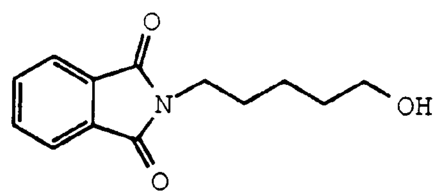

(1b)

Phthalic anhydride (14.4 g, 97 mmol) was combined with 5-amino-l-pentanol (10 g, 97 mmol) and heated at 130°C overnight (16 hours). The resulting oil was diluted with toluene, concentrated under reduced pressure to remove water and purified by chromatography ($SiO_2$, 2 % v/v $MeOH/CHCl_3$) to yield 21.42 g (94 %) of a colorless oil.

Part B Preparation of intermediate represented by the formula (2b) :

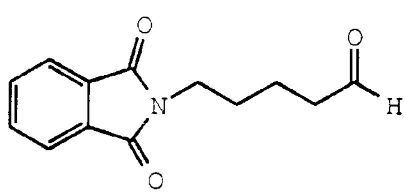

(2b)

Compound (2b) was prepared according to the method of Example 12 Part B using alcohol (1b) (prepared in Part A of this Example) as starting material instead of (1a) to yield after chromatography ($SiO_2$, 20 % v/v EtOAc-Hexane) 21.45 g (96%) of a colorless oil.

| Analysis, Calculated for $C_{13}H_{13}NO_3 \bullet$ 0.16 EtOAc | | | |
|---|---|---|---|
| Calculated: | C, 66.77 | H, 5.87 | N, 5.71 |
| Found: | C, 66.66 | H, 5.61 | N, 5.77 |

Part C Preparation of the protected α-substituted amino ester (5d) represented by the formula:

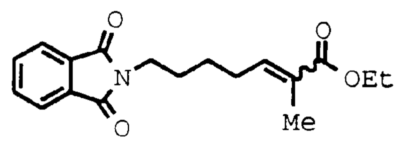

(5d)

To a mixture of triethyl-2-phosphonopropionate (7.7 g, 23.5 mmol) and dry THF (100 ml) at 0°C was added potassium tert-butoxide (3.6 g, 32.5 mmol). This was stirred at 0°C for 15 minutes then added a solution of aldehyde (2b) (prepared in Part B of this Example) in THF (10 ml). This mixture was stirred at 0°C for 1 hour then partitioned between $H_2O$ and EtOAc. The EtOAc layer was washed with $H_2O$ and dried over $MgSO_4$. After concentration under vacuum, the resulting oil was purified by chromatography ($SiO_2$, 1 % v/v MeOH/$CHCl_3$) to obtain 4.85 g (71 %) of an oil.

Part D Preparation of the intermediate (6d)

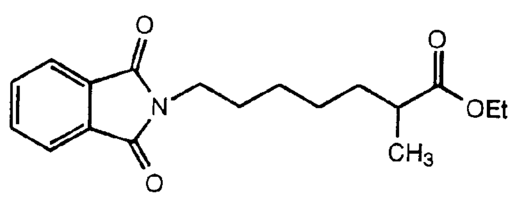

(6d)

This intermediate was prepared by the method of Example 12, Part D (6b) using starting material (5d) (prepared in Part A of this Example) in place of (5b) to give an 84% yield.

Part E Preparation of the intermediate (14e):

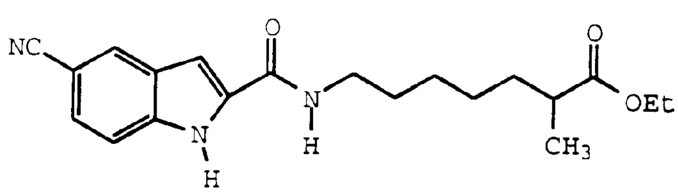

(14e)

Intermediate (14e) was prepared by the method of Example 12, Part E (14d) except that (6d) (prepared in Part B this Example) was used as starting material in place of (6b). A 50% yield was obtained after recrystallizing from EtOAc. m.p. 144-145°C. FDMS 355 (M+)

| Analysis, Calculated for $C_{20}H_{25}N_3O_3$• 0.01 EtOAc | | | |
|---|---|---|---|
| Calculated: | C, 67.55 | H, 7.09 | N, 11.79 |
| Found: | C, 67.32 | H, 7.18 | N, 12.09 |

Part F Preparation of the intermediate (15e):

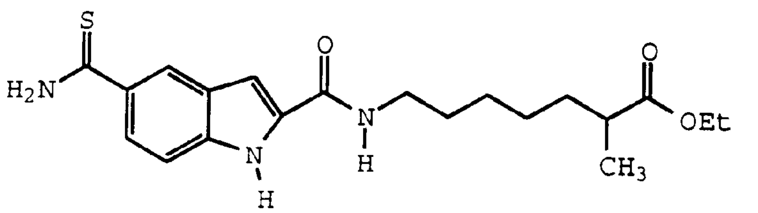

(15e)

The intermediate was prepared by the method of Example 12, Part F, (15d) in quantitative yield starting from (14e) (prepared in Part C of this Example). The product was obtained as an analytically pure sample by triturating a small amount with $CH_2Cl_2$. FDMS 389 (M+)

| Analysis, Calculated for $C_{20}H_{27}N_3SO_3$ | | | |
|---|---|---|---|
| Calculated: | C, 61.67 | H, 6.99 | N, 10.79 |
| Found: | C, 61.87 | H, 6.87 | N, 10.79 |

Part G Preparation of the intermediate (16e):

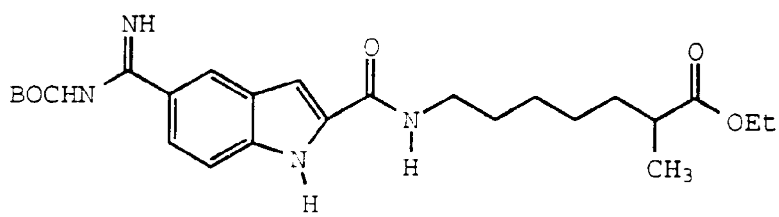

(16e)

This intermediate was prepared by the method of Example 12, Part G (16d) using as starting material the product of Part E (15e). The product was obtained in 55% yield.

Part H Preparation of the intermediate (17d):

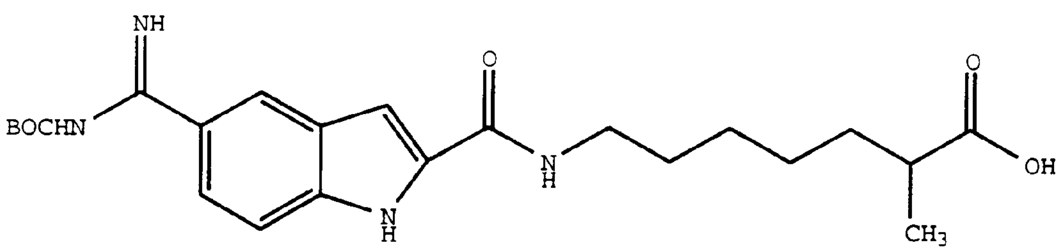

(17d)

A solution of (16e) (prepared in Part E of this Example) (0.7 g, 1.5 mmol) in EtOH (30 ml) was treated with 5 N aqueous NaOH (1.5 ml) at ambient temperature overnight (16 hours) until the reaction was over as evident by TLC ($SiO_2$, 10:1:89 v/v MeOH/HOAc/$CHCl_3$). Glacial acetic acid (0.52 ml, 9 mmol) was added and the mixture was concentrated to foam which was purified by chromatography ($SiO_2$, 10-19:1 v/v MeOH/HOAc/$CHCl_3$) to recover 0.44 g of a foam.

Part I Preparation of the title compound (18e):

The title compound was prepared by the method of Example 12, Part H (18d) using as starting material (17d) (prepared in Part F of this Example). The product was purified by reverse phase chromatography ($C_{18}$ column (41.4X3), 20% $CH_3CN$ in $H_2O$) to afford the title compound as the trifluoroacetate salt in 24% yield. m.p. 266-268°C Exact Mass Calculated for $C_{18}H_{24}N_4O_3$ 345.1972 $^{(M+1)}$. Exact mass was found to be 345.1896. $^1H$ NMR (DMSO/TMS): ∂ = 1.0-1.1 (m, 3H), 1.2-1.4 (m, 5H), 1.5-1.6 (m, 3H), 2.2-2.4 (m, 1H), 3.2-3.4 (m, 2H), 7.3 (s, 1H),7.5-7.7 (m, 2H), 3.2 (s, 1H), 3.6-8.7 (t, 1H), 8.9 (bs, 2H), 9.2 (bs, 2H), 11.9-12.2 (m, 2H)

Example 14

Preparation of Compound (18f):

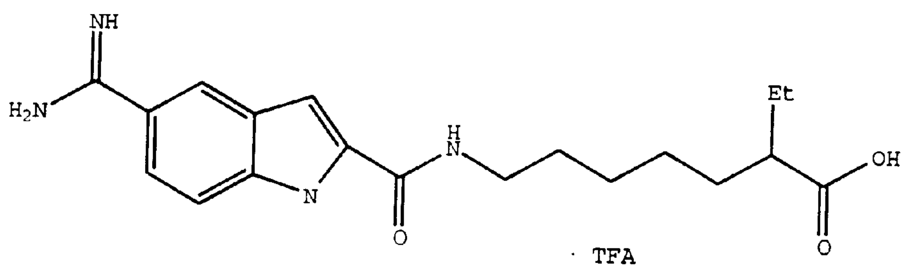

(18f)

Part A Preparation of intermediate (5e):

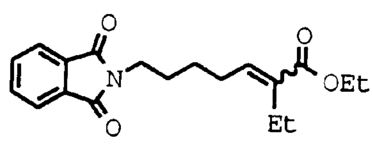

(5e)

Intermediate (5e) was prepared in 50% yield from aldehyde (2b) (Example 13, Part B) and triethyl-2-phosphonobutyrate using sodium hydride as the base by following the procedure described in Example 13, Part C. FDMS 329 (M+)

| Analysis, Calculated for $C_{19}H_{23}NO_4$ | | | |
|---|---|---|---|
| Calculated: | C, 69.28 | H, 7.04 | N, 4.25 |
| Found: | C, 69.02 | H, 7.10 | N, 4.33 |

Part B Preparation of the intermediate (6e):

Ethyl ester (5e) (prepared in Part A of this Example) (6.6 g, 20 mmol) was mixed with HOAc (50 ml) and Pd/C 10% (3.75 g). The mixture was hydrogenated on a PARR$^{tm}$ brand apparatus with initial pressure of 40

psi (2.81 Kg/cm2) until an NMR of an aliquot indicated the reaction was over (7 hours). The reaction product was filtered through a pad of celite and concentrated under reduced pressure to recover 6.16 g (93%) oil. FDMS 331 (M+).

| Analysis, Calculated for $C_{19}H_{25}NO_4$•0.2 $CH_3COOH$ | | | |
|---|---|---|---|
| Calculated: | C, 67.85 | H, 7.57 | N, 4.08 |
| Found: | C, 67.65 | H, 7.65 | N, 4.06 |

Part C Preparation of the intermediate (14f):

Intermediate (14f) was prepared by the method of Example 12, Part E (14d) using 6e (prepared in Part B of this Example) as starting material to give a 67% yield. An analytically pure sample was obtained by recrystallizing a small amount from EtOAc. m.p. 152-153°C. FDMS 369 (M+)

| Analysis, Calculated for $C_{21}H_{27}N_3O_3$ | | | |
|---|---|---|---|
| Calculated: | C, 68.27 | H, 7.37 | N, 11.37 |
| Found: | C, 68.17 | H, 7.44 | N, 11.47 |

Part D Preparation of the intermediate (15f):

The intermediate was prepared by the method of Example 12, Part F (15d) in a quantitative yield starting from (14f) (prepared in of Part C of this Example). A small sample was recrystallized from EtOH.

Part E Preparation of the intermediate (16f):

The intermediate was prepared by the method of Example 12, Part G (16d) starting from (15f) (prepared in Part D this Example) in 64% yield.

Part F Preparation of the intermediate (17e):

The intermediate was prepared using the method of Example 13, Part H (17d) using (16f) as starting material to give a 36% yield.

| | | |
|---|---|---|
| HRMS (M+1) | Calculated for $C_{24}H_{35}N_4O_5$ | 459.2607 |
| | Found | 459.2613 |

Part G Preparation of the title compound (18f):

The title compound was prepared by the method of (18d) Example 12, Part H starting from (17e). The product was purified by triturating with EtOAc to afford a 70% yield of the title compound as the trifluoroacetate salt. m.p. 196-199°C. FDMS 359 $^{(M+1)}$

$^1$H NMR (DMSO/TMS): ∂ = 0.8-0.9 (t, 3H), 1.2-1.6 (m, 10H), 2.1-2.2 (m, 1H), 3.2-3.4 (m, 2H), 7.3 (s, 1H), 7.6 (m, 2H) ,8.2 (s, 1H), 8.6 (t, 1H), 8.9 (bs, 2H), 9.2 (bs, 2H), 12.0-12.2 (m, 2H)

| Analysis, Calculated for $C_{21}H_{27}F_3N_4O_5$ | | | |
|---|---|---|---|
| Calculated: | C, 53.39 | H, 5.76 | N, 11.86 |
| Found: | C, 53.13 | H, 5.72 | N, 11.72 |

Example 15

Preparation of Compound (18g):

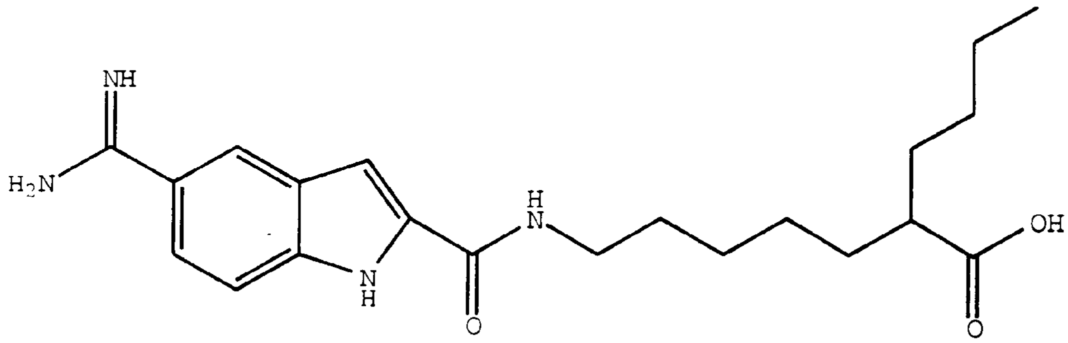

(18g)

Part A Preparation of the intermediate α-Substituted Amino Ester (5f):

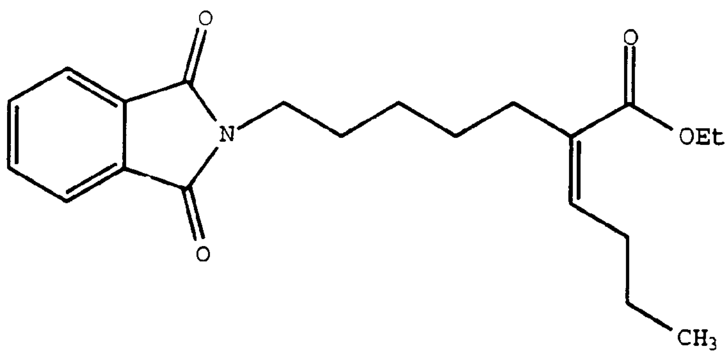

(5f)

Intermediate (5f) was prepared by the method of Example 14, Part A (5e) using as starting material triethyl-2-phosphonohexanoate and (2b) (prepared in Example 13 Part B) to give a 68 % yield.
FDMS 357 (M+)

| Analysis, Calculated for $C_{21}H_{27}NO_4$ | | | |
|---|---|---|---|
| Calculated: | C, 70.56 | H, 7.61 | N, 3.92 |
| Found: | C, 70.31 | H, 7.70 | N, 3.75 |

Part B Preparation of the intermediate (6f):

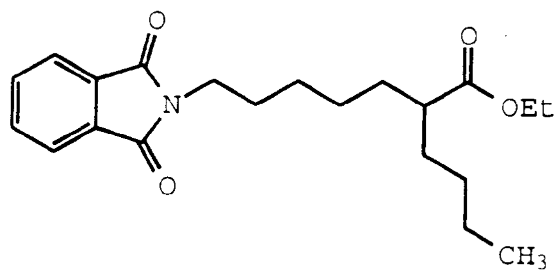

(6f)

The intermediate was prepared by the method of preparing (6e) (from Example 14, Part B) in 98% yield using (5f) of Part A as starting material. FDMS 359 (M+)

| Analysis, Calculated for $C_{21}H_{29}NO_4$ | | | |
|---|---|---|---|
| Calculated: | C, 70.17 | H, 8.13 | N, 3.90 |
| Found: | C, 70.21 | H, 8.40 | N, 3.92 |

Part C Preparation of the intermediate (14g):

The intermediate was prepared by the method of Example 12, Part E (14d) using as starting material (6f) (prepared in Part B of this Example) in 67% yield. An analytically pure sample was obtained by recrystallizing a small amount from hexane/$Et_2O$. FDMS 397 (M+)

| Analysis, Calculated for $C_{23}H_{31}N_3O_3$• 0.17 Hexane | | | |
|---|---|---|---|
| Calculated: | C, 70.00 | H, 8.716 | N, 10.19 |
| Found: | C, 70.02 | H, 7.92 | N, 10.40 |

Part D Preparation of the intermediate (15g):

The intermediate was prepared by the method of Example 12, Part F (15d) starting from the product of Part C of this Example (14g). After concentrating under reduced pressure, dissolved the residue in EtOAc, washed with $H_2O$ and reconcentrated to a yellow solid which was triturated with acetone to give analytically pure (15g) in a quantitative yield. FDMS 431 (M+)

| Analysis, Calculated for $C_{23}H_{33}N_3SO_3$ | | | |
|---|---|---|---|
| Calculated: | C, 64.01 | H, 7.71 | N, 9.74 |
| Found: | C, 64.23 | H, 7.76 | N, 9.83 |

Part E Preparation of the intermediate (16g):

The intermediate was prepared by the method of Example 12, Part G (16d) starting from (15g) in 69% yield.

Part F Preparation of the intermediate (17f):

The intermediate was prepared by the method of Example 13, Part H (17d) starting from (16g) (prepared

in Part E of this Example) in 92% yield.

Part G Preparation of the title compound (18g):

The title compound was prepared in a manner according to Example 12, Part H (18d) starting from (17f). The product was purified by triturating with $Et_2O$ to afford an 82% yield of the desired product as the TFA salt. m.p. 209-212°C. FDMS 387 $^{(M+1)}$

$^1H$ NMR (DMSO/TMS): $\partial$ = 0.8-0.9 (t, 3H), 1.1-1.6 (m, 14H), 2.1-2.3 (m, 1H), 3.2-3.3 (m, 2H), 7.3 (s, 1H), 7.6 (m, 2H), 8.2 (s, 1H), 8.6 (t, 1H), 8.9 (bs, 2H), 9.2 (bs, 2H), 11.9-12.2 (m, 2H)

| Analysis, Calculated for $C_{23}H_{31}F_3N_4O_5$•0.4 $CF_3COOH$ | | | |
|---|---|---|---|
| Calculated: | C, 52.34 | H, 5.80 | N, 10.26 |
| Found: | C, 52.52 | H, 5.85 | N, 10.50 |

Example 16

Preparation of Compound (18h):

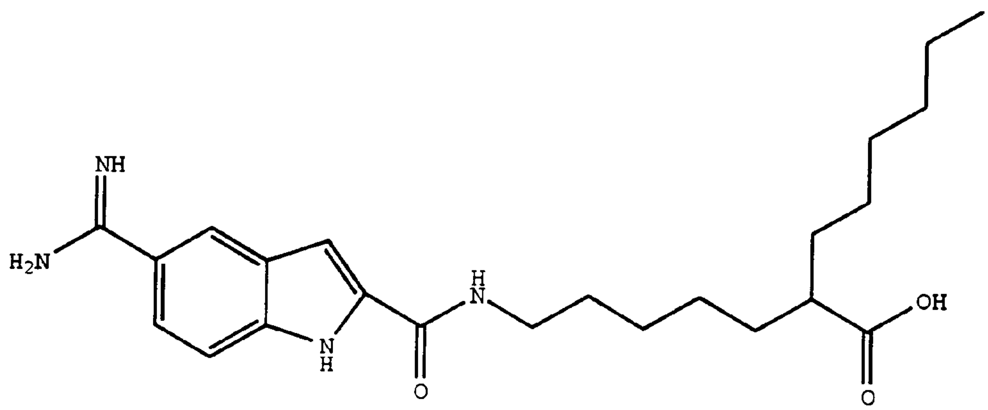

(18h)

Part A Preparation of intermediate (5g):

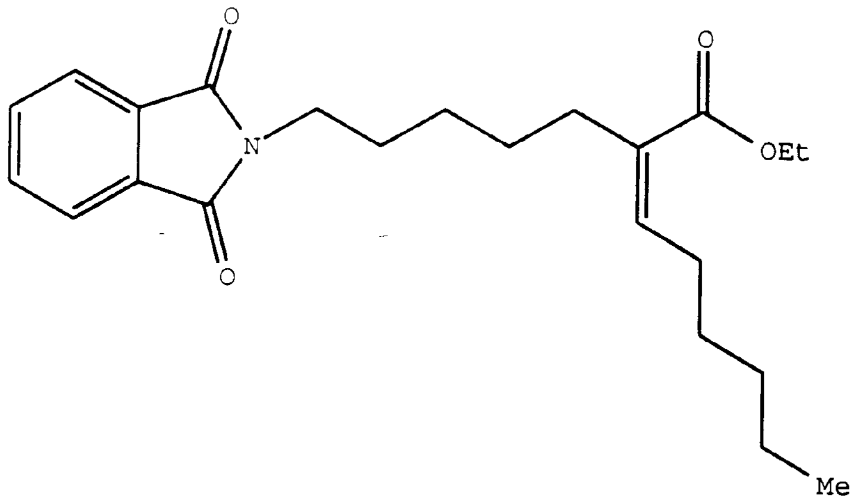

(5g)

The intermediate was prepared by the method of Example 14, Part A (5e) using as starting material triethyl-2-phosphono-octanoate and (2b) (product of Example 13 Part B) in 64 % yield.

Part B Preparation of intermediate (6g):

The intermediate was prepared by the method of Example 14, Part B (6e) using as starting material (5g) in 97% yield. FDMS 425 (M+)

| Analysis, Calculated for $C_{25}H_{35}N_3O_3$ | | | |
|---|---|---|---|
| Calculated: | C, 70.56 | H, 8.29 | N, 9.87 |
| Found: | C, 70.57 | H, 8.24 | N, 9.66 |

Part C Preparation of the intermediate (14h):

The intermediate was prepared by the method of Example 12, Part E (14d) starting from 6g (prepared in Part B this Example) in 68% yield. FDMS 425 (M+)

| Analysis, Calculated for $C_{25}H_{35}N_3O_3$ | | | |
|---|---|---|---|
| Calculated: | C, 70.56 | H, 8.29 | N, 9.87 |
| Found: | C, 70.57 | H, 8.24 | N, 9.66 |

Part D Preparation of the intermediate (15h):

This intermediate was prepared by the method of Example 12, Part F (15d) starting from (14h) (prepared in Part C this Example) . The product was purified by chromatography ($SiO_2$, 2 % v/vMeOH/$CHCl_3$) to recover a 85 % yield of the desired product. FDMS 459 (M+)

| Analysis, Calculated for $C_{25}H_{37}N_3SO_3$ | | | |
|---|---|---|---|
| Calculated: | C, 65.33 | H, 8.11 | N, 9.14 |
| Found: | C, 65.30 | H, 8.04 | N, 9.23 |

Part E Preparation of the intermediate (16h):

The intermediate was prepared according to Example 12, Part G (16d) starting from (15h) (prepared in Part D this Example) in 52% yield. FDMS 543 (M+1)

| Analysis, Calculated for $C_{30}H_{46}N_4O_5$• .05 $CHCl_3$ | | | |
|---|---|---|---|
| Calculated: | C, 65.78 | H, 8.46 | N, 10.21 |
| Found: | C, 65.64 | H, 8.19 | N, 10.09 |

Part F Preparation of the product (18h):

The title compound was prepared by treating compound (16h) (1.1 g, 2 mmol) in EtOH (30 ml) with a solution of LiOH (.48 g, 20 mmol in 10 ml $H_2O$) at ambient temperature overnight (16 hours) then at reflux for 4 hours. The reaction mixture was cooled to room temperature, quenched with 5 N aqueous HCl (3.5 ml) and concentrated to solid. The product was purified by chromatography ($SiO_2$, 4-10% v/v $CHCl_3$/MeOH, 1 % HOAc) to recover .24 g (29%) of the free base of the desired amidino acid. FDMS 415 (M+1)
$^1H$ NMR (DMSO/TMS): ∂ = 0.8-0.9 (t, 3H), 1.0-1.6 (m, 18H), 2.2-2.1 (m, 1H), 3.2-3.4 (m, 2H), 7.3 (s, 1H), 7.5-7.6 (m, 2H), 8.2 (s, 1H), 8.7 (t, 1H), 9.0 (bs, 2H), 10.3 (bs, 3H)

Example 17

Preparation of Compound (18i):

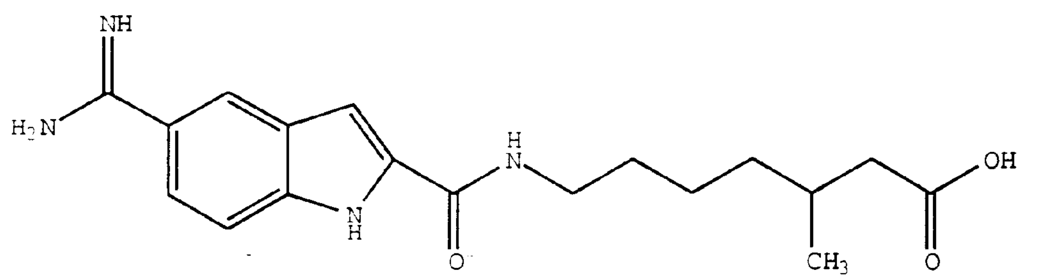

· TFA

(18i)

Part A Preparation of the protected amino acid intermediate (19) :

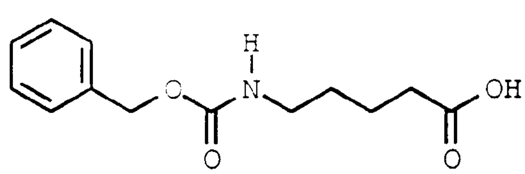

(19)

To a mixture of 5-aminovaleric acid (7.75 g, 66 mmol) and sodium carbonate (7 g, 66 mmol) in $H_2O$ (50 ml) was added benzyl chloroformate dropwise at ambient temperature. Stirring was continued for 3 hours. EtOAc was added before acidifying the mixture with 5 N aqueous HCl. The organic layer was washed with $H_2O$ and brine, dried with $MgSO_4$ and concentrated under reduced pressure. Trituration with $Et_2O$ yielded 12.6 g (76%) of a white solid.

Part B Preparation of the intermediate (20):

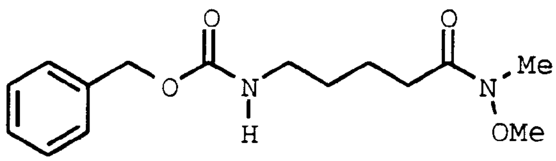

(20)

5-carbobenzyloxyamino valeric acid (19) (55g, .22 mol), N-methyl morpholine (55.4 g, .26 mol) and $CH_2Cl_2$ (500 ml) were combined in a reaction mixture. The mixture was cooled to -10°C and isobutyl chloroformate (35.9 g, .26 mol) added dropwise. The mixture was stirred for 45 minutes then N,O-dimethylhydroxylamine hydrochloride (53.4 g, .55 mol) was added all at once as a solid. Stirring was continued at 0°C for 3 hours. The mixture was quenched with saturated aqueous $NaHCO_3$, the layers were separated and the organic layer dried over $MgSO_4$. The product was concentrated under vacuum to an oil which was purified by chromatography ($SiO_2$, 40 % v/v EtOAc/Hexane) to recover 30.47 g (47%) of the Wienreb amide (20) as a colorless oil.

Part C Preparation of intermediate (21a) :

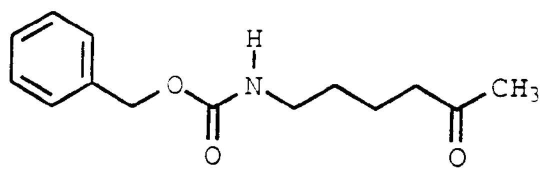

(21a)

The Wienreb amide (20) was dissolved in dry THF (450 ml) under argon and cooled to -78°C. 1.4 M $CH_3Li$ in ethyl ether (222 ml, 312 mmol) was then added. The mixture was warmed to 0°C and stirring continued for 5 hours after which it was recooled to -78°C and quenched with saturated aqueous $NaHCO_3$ (100 ml). The mixture was then partitioned between $H_2O$ and EtOAc. The organic layer was dried over $MgSO_4$ and concentrated to an oil which was purified by chromatography ($SiO_2$, 15 % v/v EtOAc/Hexane) to recover 22 g (85%) of colorless oil.

Part D Preparation of intermediate (22a):

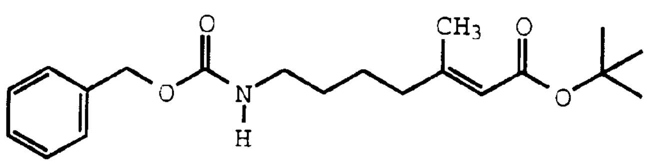

(22a)

Sodium hydride (60% in oil, 7.4 g, 185 mmol) was washed with hexane and suspended in dry THF (400 ml). The mixture was cooled to -10°C and t-butyl diethylphosphonoacetate (47 g, 185.3 mmol) was added portionwise. The temperature was allowed to rise to 0°C where it was stirred for 2 hours after which a solution of

the ketone (21a) [Part C this Example] (22 g, 88 mmol) in THF (75 ml) was added quickly. The reaction was stirred at ambient temperature for 2 hours then quenched with saturated aqueous $NH_4Cl$ (100 ml). The mixture was partitioned between $H_2O$ and EtOAc. After separating, the organic layer was dried over $MgSO_4$ and concentrated to an oil which was purified by chromatography ($SiO_2$, 15 % v/v EtOAc/Hexane) to recover 25.8 g (84%) of a colorless oil.

Part E Preparation of the intermediate (14i):

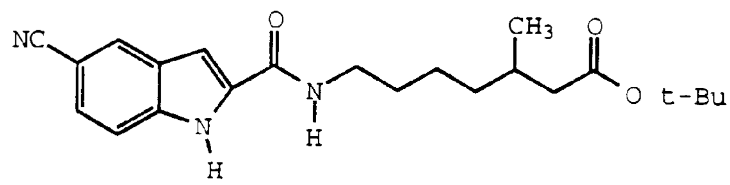

(14i)

Ester (22a) (7.3g, 21 mmol) (prepared in Part D of this Example) was mixed with EtOAc (25 ml) and Pd/C 10% (3 g). The mixture was hydrogenated at atmospheric pressure for 18 hours, filtered through celite and concentrated to give 4.5 g of (13a) as an oil.

A solution of 5-cyanoindole-2-carboxylate (1.10 g, 5.91 mmol) (prepared in Example 1 Part D), 4-dimethylaminopyridine (0.725 g, 5.93 mmol), diisopropylethylamine (2.1 ml, 12.1 mmol) and (13a) (1.05 g, 4.87 mmol) in $CH_2Cl_2$ (50 ml) was treated with 1-(3-dimethylaminopropyl)-3-ethylcarbodiimide hydrochloride (1.35 g, 7.04 mmol). The reaction was stirred at room temperature for 48 hours at which time it was washed sequentially with 1 N aqueous citric acid (50 ml) and $H_2O$ (50 ml). The organic phase was dried over $Na_2SO_4$ and evaporated to give 2.1 g of a tan solid which was purified by flash chromatography ($SiO_2$; 40 % EtOAc in hexane) to afford 850 mg (38 %) of a white solid.
MS(FD), m/e 383 (M+; 100).

| Anal Calcd. for $C_{22}H_{29}N_3O_3$: | | | |
|---|---|---|---|
| Calculated | C 69.11, | H 7.64, | N 11.10; |
| Found | C 68.90, | H 7.62, | N 10.96. |

Part F Preparation of the intermediate (15i) :

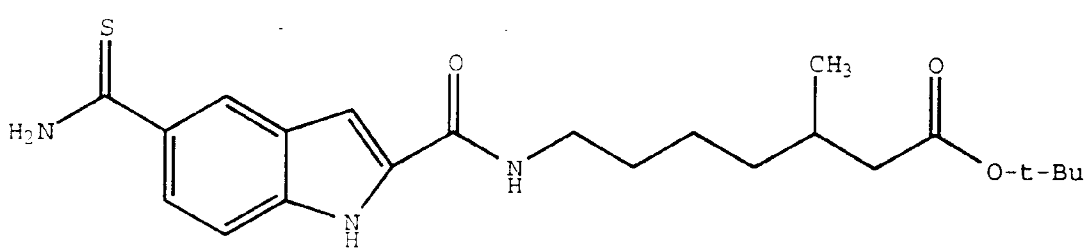

(15i)

The intermediate was prepared by the method of (15d) (Example 12, Part F) starting from (14i). The product was purified by chromatography ($SiO_2$, 2 % v/v MeOH-$CHCl_3$) to recover a 88 % yield. FDMS 417 (M+)

| Analysis, Calculated for $C_{22}H_31N_3SO_3$ | | | |
|---|---|---|---|
| Calculated: | C, 63.28 | H, 7.48 | N, 10.06 |
| Found: | C, 63.11 | H, 7.45 | N, 10.13 |

Part G Preparation of the intermediate (16i)

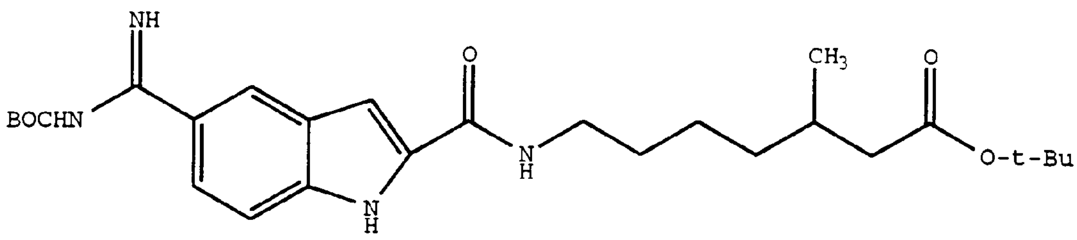

(16i)

The intermediate was prepared according to Example 12, Part G (16d) starting from (15i) in quantitative yield. FDMS 501 (M+1)

Part H Preparation of the title compound (18i) :

The title compound was prepared according to Example 9, Part G (18a) starting from (16i) (prepared in Part F of this Example) in a 92% yield of analytically pure material as the TFA salt.
FDMS 345 (M+1)
Analysis, Calculated for $C_{20}H_{25}F_3N_4O_5$
$^1$H NMR (DMSO/TMS) : ∂ = 0.8-0.9 (d, 3H), 1.1-1.6 (m, 6H), 1.8-1.9 m, 1H) , 1.9-2.3 (m, 2H), 3.2-3.4 (m, 2H), 7.3 (s, 1H), 7.5-7.7 (m, 2H), 8.2 (m, 1H), 8.6-8.7 (t, 1H), 8.8 (s, 2H), 9.2 (s, 2H), 11.9-12.2 (m, 2H)

| | | | |
|---|---|---|---|
| Calculated: | C, 52.40 | H, 5.5 | N, 12.22 |
| Found: | C, 52.30 | H, 5.47 | N, 11.94 |

Example 18

Preparation of Compound (18j):

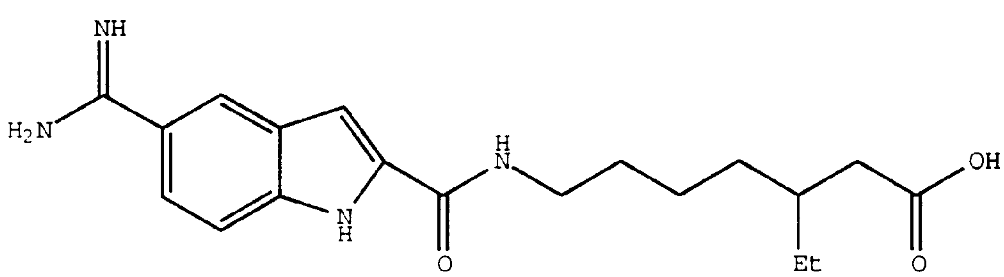

· TFA

(18j)

Part A Preparation of the intermediate (21b):

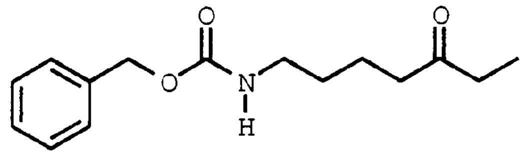

(21b)

Intermediate (21b) was prepared from Weinreb amide (Example 17, Part B) and ethyl magnesium bromide by substantially following the procedure described in Example 17, Part C. FD-MS 263 (M+; 100)

| Elemental Analysis Calcd. For $C_{15}H_{21}NO_3$: | | | |
|---|---|---|---|
| Calculated | C 68.42, | H 8.04, | N 5.32. |
| Found | C 68.29, | H 7.90, | N 5.26. |

Part B Preparation of the second intermediate (22b) :

Intermediate (22b) was prepared according to the methods described in Example 17, Part D except that ketone (21b) (Example 18, Part A) was used instead of ketone (21a) to give 2.20 g (31 %) of the product as an oil.
FDMS 362 (M+1; 100)

| Analysis, Calculated for $C_{21}H_{31}N_1O_4$ | | | |
|---|---|---|---|
| Calculated: | C, 69.78 | H, 8.64 | N, 3.88 |
| Found: | C, 69.99 | H, 8.60 | N, 4.04 |

Part C Preparation of intermediate (14j):

Intermediate (14j) was prepared using the methodology described in Example 17, Part E except that α,β-unsaturated ester (22b) was used instead of product (22a) to yield the desired product.
FDMS 397 (M+, 100)

| Analysis, Calculated for $C_{23}H_{31}N_3O_3$ | | | |
|---|---|---|---|
| Calculated: | C, 69.50 | H, 7.86 | N, 10.57 |
| Found: | C, 69.68 | H, 7.75 | N, 10.70 |

Part D Preparation of intermediate (15j) :

This intermediate was prepared according to Example 12, Part F (15d) starting from (14j). The product was purified by chromatography ($SiO_2$, 1 % v/v MeOH/$CHCl_3$) to recover a 93 % yield of the desired product.
FDMS 431 (M+)

| Analysis, Calculated for $C_{23}H_{33}N_3SO_3$ | | | |
|---|---|---|---|
| Calculated: | C, 64.01 | H, 7.71 | N, 9.74 |
| Found: | C, 63.76 | H, 7.69 | N, 9.98 |

Part E Preparation of the intermediate (16j) :

The intermediate was prepared in a manner similar to (16d) (Example 12, Part G) starting from (15j) in 89% yield. FDMS 535 (M+1)

Part F Preparation of the title compound (18j) :

The title compound was prepared by the method of Example 9, Part G (18a) starting from (16j) to give a 75% yield of analytically pure material as the TFA salt.
FDMS 359 (M+1)
Analysis, Calculated for $C_{21}H_{27}F_3N_4O_5$
$^1H$ NMR (DMSO/TMS) : ∂ = 0.8-0.9 (m, 3H), 1.2-1.4 (m, 6H), 1.3-1.4 (m, 2H),1.4-1.5 (m, 1H), 2.1-2.2 (d, 2H), 3.3-3.4 (m, 2H), 7.3 (s, 1H), 7.5-7.7 (m, 2H), 8.2 (s, 1H), 8.6-8.7 (t, 1H), 8.8 (s, 2H), 9.2 (s, 2H), 12.0 (s, 1H) , 12.1 (s, 1H)

| | | | |
|---|---|---|---|
| Calculated: | C, 53.39 | H, 5.76 | N, 11.86 |
| Found: | C, 53.44 | H, 5.69 | N, 11.79 |

Example 19

Preparation of Compound (18k) :

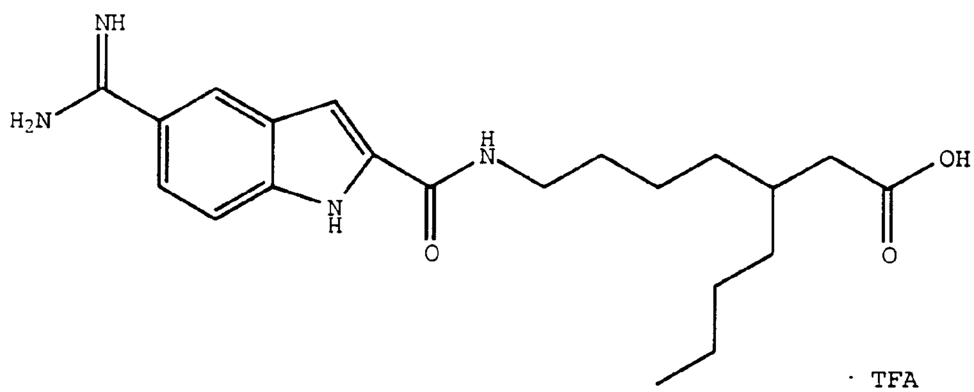

· TFA

(18k)

Part A Preparation of the intermediate (21c) :

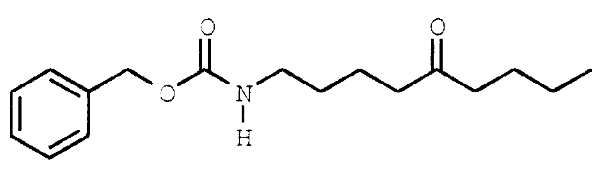

(21c)

Intermediate (21c) was prepared from Weinreb amide (20; Example 17, Part B) and n-butyl lithium by the procedure described in Example 17, Part C.

Part B Preparation of the intermediate (22c) :

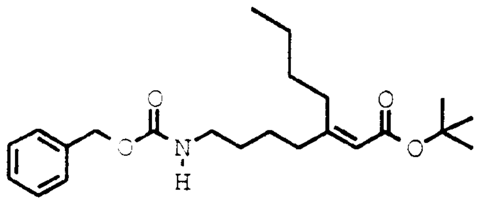

(22c)

Intermediate (22c) was prepared from ketone (21c) (prepared in Part A of this Example) and tert-butyl diethylphosphonoacetate in 89% yield by the procedure described in Example 17, Part D.

| Analysis Calculated for $C_{23}H_{35}NO_4$ | | | |
|---|---|---|---|
| Calculated | C, 70.92 | H, 9.06 | N, 3.60 |
| Found | C, 71.13 | H, 8.97 | N, 3.85 |

Part C Preparation of the intermediate (14k) :

This intermediate is prepared according to Example 17, Part E (14i) starting with (22c) to give after purification by chromatography ($SiO_2$, 1 % v/v MeOH/$CHCl_3$) a 54 % yield of (14k) as a white solid. An analytically pure sample was obtained by recrystallizing a small sample from Hexane/EtOAc. FDMS 425 (M+)

| Analysis, Calculated for $C_{25}H_{35}N_3O_3$ | | | |
|---|---|---|---|
| Calculated: | C, 70.56 | H, 8.29 | N, 9.87 |
| Found: | C, 70.84 | H, 4.11 | N, 10.10 |

Part D Preparation of the intermediate (15k) :

The intermediate was prepared according to Example 12, Part F (15d) starting from (14k). The product was purified by chromatography ($SiO_2$, 1 % v/v MeOH/$CHCl_3$) to recover a 62 % yield of the desired product. FDMS 459 (M+)

| Analysis, Calculated for $C_{25}H_{37}N_3SO_3$ | | | |
|---|---|---|---|
| Calculated: | C, 65.33 | H, 8.11 | N, 9.14 |
| Found: | C, 65.52 | H, 8.314 | N, 9.22 |

Part E Preparation of the intermediate (16k) :

The intermediate was prepared according to Example 12, Part G (16d) starting from (15k) in 60% yield. FDMS 543 (M+1)

| Analysis, Calculated for $C_{30}H_{46}N_4O_5$ | | | |
|---|---|---|---|
| Calculated: | C, 64.01 | H, 7.71 | N, 9.74 |
| Found: | C, 63.76 | H, 7.69 | N, 9.98 |

Part F Preparation of the title compound (18k) :

The title compound was prepared according to Example 9, Part G (18a) starting from (16k) in a 87% yield of analytically pure material as the TFA salt.
FDMS 387 (M+1)

| Analysis, Calculated for $C_{23}H_{31}F_3N_4O_5$ | | | |
|---|---|---|---|
| Calculated: | C, 55.19 | H, 6.24 | N, 11.19 |
| Found: | C, 55.19 | H, 6.27 | N, 11.05 |

$^1H$ NMR (DMSO/TMS): ∂ = 0.8-0.9 (m, 3H), 1.1-1.3 (m, 11H), 1.5-1.6 (m, 2H), 1.7-1.8 (m, 1H), 2.1-2.2 (m, 2H), 3.2-3.4 (m, 2H), 7.3 (s, 1H), 7.5-7.7 (m, 2H), 8.2 (s, 1H), 8.6-8.7 (t, 1H), 8.9 (s, 2H), 9.2 (s, 1H), 11.9-12.2 (m, 2H)

Example 20

Preparation of Compound (18l):

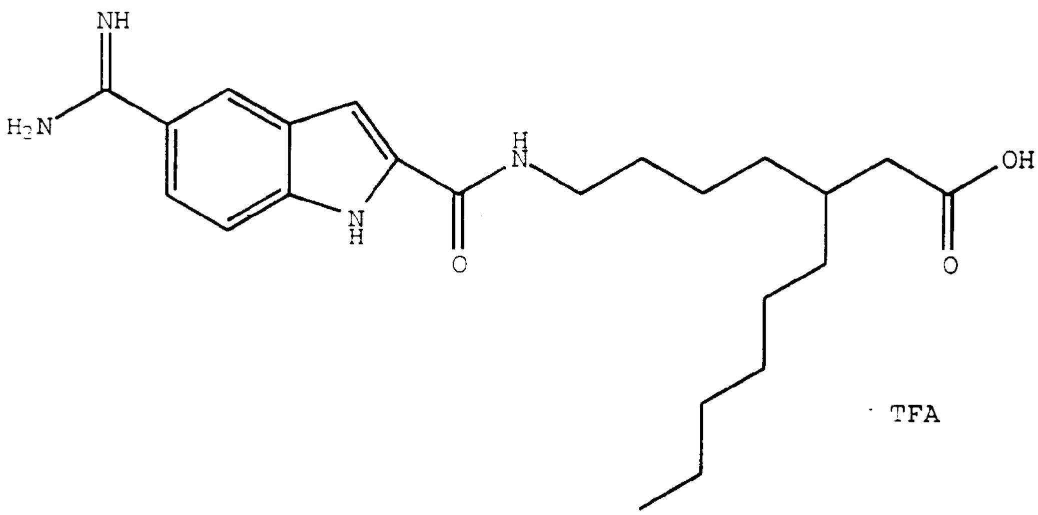

(181)

Part A Preparation of the intermediate (21d) :

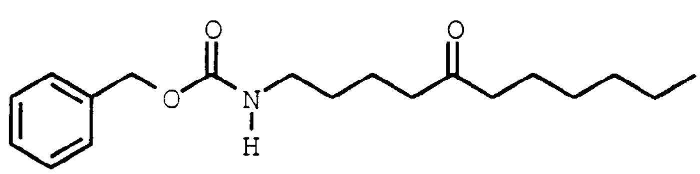

(21d)

Winereb amide (20) (10 g, 34 mmol) (Example 17, Part B) was dissolved in dry THF (200 ml) under argon and the solution cooled to -78°C. Hexyl magnesium bromide prepared from hexyl bromide (33.6 g, 204 mmol) and magnesium turnings (4.95 g, 203 mmol) in ethyl ether (50 ml) was added dropwise. The mixture was warmed to -30°C for 30 minutes, then to 0°C for 4 hours. The mixture was recooled to -78°C, quenched with saturated aqueous $NH_4Cl$ (50 ml) then partitioned between $H_2O$ and EtOAc. The organic layer was dried over $MgSO_4$ and concentrated to an oil which was purified by chromatography ($SiO_2$, 30 % v/v EtOAc/Hexane) to

recover 7.9 g (73%) of colorless oil which solidified on standing.

Part B Preparation of the intermediate (22d) :

Intermediate (22d) was prepared from ketone (21d) and tert-butyl diethylphosphonoacetate in 67% yield by following the procedure described in Example 17, Part D.

Part C Preparation of the intermediate (14l) :

Intermediate (141) was prepared in 52% yield by using the method of Example 17, Part E substituting (22d) for (22a) as the starting material.
FDMS 453 (M+)

| Analysis, Calculated for $C_{27}H_{39}N_3O_3$ | | | |
|---|---|---|---|
| Calculated: | C, 71.49 | H, 8.67 | N, 9.26 |
| Found: | C, 71.41 | H, 8.56 | N, 9.37 |

Part D Preparation of the intermediate (15l):

The intermediate was prepared by the method of Example 12, Part F (15d) starting from (141). The product was purified by chromatography ($SiO_2$, 2 % v/v MeOH/$CHCl_3$) to recover a 47 % yield of the desired product.
FDMS 487$^{(M+)}$

| Analysis, Calculated for $C_{27}H_{41}N_3SO_3$ | | | |
|---|---|---|---|
| Calculated: | C, 66.50 | H, 8.47 | N, 8.62 |
| Found: | C, 66.49 | H, 8.41 | N, 8.78 |

Part E Preparation of the intermediate (16l):

The intermediate was prepared by the method of (16d) (Example 12, Part G) starting from (151) in 56% yield. FDMS 571 (M+1)

Part F Preparation of the title compound (18l):

The title compound was prepared by the method of (18a) (Example 9, Part G) starting from (161) in a 91% yield of analytically pure material as the TFA salt.
FDMS 415 $^{(M+1)}$

| Analysis, Calculated for $C_{20}H_{25}F_3N_4O_5$ | | | |
|---|---|---|---|
| Calculated: | C, 56.81 | H, 6.67 | N, 10.60 |
| Found: | C, 57.10 | H, 6.78 | N, 10.43 |

$^1$NMR (DMSO/TMS): ∂ = 0.7-0.8 (m, 3H), 1-1.4 (m, 14H), 1.4-1.6 (m, 2H), 1.6-1.8 (m, 1H), 2.0-2.2 (m, 2H), 3.2-3.3 (m, 2H), 7.3 (s, 1H) , 7.5-7.6 (m, 2H), 8.2 (s, 1H), 8.9 (s, 2H), 9.1 (s, 2H), 11.9 (s, 1H), 12.1 (m, 1H)

Example 21

Preparation of Compound (18m) :

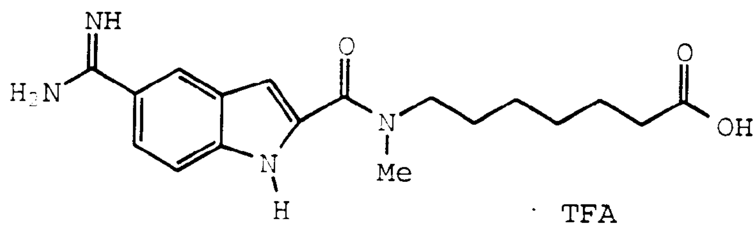

(18m)

Part A Preparation of the intermediate N-Cbz-7-aminoheptanoic acid represented by the formula below:

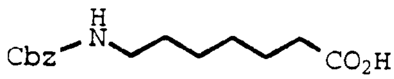

A solution of 7-aminoheptanoic acid (5.0 g, 34.4 mmol) in 2 N aqueous NaOH (75 ml) was treated simultaneously with benzyl chloroformate (6.0 ml, 42.0 mmol) and 2 N aqueous NaOH 75 ml. The reaction was stirred for 22 hours and was diluted with an additional 2 N aqueous NaOH (150 ml). The mixture was extracted with 100 ml of $Et_2O$, was acidified to pH 1 with 5 N aqueous HCl, and extracted with EtOAc (4 x 100 ml). The combined EtOAc extracts were dried over $Na_2SO_4$ and evaporated to give 7.8g (28 mmol; 81 %) of the title compound as a white solid.
MS(FD) m/e, 280 (M+1).

| Anal Calculated for $C_{17}H_{25}NO_4$ (0.2 HCl) : | | | |
|---|---|---|---|
| Calculated | C 62.86, | H 7.45, | N 4.89; |
| Found | C 62.70, | H 7.23, | N 5.00. |

Part B Preparation of the intermediate methyl *N*-Cbz-*N*-methyl-7-aminoheptanoate represented by the following formula:

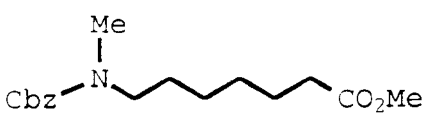

A slurry of NaH (60% dispersion in mineral oil; 2.1 g, 54.8 mmol) in THF (200 ml) at 0°C was treated sequentially with a solution of N-Cbz-7-aminoheptanoic acid (5.0 g) in THF (50 ml) followed by MeI (11.1 ml; 25.3 g; 178 mmol). The reaction was allowed to stir at room temperature for 18 hours at which time it was cooled to 0°C and quenched by the addition of brine (100 ml). The organic layer was separated, dried over $Na_2SO_4$, and evaporated to afford 8.10 g of an oil. Purification by flash chromatography ($SiO_2$; 40 % EtOAc/hexanes) afforded 4.11 g (75 %) of the title compound as a viscous oil which solidified on standing.
$^1H$ NMR (300 MHz; DMSO-$d_6$) d 7.37-7.22 (m, 5H), 5.03 (s, 2H), 3.55 (s, 3H), 3.19 (t, J = 6.8 Hz, 2H), 2.81 (bd, J = 8.7 Hz, 3H), 2.29-2.17 (m, 2H), 1.55-1.36 (m, 4H), 1.33-1.03 (m, 4H).
MS(FD) m/e, 307 (M+1).

| Anal Calculated for $C_{17}H_{25}NO_4$: | | | |
|---|---|---|---|
| Calculated | C 66.43, | H 8.20, | N 4.60; |
| Found | C 66.19, | H 8.22, | N 5.17. |

Part C Preparation of the intermediate (14m):

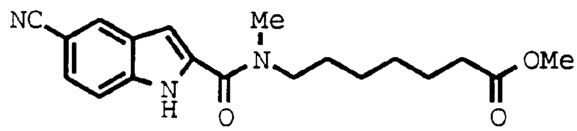

(14m)

A solution of methyl *N*-Cbz-*N*-methyl-7-aminoheptanoate (4.0 g, 13.0 mmol) (prepared in Part B of this Example) in EtOAc (150 ml) was treated with 10 % Pd/C (1.0 g) and the mixture was hydrogenated at atmospheric pressure for 2 hours. The catalyst was filtered and the organic solvent evaporated in vacuo to afford 2.20 g (98 %) of methyl N-methyl-7-aminoheptanoate which was directly coupled to 5-cyanoindole-2-carboxylate in 62% yield by following the procedure described in Example 9, Part C.
MS(FD) m/e, 341 (M+1).

| Anal Calculated for $C_{19}H_{23}N_3O_3$: | | | |
|---|---|---|---|
| Calculated | C 66.84, | H 6.79, | N 12.31; |
| Found | C 67.03, | H 6.88, | N 12.26. |

Part D Preparation of the intermediate (15m) :

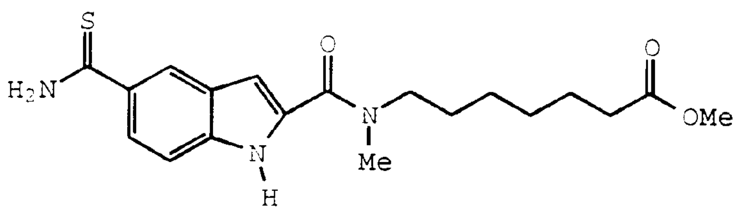

(15m)

The intermediate was prepared by the method of (15d) (Example 12, Part F) starting from (14m) to recover a 97 % yield of the desired product FDMS 375 $^{(M+)}$

| Analysis, Calculated for $C_{19}H_{25}N_3SO_3$ | | | |
|---|---|---|---|
| Calculated: | C, 60.78 | H, 6.71 | N, 11.19 |
| Found: | C, 60.75 | H, 6.370 | N, 11.26 |

Part E Preparation of the intermediate (16m) :

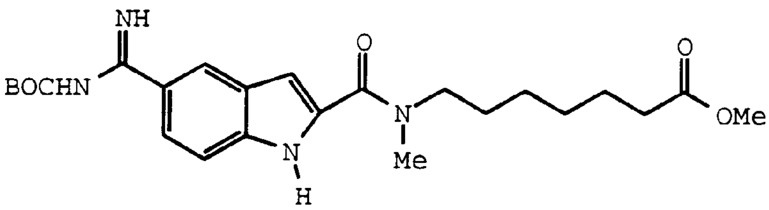

(16m)

The intermediate was prepared according to Example 12, Part G (16d) starting from (15m) in 89% yield. FDMS 459 (M+1)

Analysis, Calculated for $C_{24}H_{34}N_4O_5 \cdot 0.04\ CHCl_3$

| | | | |
|---|---|---|---|
| Calculated: | C, 62.32 | H, 7.59 | N, 11.85 |
| Found: | C, 62.16 | H, 7.40 | N, 12.09 |

Part F Preparation of the intermediate (17g) :

The intermediate was prepared according to Example 13, Part H (17d) starting from (16m) in 31% yield. FDMS 445 (M+1)

Part G Preparation of the title compound (18m):

The title compound was prepared as the TFA salt according to Example 9, Part G (18a) starting from (17g) in 81% yield. FDMS 345 (M+1)
$^1$H NMR (DMSO/TMS) : $\partial$ = 1.2 (s 4H), 1.4-1.8 (m, 4H), 2-2.2 (m, 2H), 2.9-3.7 (m, 5H), 7.0 (s, 1H), 7.5-7.6 (m, 2H), 8.2 (s, 1H), 8.8 (s, 2H), 9.1 (s, 2H), 12.1 (s, 1H)

| Analysis, Calculated for $C_{20}H_{25}F_3N_4O_5$ | | | |
|---|---|---|---|
| Calculated: | C, 52.40 | H, 5.50 | N, 12.22 |
| Found: | C, 52.53 | H, 5.49 | N, 11.97 |

Example 22

Preparation of Compound (18n) :

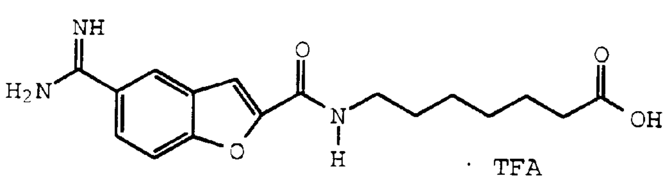

(18n)

Part A Preparation of the intermediate, (14n):

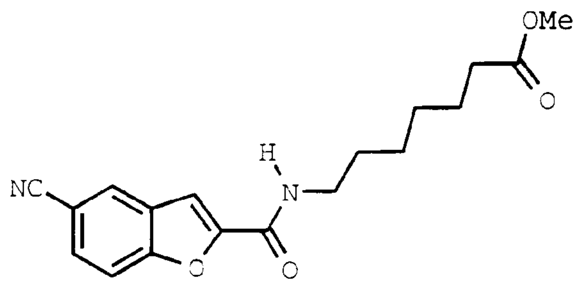

(14n)

Intermediate (14n) was prepared in 48% yield from 5-cyanobenzofuran-2-carboxylate and methyl 7-heptanoate by following the procedure described in Example 9, Part C. MS(FD), m/e 328 (M+).

| Anal Calcd. for $C_{18}H_{20}N_2O_4$: | | | |
| --- | --- | --- | --- |
| Calculated | C 65.84, | H 6.14, | N 8.53; |
| Found | C 65.87, | H 6.29, | N 8.59. |

Part B Preparation of the intermediate (15n):

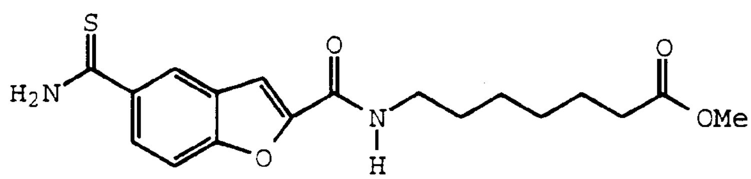

(15n)

The intermediate was prepared according to Example 12, Part F (15d) starting from (14n) to afford a 97 % yield.
FDMS 362 (M+)

| Analysis, Calculated for $C_{18}H_{22}N_2SO_4$ | | | |
| --- | --- | --- | --- |
| Calculated: | C, 59.65 | H, 6.12 | N, 7.73 |
| Found: | C, 59.43 | H, 6.15 | N, 7.59 |

Part C Preparation of the intermediate (16n):

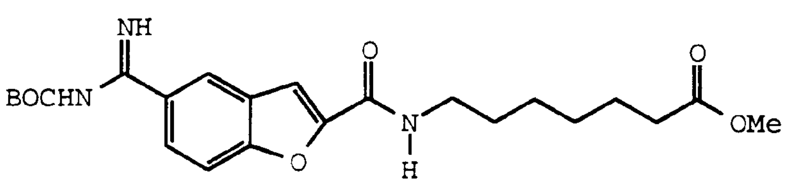

(16n)

The intermediate was prepared according to Example 12, Part G (16d) starting from (15n) to recover a 58 % yield of the desired product FDMS 446 (M+1)

Analysis, Calculated for $C_{23}H_{31}N_3O_6$· .09 $CHCl_3$

| Analysis, Calculated for $C_{23}H_{31}N_3O_6$• .09 $CHCl_3$ | | | |
|---|---|---|---|
| Calculated: | C, 60.78 | H, 6.87 | N, 9.21 |
| Found: | C, 60.85 | H, 7.01 | N, 9.17 |

Part D Preparation of the title compound (18n)

Compound (16n) (0.22 g, 0.5 mmol) in MeOH (10 ml) was treated with a solution of 1 N aqueous NaOH (2.5 ml) at ambient temperature overnight (16 hours). The reaction was quenched with 1 N aqueous HCl (2.5 ml) and concentrated to solid under reduced pressure. The product was then mixed with toluene and reconcentrated. The resulting solid was mixed with anhydrous anisole (5 ml) and trifluoroacetic acid (5 ml). The reaction mixture was stirred at ambient temperature for 4 hours. After concentrating under reduced pressure the resulting solid was triturated with a mixture of hexane and $H_2O$ and filtered to recover 155 mg (70 %) of the title compound as the TFA salt. FDMS 332 (M+1)

$^1H$ NMR (DMSO/TMS): $\partial$ = 1.1-1.3 (m, 4H), 1.4-1.6 (m, 4H), 2.1 (t, 2H), 3.2-3.4 (m, 2H), 7.6 (s, 1H) , 7.8-7.9 (m, 2H), 8.2 (s, 1H), 8.8 (t, 1H), 9.1 (s, 2H), 9.3 (s, 2H), 11.9 (s, 1H)

| Analysis, Calculated for $C_{19}H_{22}F_3N_3O_6$•0.14 $CF_3COOH$ | | | |
|---|---|---|---|
| Calculated: | C, 50.19 | H, 4.84 | N, 9.11 |
| Found: | C, 50.45 | H, 4.89 | N, 8.69 |

Example 23

Preparation of Compound (18o) :

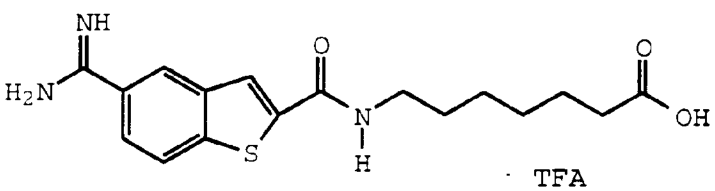

· TFA

(18o)

Part A Preparation of the intermediate (14o) :

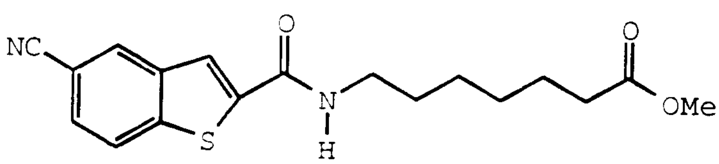

(14o)

Intermediate (14o) was prepared in 47% yield from 5- cyanobenzothiophene-2-carboxylate (Example 5, Part B) and methyl 7-aminoheptanoate by following the procedure described in Example 9, Part C.
FDMS 344 (M+)

| Analysis, Calculated for $C_{18}H_{20}N_2SO_3$ | | | |
|---|---|---|---|
| Calculated: | C, 62.77 | H, 5.85, | N, 8.13 |
| Found: | C, 62.77 | H, 5.87 | N, 8.08 |

Part B Preparation of the intermediate (15o) :

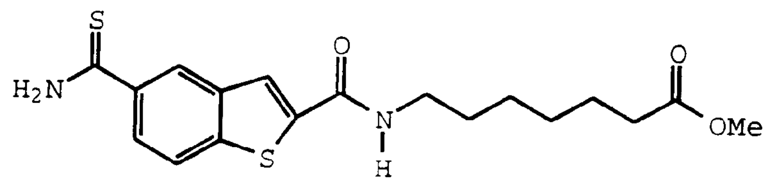

(15o)

The intermediate was prepared by the method of (15d) (Example 12, Part F) starting from (14o) to recover a quantitative yield of the desired product.

Part C Preparation of the intermediate (16o) :

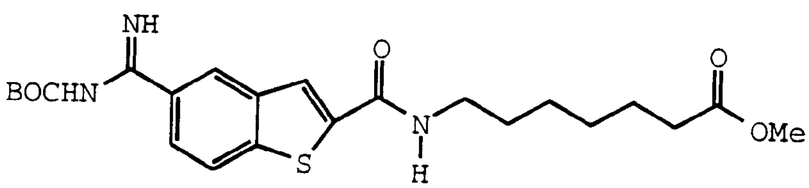

(16o)

The intermediate was prepared according to Example 12, Part G (16d) starting from (15o) in 67% yield.

Part D Preparation of the title compound (18o) :

The title compound was prepared as the TFA salt according to Example 22, Part D (18n) starting from (16o) in 60% yield. FDMS 348 $^{(M+1)}$
$^1H$ NMR (DMSO/TMS): ∂ = 1.4 (m, 4H), 1.5 (m, 4H), 2.2 (t, 2H), 3.2 (m, 2H), 7.7 (d, 1H), 8.1 (s, 1H), 8.2 (d, 1H), 8.8 (t, 1H), 9.2 (bs, 2H), 9.3 (bs, 2H), 11.9 (bs, 1H)

| Analysis, Calculated for $C_{17}H_{21}N_3O_3$•0.87 $CF_3COOH$ | | | |
|---|---|---|---|
| Calculated: | C, 50.40 | H, 4.94 | N, 9.41 |
| Found: | C, 50.40 | H, 4.73 | N, 9.20 |

Example 25

Preparation of Compound (18q) :

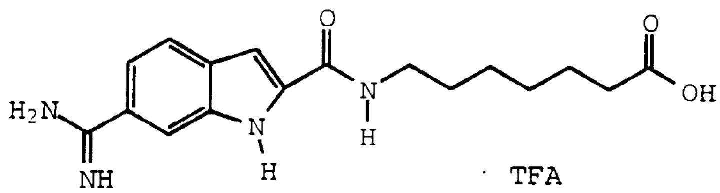

· TFA

(18q)

Part A Preparation of the intermediate (14p) :

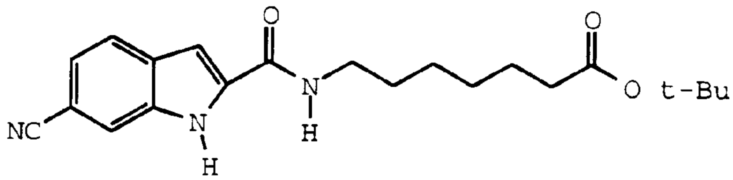

(14p)

Intermediate (14p) was prepared according to Example 9, Part C starting from (6c) and (40) (from Example 2, Part C) to afford a 59 % yield of product.
FDMS 369 (M+)

Analysis, Calculated for $C_{21}H_{27}N_3O_3$

| | | | |
|---|---|---|---|
| Calculated: | C, 68.72 | H, 7.37 | N, 11.37 |
| Found: | C, 68.51 | H, 7.50 | N, 11.41 |

Part D Preparation of the intermediate (15p) :

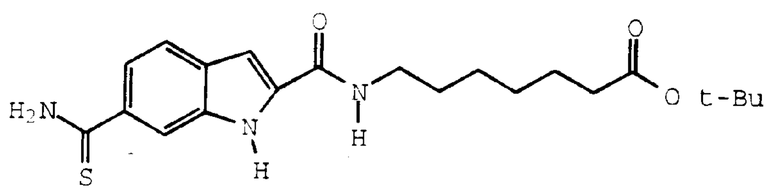

15p

The intermediate was prepared according to Example 12, Part F (15d) Starting from (41) prepared in Part C of this Example) to recover a 56 % yield of the desired product FDMS. 403 (M+)

| Analysis, Calculated for $C_{21}H_{29}N_3SO_3$ | | | |
|---|---|---|---|
| Calculated: | C, 62.50 | H, 7.34 | N, 10.41 |
| Found: | C, 62.44 | H, 7.24 | N, 10.51 |

Part E Preparation of the intermediate (16p) :

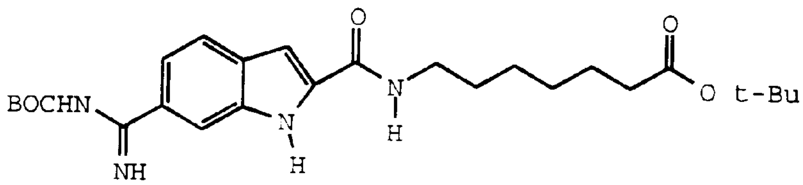

(16p)

Prepared according to Example 12, Part G (16d) starting from (15p) in 74% yield. FDMS 487 (M+1)

| Analysis, Calculated for $C_{26}H_{38}N_4O_5$• .21 $CHCl_3$ | | | |
|---|---|---|---|
| Calculated: | C, 61.52 | H, 7.53 | N, 10.95 |
| Found: | C, 61.62 | H, 7.76 | N, 10.62 |

Part F Preparation of the title compound (18q) :

The title compound was prepared as the TFA salt according to Example 9, Part G (18a) starting from (16p) in 86% yield.
FDMS 331 (M+1)
$^1$H NMR ($CDCl_3$/TMS) : ∂ = 1.3 (m, 4H), 1.5 (m, 4H), 2.2 (t, 2H), 3.2 (q, 2H), 7.2 (s, 1H), 7.4 (d, 1H), 7.8 (m, 2H), 8.6 (t, 1H), 8.9 (s, 2H), 9.2 (s, 2H), 12.2 (s, 1H)

| Analysis, Calculated for $C_{19}H_{23}F_3N_4O_5$ | | | |
|---|---|---|---|
| Calculated: | C, 51.35 | H, 5.22 | N, 12.61 |
| Found: | C, 51.12 | H, 5.02 | N, 12.48 |

Example 26

Preparation of Compound (18s) :

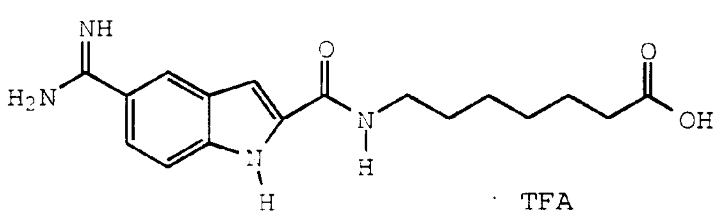

(18s)

Part A Preparation of intermediate (14r) :

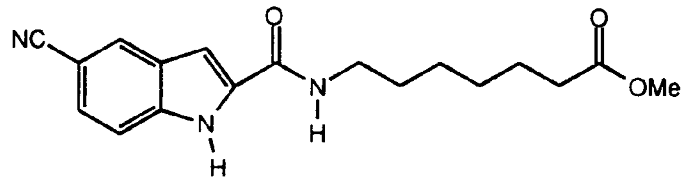

(14r)

A solution of 5-cyanoindole-2-carboxylic acid (2.75 g, 14.8 mmol) (from Example 1, Part D), dimethylaminopyridine (3.50 g, 28.6 mmol), diisopropylethylamine (4.0 ml, 23.0 mmol) and ethyl 7-aminoheptanoate hydrochloride (4.0 g, 19.1 mmol) in $CH_2Cl_2$ (100 ml) was treated with 1-(3-dimethylaminopropyl)-3-ethylcarbodiimide hydrochloride (3.70 g, 19.3 mmol). The reaction was stirred at ambient temperature for 48 hours at which time it was washed sequentially with 1 N aqueous citric acid (50 ml) and $H_2O$ (50 ml). The organic phase was dried over $Na_2SO_4$ and evaporated to give 4.1 g of a tan solid which was recrystallized from THF/EtOAc to afford 3.25 g (33 %) of the title compound as a white solid.
MS(FD), m/e 341 (M+1).

Anal Calcalculated for $C_{19}H_{23}N_3O_3$:

| | | | |
|---|---|---|---|
| Calculated: | C 66.84, | H 6.79, | N 12.31; |
| Found: | C 66.62, | H 6.75, | N 12.38. |

Part B Preparation of the title compound (18s):

The title compound was prepared by treating a slurry of (14r) (1.50 g, 4.18 mmol) in EtOH 250 ml with a stream of HCl gas for 30 min. resulting in a clear solution. The reaction was capped and stirred for 48 hours at ambient temperature at which time it was concentrated in vacuo. The residue was resuspended in EtOH 250 ml and the mixture treated with a stream of ammonia gas for 30 min. The mixture was capped and was stirred at room temperature for 24 hours at which time it was treated with ammonia gas for an additional 30 min. After stirring for 72 hours, the reaction was evaporated in vacuo to afford 820 mg of a solid which was taken on directly to the next reaction. A solution of 260 mg (0.65 mmol) of the above ester in THF (13 ml) was treated with 0.1 N aqueous LiOH (13 ml) and the reaction stirred for 16 hours. The organic solvent was evaporated and the aqueous layer acidified to pH 4 with 1 N aqueous HCl. The solid was filtered and dried to afford 151 mg (67 %) of the title compound as hydrochloride salt.

Example 27

Preparation of Compound (18t) :

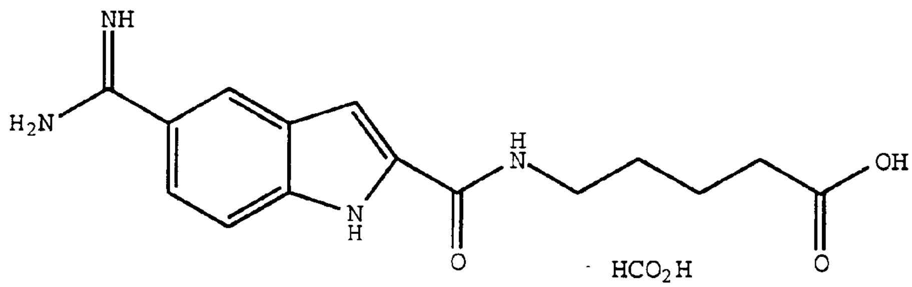

(18t)

Part A Preparation of the intermediate (14s) :

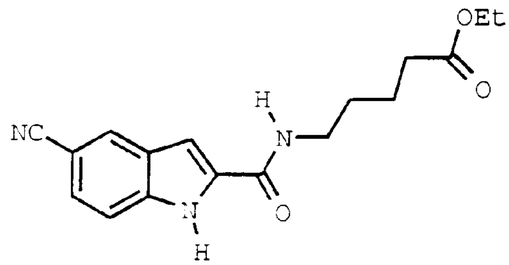

(14s)

Compound (14s) was prepared according to Example 26, Part A (14r) by substituting ethyl 5-aminopentanoate hydrochloride for ethyl 7-aminoheptanoate hydrochloride. The product was isolated as a tan solid which was recrystallized from THF/EtOAc to afford 2.65 g (53 %) of the title compound as a white solid.
FD-MS 313 (M+).

| Elemental Analysis Calculated For $C_{17}H_{19}N_3O_3$: | | | |
|---|---|---|---|
| Calculated: | C 65.15, | H 6.11, | N 13.41. |
| Found: | C 64.93, | H 6.09, | N 13.60. |

Part B Preparation of the title compound (18t):

A 5°C solution of the nitrile ester (14s) (1.50 g, 4.79 mmol) in EtOH (130 ml) was treated with a stream of HCl gas for 15 min. This was repeated once a day for 6 days at which time the solvent was evaporated in vacuo to give a white solid. The residue was taken up in 250 ml of EtOH and was treated with a stream of $NH_3$ gas. After 48 hours, the solvent was evaporated in vacuo to afford 1.63 g of a white solid which was purified by reverse phase HPLC on a Rainin Dynamax C-18 column using 50 % $CH_3CN$ and 1 % $CH_3CO_2H$ in $H_2O$ as the eluent. The homogeneous fractions were combined, the organic solvents were evaporated in vacuo, and the aqueous phase was lyophilized to afford 1.04 g (2.84 mmol, 59 %) of the imino ether as the hydrochloride salt.
FD-MS 331 (M+1, 100).
Elemental Analysis Calculated For $C_{17}H_{23}ClN_4O_3$: C 55.66, H 6.32, N 15.27. Found C 55.42, H 6.29, N 15.15.

A solution of the above imidate (L14-7VK-113) (240 mg, 0.65 mmol) in 13 ml THF was treated with 13.1 ml of 0.1 N aqueous LiOH solution. The reaction was stirred at ambient temperature for 16 hours at which time the THF was evaporated in vacuo. The aqueous layer was acidified to pH 4 with 1 N aqueous HCl. The resulting solid filtered and dried to give 195 mg of material which was purified by reverse phase chromatography over a Novapak C18 column using 0.5 % ammonium formate and 40 % MeOH in $H_2O$ as the eluent. The homogeneous fractions were combined, the MeOH was evaporated in vacuo, and the resulting aqueous layer lyophilized to afford 120 mg (0.34 mmol; 52 %) of the title compound as the formate salt.

FD-MS 303 (M+, 100)
HRMS Calculated for $C_{15}H_{18}N_4O_3$ 303.1457. Found 303.1446

| Elemental Analysis Calculated For $C_{16}H_{20}N_4O_5$: | | | |
|---|---|---|---|
| Calculated: | C 55.17, | H 5.79, | N 16.08. |
| Found: | C 55.92, | H 5.97, | N 15.83. |

Example 28

Preparation of Compound (18u) :

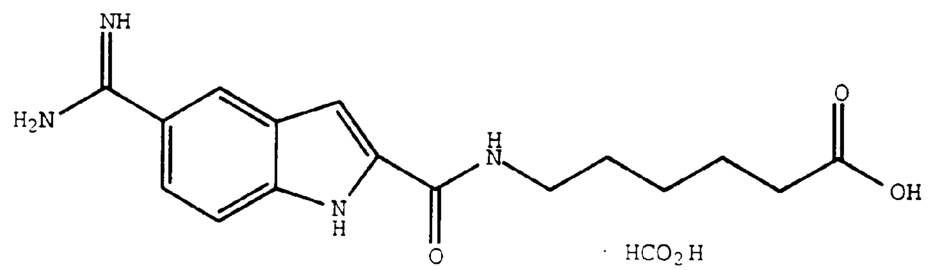

(18u)

Part A Preparation of the intermediate nitrile ester :

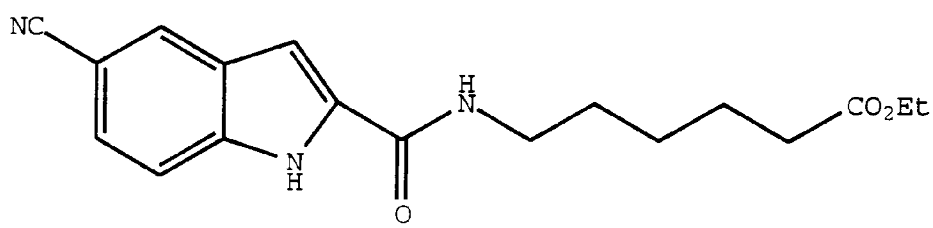

(14t)

Intermediate nitrile ester was prepared according to Example 26, Part A (14r) by substituting ethyl 6-aminohexanoate hydrochloride for ethyl 7-aminoheptanoate hydrochloride. The product was isolated as a white solid which was recrystallized from THF/EtOAc to afford 2.30 g (44 %) of the title compound as a white solid. FD-MS 327 (M+).

| Elemental Analysis Calculated For $C_{18}H_{21}N_3O_3$: | | | |
|---|---|---|---|
| Calculated: | C 66.04, | H 6.47, | N 12.84 |
| Found: | C 65.75, | H 6.45, | N 12.80. |

Part B Preparation of the title compound (18u) :

A 5°C solution of 1.50 g (4.39 mmol) of the nitrile prepared in Part A) in 130 ml of EtOH was treated with a stream of HCl gas for 15 min. This was repeated once a day for 5 days at which time the solvent was evaporatea in vacuo to give a white solid. The residue was taken up in 130 ml of EtOH and was treated with a stream of $NH_3$ gas for 15 min. After 96 hours, the solvent was evaporated in vacuo to afford 1.63 g of a white solid which was purified by reverse phase HPLC on a Rainin Dynamax C-18 column using a gradient of 45 % to 55

% $CH_3CN$ and 1 % $CH_3CO_2H$ in $H_2O$ as the eluent. The homogeneous fractions were combined, the organic solvents were evaporated in vacuo, and the aqueous phase was lyophilized to afford 1.76 g (51 %) of the title compound as the hydrochloride salt.
FD-MS 345 (M+1).

| Elemental Analysis Calculated For $C_{18}H_{24}ClN_4O_3$: | | | |
|---|---|---|---|
| Calculated | C 56.76, | H 6.62, | N 14.71. |
| Found | C 56.52, | H 6.619, | N 14.60. |

A solution of 250 mg (0.66 mmol) of the imidate (L14-7VK-111) in THF (13 ml) was treated with 0.1 N aqueous LiOH solution (13.2 ml). The reaction was stirred at ambient temperature for 16 hours at which time the THF was evaporated *in vacuo.* The aqueous layer was acidified to pH 4 with 1 N aqueous HCl. The resulting solid filtered and dried to give 243 mg of material which was purified by reverse phase chromatography over a Novapak C18 column using 0.5 % ammonium formate and 35 % MeOH in $H_2O$ as the eluent. The homogeneous fractions were combined, the MeOH was evaporated in vacuo, and the resulting aqueous layer lyophilized to afford 184 mg (0.58 mmol; 88%) of the title compound.
FAB-MS 317 (M+, 100)
HRMS Calculated for $C_{16}H_{20}N_4O_3$ 317.1614. Found 317.1570

| Elemental Analysis Calculated For $C_{16}H_{20}N_4O_5$: | | | |
|---|---|---|---|
| Calculated | C 60.75, | H 6.37, | N 17.71. |
| Found | C 61.03, | H 6.47, | N 17.50. |

Example 29

Preparation of the aminomethyl basic functional compound (52b) :

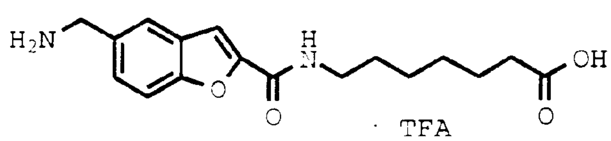

(52b)

Part A Preparation of the intermediate (51b):

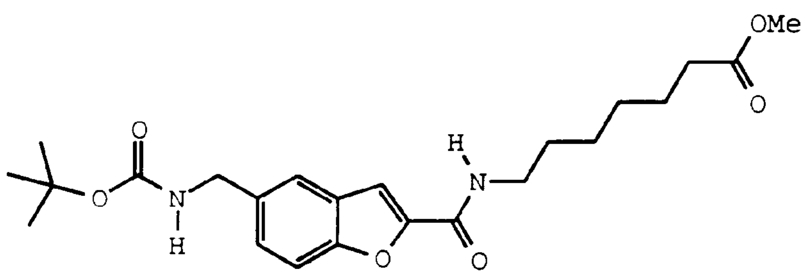

(51b)

A solution of (14n) (0.5 g, 1.5 mmol) (from Example 22, Part A) in EtOH (100 ml) and $NH_3$ (5 ml) was treated with Raney Ni (0.25 g) and the mixture was hydrogenated at 500 psi (35.15 Kg/cm$^2$) for 9 hours at 90°C. The reaction was filtered, evaporated in vacuo, and the residue taken up in 50 ml THF. The solution was treated with $K_2CO_3$ (2.20 g, 16 mmol) followed by 15 ml of $H_2O$. The reaction was stirred for 18 hours at room temperature and the organic layer was separated and dried over $Na_2SO_4$. Evaporation of the solvent in vacuo gave

1.30 g of crude product which was purified by radial chromatography ($SiO_2$; 40 % EtOAc in hexane) to afford 310 mg (48%) of (51b) as a white solid.
MS(FD) m/e, 432 (M+).

| Anal Calcd for $C_{23}H_{32}N_2O_2$ (0.1 $H_2O$) : | | | |
|---|---|---|---|
| Calculated | C 63.61, | H 7.47, | N 6.45; |
| Found | C 63.87, | H 7.46, | N 6.48. |

Part B Preparation of the title compound (52b) :

A solution of 51b (280 mg, 0.65 mmol) in THF (10 ml) was treated with of LiOH (75 mg, 3.3 mmol) followed by 3 ml of $H_2O$. The reaction was heated to mild reflux for 6 hours and was concentrated in vacuo. The residue was partitioned between 50 ml of $H_2O$ and 50 ml EtOAc. The organic layer was separated and was washed with of 2 N aqueous NaOH (10 ml). The combined aqueous layers were acidified to ~pH 5 with solid citric acid and were extracted with EtOAc (2 x 50 ml). The combined EtOAc extracts were dried over $Na_2SO_4$ and evaporated in vacuo to afford 346 mg of the crude acid. Purification by radial chromatography ($SiO_2$; EtOAc) gave 200 mg of the acid as a white solid.

The acid (200 mg) was treated with 10 ml of a 10% anisole in TFA solution and the mixture stirred at room temperature for 3 hours. The reaction was concentrated in vacuo and the residue partitioned between 30 ml of $H_2O$ and 30 ml EtOAc. The aqueous layer was separated, washed with 30 ml EtOAc, acidified to pH 1 with 5 N aqueous HCl, and lyophilized to afford 45 mg of the title compound as the TFA salt.
MS(FD) m/e, 304 (M+1).

Example 30

Preparation of (18w) :

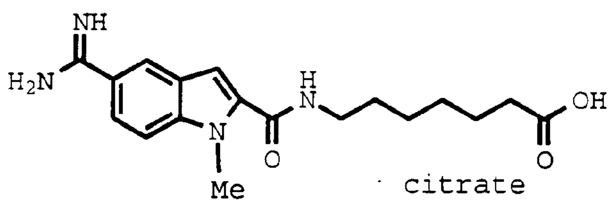

(18w)

Part A Preparation of (14v) :

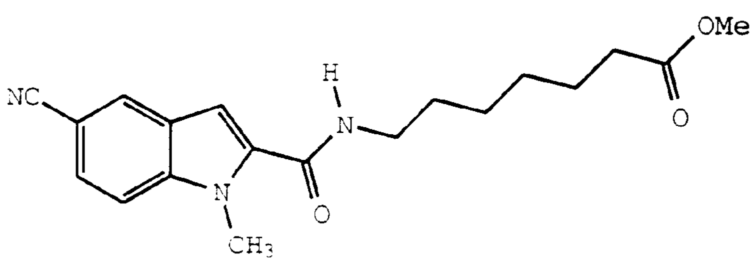

(14v)

A solution of methyl 1-methyl-5-cyanoindole-2-carboxylate (48) (5.04 g, 23.5 mmol) (prepared in Example 4, Part A) in THF (250 ml) was treated with a solution of LiOH (0.675 g, 28.2 mmol) in $H_2O$ (250 ml). The mixture was stirred for 16 hours at room temperature at which time the organic solvent was evaporated in vacuo. The aqueous layer was acidified to pH 4 with solid citric acid and was extracted with EtOAc (4 x 50 ml). The combined organic layers were dried over $Na_2SO_4$ and evaporated to give 4.10 g of 5-cyano-1-methylindole-2-carboxylic acid as an off-white solid.

A solution of 3.25 g of the crude acid of Part A, 4-dimethylaminopyridine (2.55 g, 20.1 mmol), methyl 7-aminoheptanoate hydrochloride (4.11 g, 21.0 mmol), and diisopropylethylamine (8.80 ml, 50.6 mmol) in $CH_2Cl_2$ (100 ml) was treated with 1-(3-dimethylaminopropyl)-3-ethylcarbodiimide hydrochloride (4.0 g, 20.9 mmol). The reaction was stirred at room temperature for 18 hours and was washed sequentially with $H_2O$ (2 x 100 ml), 1 M aqueous citric acid (100 ml), $H_20$ (100 ml), saturated aqueous $NaHCO_3$ (100 ml) and $H_2O$ (100 ml). The organic layer was dried over $Na_2SO_4$ and was evaporated in vacuo to give a solid which was recrystallized from THF/EtOAc to afford 4.62 g of the title compound as a white solid.
MS(FD), m/e 341 (M+; 100).

| Anal Calcd for $C_{19}H_{23}N_3O_3$: | | | |
|---|---|---|---|
| Calculated | C 66.84, | H 6.79, | N 12.31; |
| Found | C 66.70, | H 6.90, | N 12.36. |

Part B Preparation of the final product (18w) :

A slurry of (14v) (750 mg, 2.20 mmol) in of EtOH (80 ml) was treated with a stream of HCl gas for 30 min. resulting in a clear solution. The reaction capped and was stirred for 48 hours at ambient temperature at which time it was concentrated in vacuo. The residue was resuspended in EtOH (80 ml) and the mixture treated with a stream of ammonia gas for 30 min. The mixture was capped and was stirred at room temperature for 48 hours at which time it was evaporated in vacuo to afford 900 mg of a solid which was purified by reverse phase chromatography over a Nova C-18 column using a gradient of 0.5 % $NH_4OAc$ in 25 % $CH_3CN$ in $H_2O$ to 0.5 % $NH_4OAc$ in 50 % $CH_3CN$ in $H_2O$ as the eluent to afford 750 mg of the amidino ester as a white solid.

A slurry of 500 mg (1.16 mmol) of the imino ether in THF (38 ml) was treated with 0.1 N aqueous LiOH (38 ml). The reaction was stirred at room temperature for 18 hours and the organic solvent evaporated in vacuo. The aqueous layer was acidified to pH 5 with 1 N aqueous citric acid and the resulting solid filtered and dried to afford 382 mg of the title compound as a citrate salt.
$^1H$ NMR (300 MHz; MeOH-$d_4$) d 8.20 (s, 1H), 7.73-7.65 (m, 2H), 7.16 (s, 1H), 4.05 (s 3H), 3.44-3.35 (m, 2H), 2.30 (t, J = 7.3 Hz, 2H), 1.72-1.55 (m, 4H), 1.46-1.35 (m, 4H).
MS(FD) m/e, 345 (M+1; 100).

| Anal Calcd for $C_{12}H_{10}N_2O_2$: | | | |
|---|---|---|---|
| Calculated | C 62.77, | H 7.02, | N 16.27; |
| Found | C 62.19, | H 7.06, | N 15.14. |

HRMS Calculated for $C_{18}H_{25}N_4O_3$ 345.1927; Found 345.1950

Example 31

Preparation of compound (18x) :

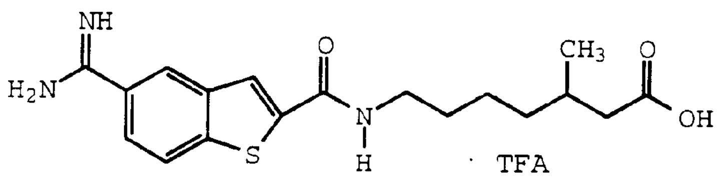

· TFA

(18x)

Part A Preparation of the intermediate (14w) :

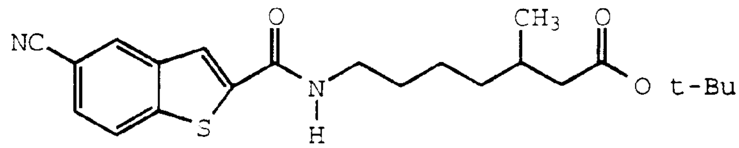

(14w)

The intermediate was prepared by the method of (14i) (from Example 17, Part E) by substituting 5-cyanobenzothiophene-2-carboxylic acid (35) (from Example 5, Part B) for 5-cyanoindole-2-carboxylic acid. The product was purified by chromatography ($SiO_2$, 5 % v/v $MeOH/CHCl_3$) to recover a 32% yield of desired product as an oil.
MS(FD), m/e 400 (M+)

Part B Preparation of the intermediate (15r):

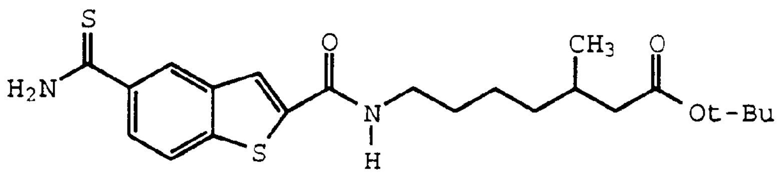

(15r)

The intermediate was prepared according to Example 12, Part F (15d) starting from (14w) to recover a 85 % yield of the desired product.
FDMS 434 (M+)

| Analysis, Calculated for $C_{22}H_{30}N_2S_2O_3$• .3 $H_2O$ | | | |
|---|---|---|---|
| Calculated: | C, 60.03 | H, 7.01 | N, 6.36 |
| Found: | C, 59.89 | H, 7.03 | N, 6.72 |

Part C Preparation of the intermediate (16r) :

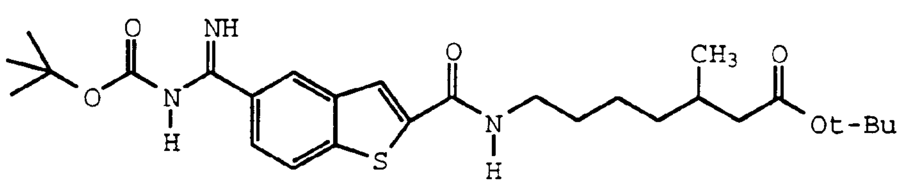

(16r)

The intermediate was prepared according to Example 12, Part G (16d) starting from (15r) to recover a 34 % yield of the desired product.

Part D Preparation of the title compound (18x) :

Compound (16r) (0.51 g, 1 mmol) was treated with anhydrous anisole (10 ml) and trifluoroacetic acid (10 ml) at ambient temperature overnight (16 hours). The mixture was concentrated under reduced pressure,

mixed with hot EtOAc/MeOH and filtered to recover to 215 mg (45%) of the title compound as the TFA salt. FDMS m/e 362 (M+1)

$^1$H NMR (DMSO/TMS) : ∂ = .8-.9 (d, 3H), 1.1-1.4 (m, 4H), 1.5-1.6 m, 2H), 1.8-1.9 (m, 1H), 1.9-2.3 (m, 2H), 3.2-3.3 (m, 2H), 7.8 (m, 1H) ,8.2 (s, 1H),8.2-8.3 (d, 1H), 8.4 (s, 1H), 8.8-8.9 (t, 1H), 9-9.1 (bs, 2H), 9.3-9.4 (bs, 2H), 11.9-12.0 (bs, 1H)

| Analysis, calculated for $C_{20}H_{24}F_3N_3O_5$ | | | |
|---|---|---|---|
| Calculated: | C, 50.52 | H, 5.09 | N, 8.84 |
| Found: | C, 50.63 | H, 5.21 | N, 8.90 |

Example 32

Preparation of compound (18y) :

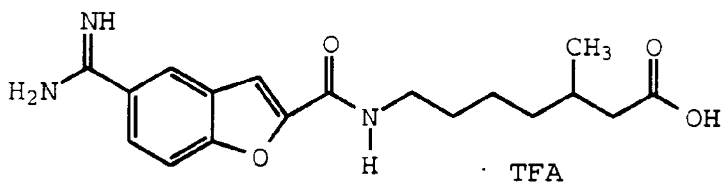

(18y)

Part A Preparation of intermediate (14x) :

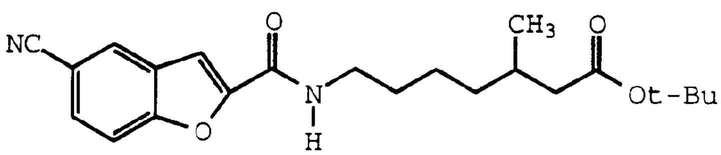

(14x)

The intermediate was prepared by the method of (14i) (from Example 17, Part E) starting from (22a) (from Example 17, Part C) and 5-Cyanobenzofuran-2-carboxylic acid (33) (from Example 6 Part D) to recover a 29 % yield of the desired product FDMS 384 (M+)

Part B Preparation of the intermediate (15s) :

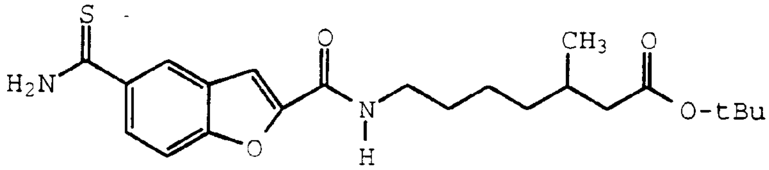

(15s)

The intermediate was prepared according to Example 12, Part F (15d), starting from (14x) to recover a 92 % yield of the desired product FDMS 418 (M+)

| Analysis, Calculated for $C_{20}H_{30}N_2SO_4$ | | | |
|---|---|---|---|
| Calculated: | C, 63.13 | H, 7.23 | N, 6.69 |
| Found: | C, 63.17 | H, 7.21 | N, 6.83 |

Part C Preparation of the intermediate (16s):

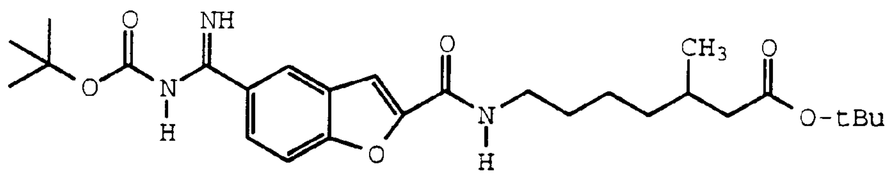

(16s)

The intermediate was prepared according to Example 12, Part G (16d) starting from (15s) to recover a 46 % yield of the desired product.

Part D Preparation of the title compound (18y):

Compound (16s) (0.552 g, 1.1 mmol) was treated with anhydrous anisole (10 ml) and trifluoroacetic acid (10 ml) at ambient temperature overnight (16 hours). The mixture was concentrated under reduced pressure, mixed with hot EtOAc and filtered to recover 369 mg (73%) of the title compound as the TFA salt.
FDMS 346 $^{(M+1)}$
$^1$H NMR (DMSO/TMS): ∂ = .8-.9 (d, 3H), 1.1-1.4 (m,4H), 1.5-1.6 (m, 2H), 1.8-1.9 (m, 1H) , 1.9-2.3 (m, 2H), 3.2-3.4 (m, 2H), 7.7 (s, 1H) , 7.8-8 (m,2H), 8.3 (m, 1H) ,8.8-8.9 (t, 1H) , 9.2 (bs, 4H), 11.9-12.0 (bs, 1H)

| Analysis, calculated for $C_{20}H_{24}F_3N_3O_6 \bullet H_2O$ | | | |
|---|---|---|---|
| Calculated: | C, 50.31 | H, 5.49 | N, 8.80 |
| Found: | C, 49.94 | H, 5.10 | N, 8.99 |

Example 33

Preparation of compound (18z) :

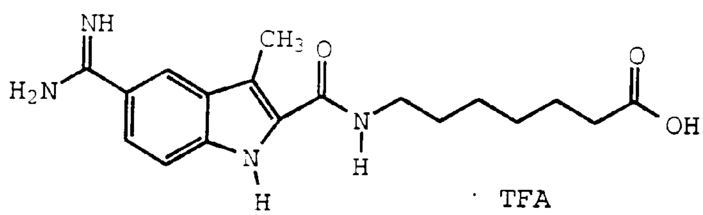

(18z)

Part A Preparation of the intermediate (14y) :

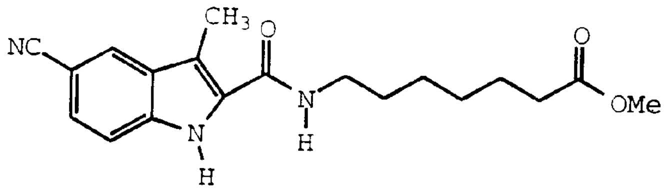

(14y)

The intermediate was prepared according to Example 23, Part A (14o) starting from 3-methyl-5-cyanoindole-2-carboxylic acid (62) (prepared in Example 3 Part C) and methyl-6-amino heptanoate hydrochloride to recover a 73 % yield of the desired product as a tan solid

| Analysis, calculated for $C_{19}H_{23}N_3O_3$ | | | |
|---|---|---|---|
| Calculated: | C, 66.84 | H, 6.79 | N, 12.31 |
| Found: | C, 66.79 | H, 6.86 | N, 12.32 |

Part B Preparation of the intermediate (15t) :

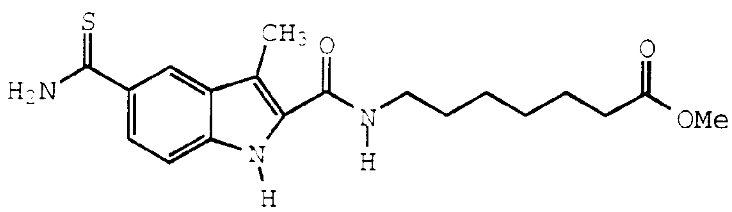

(15t)

The intermediate was prepared by the method of (150) (per Example 23, Part B) starting from (14y) to recover a quantitative yield of the desired product.

| Analysis, calculated for $C_{19}H_{25}N_3SO_3$•1 EtOAc | | | |
|---|---|---|---|
| Calculated: | C, 59.44 | H, 7.17 | N, 9.06 |
| Found: | C, 59.44 | H, 7.08 | N, 9.30 |

Part C Preparation of the intermediate (16t) :

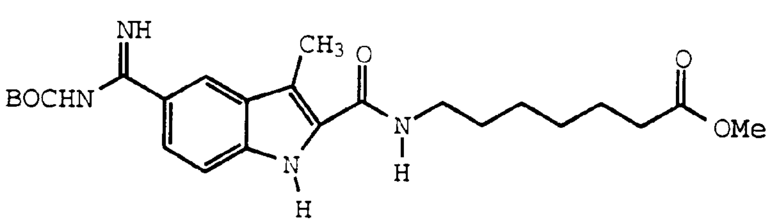

(16t)

The intermediate was prepared in a manner similar to (16o) (from Example 23, Part C) starting from (15t) in 13% yield.

Part D Preparation of the title compound (18y) :

The title compound was prepared as the TFA salt in a manner similar to (18n) (from Example 22, Part D) starting from (16t) in 60% yield. FDMS 345 (M+1)
$^1$H NMR (DMSO/TMS): ∂ = 1.3-1.4 (m,4H), 1.5-1.6 (m, 4H), 2.2 (t, 2H), 2.55 (s, 3H), 3.2-3.4 (m, 2H), 7.5-7.7 (m, 2H), 8.0 (t. 1H), 8.2 (s, 1H), 8.8 (bs, 2H), 9.2 (bs, 2H), 11.6 (s, 1H), 12.0 (s, 1H)

Analysis, Calculated for $C_{20}H_{25}F_3N_4O_5$

| | | | |
|---|---|---|---|
| Calculated: | C, 52.40 | H, 5.50 | N, 12.22 |
| Found: | C, 52.64 | H, 5.48 | N, 12.02 |

Example 34

Preparation of compound (18aa) :

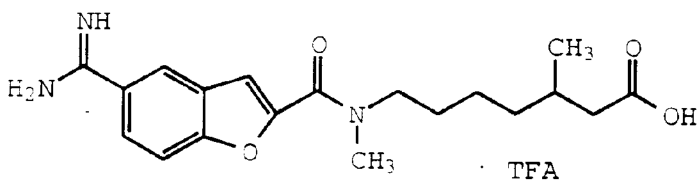

(18aa)

Part A Preparation of the intermediate (60):

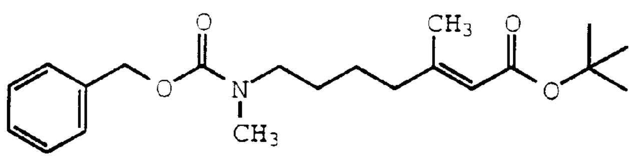

(60)

A 60% dispersion of NaH (1.7 g, 43.2 mmol) was washed with hexane. Dry DMF (40 ml) was added and the mixture cooled to 0°C. A solution of (22a) (from Example 17, part D) in dry THF (40 ml) was added portionwise and stirred for 1 hour at 0°C. This mixture was treated with methyl iodide (4 g, 28.8 mmol) and stirred at ambient temperature overnight. The reaction was cooled to 0°C and quenched with saturated aqueous $NH_4Cl$ dropwise. The resulting mixture was diluted with EtOAc and washed with $H_2O$ then brine. The organic layer was dried over $MgSO_4$ and concentrated under vacuum to 5.6 g oil which by NMR contained a small amount of DMF.

Part B Preparation of the intermediate (14z) :

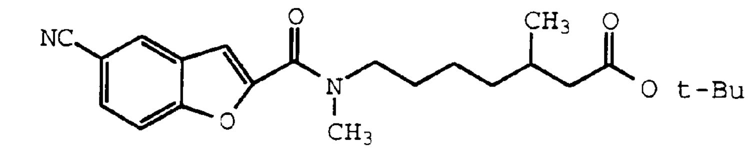

(14z)

The intermediate was prepared by the method of (14w) (from Example 31, Part A) starting from (60) and (33) (from Example 6 Part D) to recover a 75 % yield of the desired product.

Part C Preparation of the intermediate (15u) :

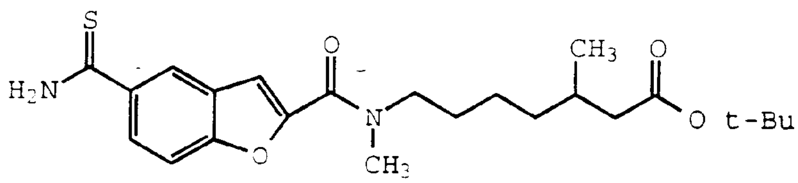

(15u)

The intermediate was prepared by the method of (15d) (from Example 12, Part F) starting from (14z) to recover a 85 % yield of the desired product FDMS 432(M+)

Part D Preparation of the intermediate (16u) :

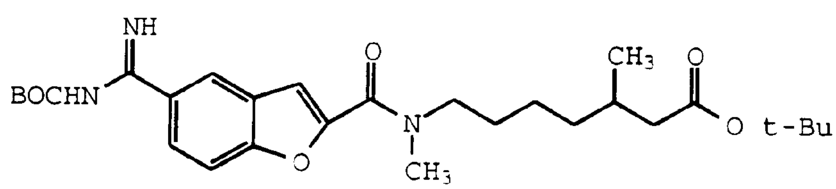

(16u)

The intermediate was prepared by the method of (16d) (from Example 12, Part G) starting from (15u) (prepared in Part C of this Example) to recover a 42 % yield of the desired product.

Part E Preparation of the final product (18aa) :

The title compound was prepared as the TFA salt by the method used to prepare (18x) (from Example 31, Part D) starting from (16u) (from Part D, of this Example) to recover a 66 % yield of the desired product. FDMS 474 (M+1)

$^1$H NMR (DMSO): ∂ = .8-.9 (m,4H), 1.0-1.4 (m, 4H), 1.4-1.6 (m, 2H), 1.7-1.9 (m, 1H), 1.9-2.0 (m, 1H), 2-2.2 (m, 1H), 2.8-3.6.(m, 4H), 7.4-7.6 (d, 1H), 7.8-8.0 (m, 2H),8.2 (s, 1H), 9-9.2 (bs,2H), 9.3-9.4 (bs, 2H), 11.9-12.0 (bs, 1H)

| Analysis, calculated for $C_{20}H_{24}F_3N_3O_5$ | | | |
|---|---|---|---|
| Calculated: | C, 53.28 | H, 5.54 | N, 8.88 |
| Found: | C, 53.45 | H, 5.59 | N, 8.65 |

Example 35

Preparation of the benzyloxy substituted benzofuran (62) :

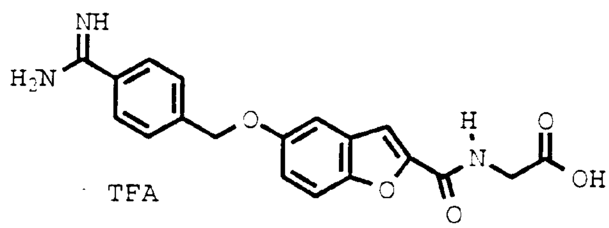

(62)

Part A Preparation of the intermediate (50) :

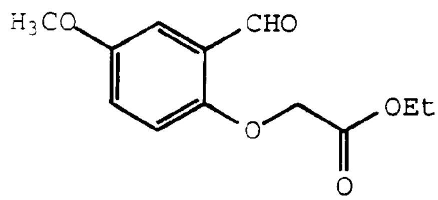

(50)

A mixture of p-methoxy salicylaldehyde (20 g, 131 mmol), potassium carbonate (19.9 g, 145 mmol), ethyl bromoacetate (24 g, 145 mmol) and DMF (30 ml) was treated with sodium iodide (4 g, 26 mmol) overnight at ambient temperature. The mixture was warmed to 50°C for an additional 24 hours. The reaction mixture was then poured into $H_2O$ and extracted with ethyl ether. The extracts were washed with $H_2O$, brine, and dried with $MgSO_4$. The mixture was concentrated in vacuo to give 33.66 g oil which was used in the next reaction without further purification.

Part B Preparation of the intermediate (55) :

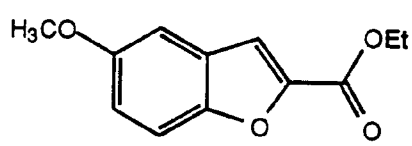

(55)

A mixture of the crude ethyl ester (50) (32.66 g, 137 mmol), 1,8-diazabicyclo[5.4.0]undec-7-ene (10 g, 66 mmol) and EtOH (100 ml) was refluxed for 2 hours, cooled to room temperature, then saturated $NH_4Cl$ (50 ml) added. The mixture was extracted with ether and the extracts washed with $H_2O$. It was then dried with $MgSO_4$, concentrated under reduced pressure, and purified by chromatography ($SiO_2$, 10% v/v EtOAc-Hexanes) to recover 17.72 g (59 %) of a crystalline solid.

Part C Preparation of the intermediate (56) :

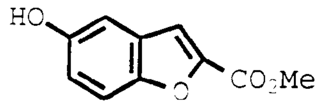

(56)

The methoxy ethyl ester (55) (17 g, 77.3 mmol) was heated at 175°C under argon with dry pyridine hydrochloride 19 hours. The mixture was cooled to room temperature and mixed with 1 N aqueous HCl and EtOAc. The organic layer was washed with 1 N aqueous HCl then brine, dried with $MgSO_4$ and concentrated in vacuo to 12.2 g solid.

The solid was mixed with dry MeOH (100 ml) and HCl gas was bubbled into the solution for 10 minutes. This mixture was stirred overnight at ambient temperature then concentrated to 12.83 g (86%) solid.
FDMS 192 (M+)

| Analysis, Calculated for $C_{10}H_8O_4$ | | |
|---|---|---|
| Calculated: | C, 62.50 | H, 4.20 |
| Found: | C, 62.76 | H, 4.25 |

Part D Preparation of the intermediate (57) :

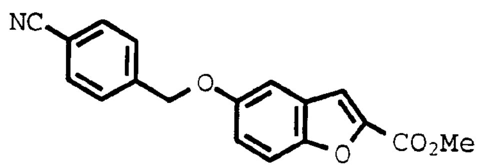

(57)

Phenol (56) (5.7 g, 29.7 mmol) was heated to reflux with potassium carbonate (8.2 g, 59.3 mmol), p-cyanobenzylbromide (6.4 g, 33 mmol) and acetone (50 ml) overnight. The mixture was concentrated in vacuo to dryness and mixed with EtOAc and $H_2O$ to get a precipitate which was filtered and rinsed with EtOAc then $H_2O$. Recovered 6.5 g (71%) white solid.
FDMS 307 (M+)

| Analysis, Calculated for $C_{18}H_{13}NO_4$ | | | |
|---|---|---|---|
| Calculated: | C, 70.35 | H, 4.26 | N, 4.56 |
| Found: | C, 70.57 | H, 4.30 | N, 4.44 |

Part E Preparation of the intermediate (58)

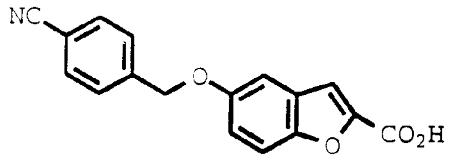

(58)

The methyl ester (57) (7 g, 22.8 mmol), was mixed with EtOH (100 ml) and 1 N aqueous NaOH (25 ml). This mixture was refluxed overnight, cooled to room temperature and neutralized with 1 N aqueous. HCl. The mixture was concentrated in vacuo gave a solid which was mixed with $H_2O$ and EtOAc. This mixture was filtered to yield 4.35 g (65%) solid which was recrystallized from a small amount from MeOH to obtain an analytically pure sample.
FDMS 293 $^{(M+)}$

| Analysis, Calculated for $C_{17}H_{11}NO_4$ | | | |
|---|---|---|---|
| Calculated: | C, 69.62 | H, 3.78 | N, 4.78 |
| Found: | C, 69.74 | H, 3.78 | N, 4.79 |

Part F Preparation of the intermediate (59) :

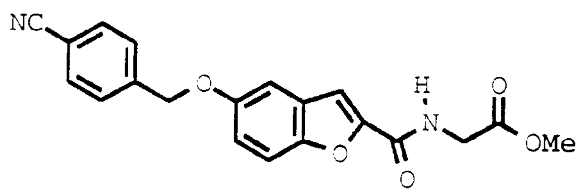

(59)

The intermediate was prepared according to Example 9, Part C (14a) using as starting material (58) and glycine hydrochloride methyl ester.
FDMS 364 $^{(M+)}$

| Analysis, Calculated for $C_{20}H_{16}N_2O_5$ | | | |
|---|---|---|---|
| Calculated: | C, 65.93 | H, 4.43 | N, 7.69 |
| Found: | C, 65.68 | H, 4.51 | N, 7.58 |

Part G Preparation of the intermediate (60) :

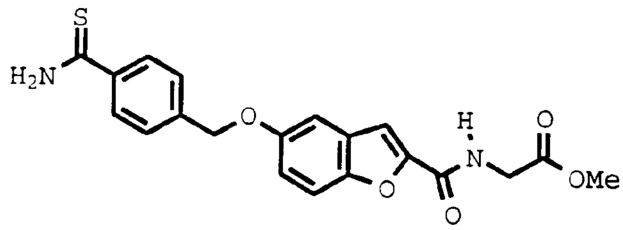

(60)

The intermediate was prepared from (59) by the method of (15a) (from Example 9 Part D) to recover a 92% yield of the desired product. An analytical sample was obtained by trituration with hot $CHCl_3$/MeOH. FDMS 398 (M+)

| Analysis, Calculated for $C_{20}H_{18}N_2SO_5$ | | | |
|---|---|---|---|
| Calculated: | C, 60.29 | H, 4.55 | N, 7.03 |
| Found: | C, 60.43 | H, 4.55 | N, 7.02 |

Part H Preparation of the intermediate (61) :

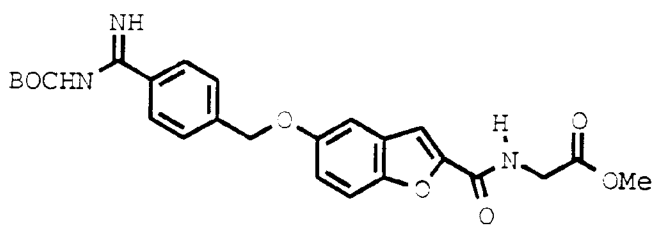

(61)

The intermediate was prepared from (60) according to Example 9 Part E (16a) in a 34% yield. FDMS 482 (M+1)

| Analysis, Calculated for $C_{25}H_{27}N_3O_7$• .29 $CHCl_3$ | | | |
|---|---|---|---|
| Calculated: | C, 51.82 | H, 4.23 | N, 8.02 |
| Found: | C, 51.59 | H, 4.09 | N, 7.71 |

Part I Preparation of the title compound (62):

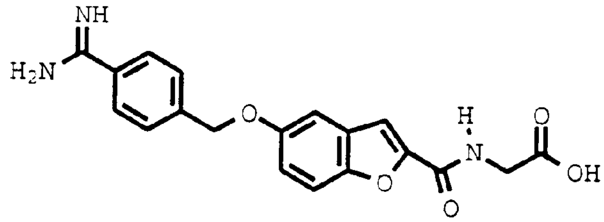

(62)

The title compound (62) was prepared as the TFA salt from (61) by the procedure of (18n) (from Example 22 Part D) in a 76% yield.
$^1$H NMR (DMSO-$d_6$) : ∂ = 3.9-4.0 (d, 2H), 5.3 (s, 2H), 7.2 (m,1H), 7.4 (d, 1H), 7.5 (s, 1H), 7.6-7.7 (m, 1H), 7.7-7.9 (m, 4H), 8.9-9.0 (t, 1H), 9.0-9.1 (bs, 2H), 9.3-9.4 (bs, 2H), 12.7 (bs, 1H) FDMS 368 $^{(M+1)}$

| Analysis, Calculated for $C_{21}H_{18}F_3N_3O_7$• .4 $H_2O$, .4 EtOAc | | | |
|---|---|---|---|
| Calculated: | C, 51.82 | H, 4.23 | N, 8.02 |
| Found: | C, 51.59 | H, 4.09 | N, 7.71 |

Example 36

Preparation of compound with an aminomethyl base functional compound (52a) :

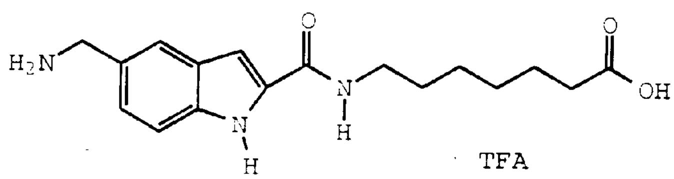

· TFA

(52a)

Part A Preparation of the intermediate (51a) :

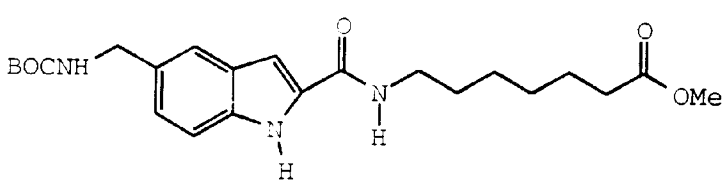

(51a)

A mixture of (14r) (1.50 g, 4.58 mmol) (from Example 26, Part A) and Raney Ni (0.5 g) in MeOH (100 ml) and of $NH_3$ (15 ml) was hydrogenated at 500 psi (36.53 Kg/cm$^2$) for 9 hours at 90°C. The reaction was filtered and evaporated in vacuo to give 1.43 g of the primary amine as an oil.

A solution of the crude primary amine (0.450 g) in THF (40 ml) was treated with $K_2CO_3$ (1.90 g, 13.7 mmol) and $H_2O$ (30 ml). The mixture was treated with di-tert-butyldicarbonate (1.50 g, 6.87 mmol) and the reaction

stirred at room temperature overnight. The organic solvent was evaporated in vacuo and the aqueous layer extracted with EtOAc (3 x 50 ml). The combined organic layers were dried over $Na_2SO_4$ and evaporated in vacuo to give 725 mg of an oily solid which was purified by radial chromatography ($SiO_2$; 50 % EtOAc in Hexanes) to give 550 mg of a white solid.
MS(FD), m/e 431 (M+).

| Anal Calcd for $C_{23}H_{33}N_3O_5$: | | | |
|---|---|---|---|
| Calculated | C 64.02, | H 7.71, | N 9.74; |
| Found | C 64.26, | H 7.72, | N 9.77. |

Part B Preparation of the title compound (52a):

A solution of (51a) (300 mg, 0.67 mmol) in THF (20 ml) was treated with LiOH (50 mg, 3.5 mmol) in $H_20$ (6 ml). The reaction was heated to mild reflux for 6 hours and was concentrated in vacuo. The residue was partitioned between $H_2O$ (50 ml) and EtOAc (50 ml). The organic layer was separated and was washed with 2 N aqueous NaOH (10 ml). The combined aqueous layers were acidified to ~ pH 5 with solid citric acid and were extracted with EtOAc (2 x 50 ml). The combined EtOAc extracts were dried over $Na_2SO_4$ and evaporated in vacuo to afford 295 mg of the acid as a white solid.

The acid (295 mg) was treated with 10 ml of a 10% anisole in TFA solution and the mixture stirred at room temperature for 3 hours. The reaction was concentrated in vacuo and the residue partitioned between 30 ml of $H_2O$ and 30 ml EtOAc. The aqueous layer was separated, washed with 30 ml EtOAc, acidified to pH 1 with 5 N aqueous HCl, and lyophilized to afford 210 mg of the title compound as the TFA salt.
MS(FD), m/e 317 (M+).

Assay Methods

The identification of compounds which are active platelet aggregation inhibitors (PAI) is made possible by the observation that compounds which block the binding of fibrinogen to the GPIIb-IIIa complex in vitro also are capable of inhibiting thrombin or ADP-induced aggregation of human platelets and the formation of platelet-thrombi in vivo. This observation provides the basis for obtaining potent PAI's by evaluating the ability of test materials to disrupt fibrinogen-GP IIb-IIIa interactions.

The following assay methods were used to evaluate the compounds of the invention.

No. 1 - The GPIIb-IIIa ELISA Assay:

In the following assay, GPIIb-IIIa is prepared in purified form, by a method such as described by Fitzgerald, L.A., *et al.*, Anal Biochem (1985) 151:159-177,(the disclosure of which is incorporated herein by reference). GPIIb-IIIa is coated onto microtiter plates. The coated support is then contacted with fibrinogen and with the test material and incubated for a sufficient time to permit maximal binding of fibrinogen to the immobilized GPIIb-IIIa. Fibrinogen is typically provided at a concentration of about 5-50 nM and the test material can, if desired, be added at a series of dilution. Typical incubations are 2-4 hr at 25°C, the time and temperature being interdependent.

After incubation, the solution containing the fibrinogen and test material is removed and the level of binding of fibrinogen measured by quantitating bound fibrinogen to GPIIb-IIIa. Any suitable means of detection may be used, but it is convenient to employ labeled fibrinogen, for example using biotinylated labels. Such methods are well known in the art.

Detailed Description of GPIIb-IIIa ELISA Assay

Purified platelet GPIIb-IIIa receptor was prepared as described by Fitzgerald, L.A., *et al.*, Anal Biochem (1985) 151:169-177. Vitronectin receptor was prepared as described by Smith, J.W., J. Biol Chem (1988) 263:18726-18731. After purification, the receptors were stored in 0.1% Triton X-100 at 0.1-1.0 mg/ml.

The receptors were coated to the wells of 96-well flat-bottom ELISA plates (Linbro EIA-Plus microtiter plate, Flow Laboratories) after diluting 1:200 with a solution of 20 mM Tris-HCl, 150 MM NaCl, 1 mM $CaCl_2$, pH 7.4, to reduce the Triton x-100 concentration to below its critical micellar concentration and adding an aliquot of

100 ul to each well. The wells were all allowed to incubate overnight at 4°C, and then aspirated to dryness. Additional sites were blocked by the addition of bovine serum albumin (BSA) at 35 mg/ml in the above buffer for 2 hours at 30°C to prevent nonspecific binding. The wells were then washed once with binding buffer (50 nM Tris-HCl, 100 mM Nacl 2 mM $CaCl_2$, 1 mg/ml BSA).

The corresponding ligands (fibrinogen, von Willebrand Factor, or vitronectin) were conjugated to biotin using commercially available reagents and standard protocols. The labeled ligands were added to the receptor-coated wells at final concentration of 10 nM (100 ul/well) and incubated for 3 hours at 25 °C in the presence or absence of the test samples. After incubation, the wells are aspirated to dryness and bound ligand is quantitated.

The bound protein is detected by the addition of antibiotin antibody conjugated to alkaline phosphatase followed by addition of substrate (p-nitrcphenyl phosphate), and determination of the optical density of each well at 405 nM. Decreased color development is observed in wells incubated with test samples which inhibit binding of ligand to receptor.

No. 2 - The Platelet Aggregation Assay (PRP):

In addition to the ELISA GPIIb-IIIa assay previously described the Aggregation-Human/PRP/ADP Assay is useful for evaluating therapeutic compounds.

Platelet-rich plasma was prepared from healthy human volunteers for use in determining inhibition of platelet aggregation by the compounds. Blood was collected via a 21 gauge butterfly cannula, using a two-syringe technique into 1/10 volume of 10% trisodium citrate.

Platelet-rich plasma was prepared at room temperature by centrifugation of the citrated whole blood at 100 x g for 15 minutes. The platelet rich plasma contained approximately 200-400,000 platelets/µl.

Platelet-poor plasma was prepared by centrifugation of citrated whole blood at 12,000 x g for 2 minutes.

Platelet aggregation was assayed in a 4-channel platelet aggregation profiler (PAP-4, Biodata, Hatboro, PA) according to the manufacturers directions. Inhibition of platelet aggregation was studied by adding varying amounts of test compounds to stirred human platelet-rich plasma. Specifically, the human platelet-rich plasma was incubated with test compounds for 1 minute at 37°C prior to the addition of a variety of aggregating agents most often ADP 5 µM, but also 1 µg/ml collagen, 1 µM U46619 or 1 µM platelet activating factor.

TABLE OF ASSAY TEST RESULTS

| Biological Data | | |
|---|---|---|
| Example # | ELISA $IC_{50}$ μm | PRP $IC_{50}$ μm |
| 9 | 4.45 | 40 |
| 10 | 68 | >>100 |
| 11 | >>100 | >>100 |
| 12 | 1.2 | 9.0 |
| 13 | 5.25 | 28 |
| 14 | 21.5 | >>100 |
| 15 | 12 | >>100 |
| 16 | 19 | >>100 |
| 17 | 0.21 | 2.7 |
| 18 | 0.33 | 5.8 |
| 19 | 0.63 | 30 |
| 20 | 0.8 | 70 |
| 21 | 0.38 | 2.5 |
| 22 | 0.04 | 0.27 |
| 23 | 0.74 | 9.0 |
| 25 | 0.13 | 0.8 |
| 26 | .7 | 2.7 |
| 27 | 41.33 | 100 |
| 28 | 42.66 | 100 |
| 29 | 1.4 | 5.8 |
| 30 | 3.0 | 8 |
| 31 | 0.32 | 3.3 |
| 32 | 0.01 | 0.23 |
| 33 | 2.6 | 5.0 |
| 34 | 0.03 | 0.3 |
| 35 | 3.4 | 100 |
| 36 | 4.23 | 25 |

Pharmaceutical Compositions

Pharmaceuticals containing compounds of the invention can be administered orally in the form of tablets, capsules, solutions, emulsions or suspensions, or rectally, for example in the form of suppositories, or as a spray. Administration can also take place parenterally, for example in the form of injectable solutions.

Tablets are prepared by mixing the Active Ingredient ("Active Ingredient" is a compound corresponding to formula I, of the invention) with pharmaceutically inert, inorganic or organic excipients. Examples of such excipients which can be used for tablets, are lactose, maize starch or derivatives thereof, talc, stearic acid or salts thereof. Examples of suitable excipients for soft gelatin capsules are vegetable oils, waxes, fats, semisolid and liquid polyols.

Suitable excipients for the preparation of solutions and syrups are water, polyols, sucrose, invert sugar and glucose, suitable

Suitable excipients for injectable solutions are water, alcohols, polyols, glycerol and vegetable oils.

These pharmaceutical products can additionally contain preservatives, solubilizers, stabilizers, wetting agents, emulsifiers, sweeteners, colorants, flavorings, buffers, coating agents and antioxidants.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use.

The Active Ingredient can also be made in micro-encapsulated form.

Exemplary formulations using the Active Ingredient are described below:

Formulation 1

Hard gelatin capsules are prepared using the following ingredients:

| | (mg/capsule) |
|---|---|
| Active Ingredient | 250.0 |
| Starch | 305.0 |
| Magnesium stearate | 5.0 |

The above ingredients are mixed and filled into hard gelatin capsules in 560 mg quantities.

Formulation 2

A tablet formula is prepared using the ingredients below:

| | (mg/tablet) |
|---|---|
| Active Ingredient | 250.0 |
| Cellulose, microcrystalline | 400.0 |
| Colloidal silicon dioxide | 10.0 |
| Stearic acid | 5.0 |

The components are blended and compressed to form tablets, each weighing 665 mg.

Formulation 3

A dry powder inhaler formulation is prepared containing the following components:

| | Weight % |
|---|---|
| Active Ingredient | 5 |
| Lactose | 95 |

The active mixture is mixed with the lactose and the mixture is added to a dry powder inhaling appliance.

Formulation 4

Tablets, each containing 60 mg of active ingredient, are prepared as follows:

| | (milligrams) |
|---|---|
| Active Ingredient | 60.0 |
| Starch | 45.0 |
| Microcrystalline cellulose | 35.0 |
| Polyvinylpyrrolidone (as 10% solution in water) | 4.0 |
| Sodium carboxymethyl starch | 4.5 |
| Magnesium stearate | 0.5 |
| Talc | 1.0 |
| Total | 150.0 |

The Active Ingredient, starch and cellulose are passed through a No. 20 mesh U.S. sieve and mixed thoroughly. The solution of polyvinylpyrrolidone is mixed with the resultant powders, which are then passed through a 16 mesh U.S. sieve. The granules ao produced are dried at 50-60°C and passed through a 16 mesh U.S. Sieve. The sodium carboxymethyl starch, magnesium stearate, and talc, previously passed through a No. 30 mesh U.S. Sieve, are then added to the granules which, after mixing, are compressed on a tablet machine to yield tablets each weighing 150mg.

Formulation 5

Capsules, each containing 80 mg of medicament are made as follows:

| | (milligrams) |
|---|---|
| Active Ingredient | 80.0 |
| Starch | 109.0 |
| Magnesium Stearate | 1.0 |
| Total | 190.0 |

The active ingredient, cellulose, starch, and magnesium stearate are blended, passed through a No. 20 mesh U.S. sieve, and filled into hard gelatin capsules in 190 mg quantities.

Formulation 6

Suppositories, each containing 225 mg of active ingredient are made as follows:

| | |
|---|---|
| Active Ingredient | 225 mg |
| Saturated fatty acid glycerides to | 2000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

Formulation 7

Suspensions, each containing 50 mg of medicament per 5.0 mL dose are made as-follows:

| | |
|---|---|
| Active Ingredient | 50.0 mg |
| Xanthan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |
| Flavor | q.v. |
| Color | q.v. |
| Purifed water to | 5.0 mL |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

Formulation 8

Capsules, each containing 150 mg of medicament, are made as follows:

| | (milligrams) |
|---|---|
| Active Ingredient | 150.0 |
| Starch | 407.0 |
| Magnesium stearate | 3.0 |
| Total | 560.0 |

The Active Ingredient, cellulose, starch, and magnesium stearate are blended, passed through a No. 20 mesh U.S. sieve, and filled into hard gelatin capsules in 560 mg quantities.

Method of Treatment

This invention provides a method of preventing or treating thrombosis in mammals, especially humans, which method comprises administering to the human or mammal a therapeutically effective amount of the compounds of this invention. The platelet aggregation Inhibitors ("PAIs") of the invention are useful therapeutically to prevent thrombus formation. Indications appropriate to such treatment include, without limitation, atherosclerosis and arteriosclerosis, acute myocardial infarction, chronic unstable angina, transient ischemic attacks and strokes, peripheral vascular disease, arterial thrombosis, preeclampsia, embolism, restenosis and/or thrombosis following angioplasty, carotid endarterectomy, anastomosis of vascular grafts, and chronic cardiovascular devices (e.g., in-dwelling catheters or shunts "extracorporeal circulating devices"). These syndromes represent a variety of stenotic and occlusive vascular disorders thought to be initiated by platelet activation on vessel walls.

The PAIs may be used for prevention or abortion of arterial thrombus formation, in unstable angina and arterial emboli or thrombosis, as well as treatment or prevention of myocardial infarction (MI) and mural thrombus formation post MI. For brain-related disorders, treatment or prevention of transient ischemic attack and treatment of thrombotic stroke or stroke-in-evolution are included.

The PAIs may also be used for prevention of platelet aggregation, embolization, or consumption in extracorporeal circulations, including improving renal dialysis, cardiopulmonary bypasses, hemoperfusions, and plasmapheresis.

PAIs prevent platelet aggregation, embolization, or consumption associated with intravascular devices, and administration results in improved utility of intraaortic balloon pumps, ventricular assist devices, and ar-

terial catheters.

The PAIs will also be useful in treatment or prevention of venous thrombosis as in deep venous thrombosis, IVC, renal vein or portal vein thrombosis, and pulmonary venous thrombosis.

Various disorders involving platelet consumption, such as thrombotic thrombocytopenic purpura are also treatable.

In addition, the PAIs of the present invention may be used in numerous nontherapeutic applications where inhibiting platelet aggregation is desired. For example, improved platelet and whole blood storage can be obtained by adding sufficient quantities of the compounds, the amount of which will vary depending upon the length of proposed storage time, the conditions of storage, the ultimate use of the stored material, etc.

Preferably, the compounds of this invention are administered in the form of a pharmaceutical formulation. Thus, the compounds of this invention may be administered orally, parenterally, topically, rectally and etc., in, appropriate dosage units, as desired.

The term, "parenteral" as used herein includes subcutaneous, intravenous, intraarterial, injection or infusion techniques, without limitation. The term, "topically" encompasses administration rectally and by inhalation spray, as well as the more common routes of the skin and the mucous membranes of the mouth and nose.

Actual dosage levels of active ingredients in the pharmaceutical compositions of this invention may be varied so as to administer an amount of the active compound(s) that is effective to achieve the desired therapeutic response for a particular patient. A typical daily dose will contain a non-toxic dosage level of from about 0.01 mg/kg to about 50 mg/kg of body weight of an active compound (of formula I) of this invention. Preferred daily doses generally will be from about 0.05 mg/kg to about 20 mg/kg and ideally from about 0.1 mg/kg to about 10 mg./kg.

The active ingredient in a pharmaceutical formulation of this invention comprises from 0.1% to 99.9% by weight of the fomulation.

The selected dosage level will depend upon the activity of the particular compound, the route of administration, the severity of the condition being treated, and the condition and prior medical history of the patient being treated. However, it is within the skill of the art to start doses of the compound at levels lower than required to achieve the desired therapeutic effect and to gradually increase the dosage until the desired effect is achieved. If desired, the effective daily dose may be divided into multiple doses for purposes of administration, e.g., two to four separate doses per day. It will be understood, however, that the specific dose level for any particular patient will depend upon a variety of factors including the body weight, general health, diet, time and route of administration, combination with other drugs and the severity of the particular disease being treated.

Many modifications and variations of this invention may be made without departing from its scope, as is apparent to those skilled in the art. The specific embodiments described herein are offered by way of example only, and the invention is to be limited only by the terms of the appended claims.

## Claims

1. A 5,6 bicyclic compound which inhibits platelet aggregation in mammals, said compound represented by the formula (I), and all pharmaceutically acceptable salts, solvates, and prodrug derivatives thereof:

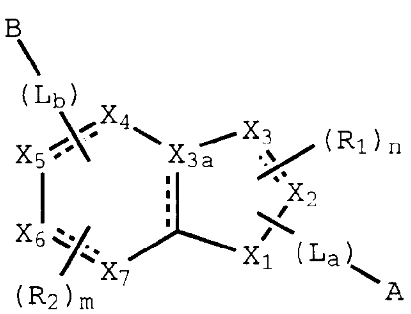

(I)

wherein:

$X_1$, $X_2$, and $X_3$ of the 5 membered ring are independently selected from carbon, nitrogen, sulfur, and oxygen, with the proviso that at least one of $X_1$, $X_2$, and $X_3$ is carbon;

$X_4$, $X_5$, $X_6$, and $X_7$ are independently selected from carbon, nitrogen, sulfur, and oxygen, with the proviso that at least two of $X_4$, $X_5$, $X_6$, and $X_7$ are carbon;

$X_{3a}$, the bridging atom, is selected from the group consisting of carbon and nitrogen;

B is a basic group linked to the six membered ring by linking group -($L_b$)-, where -($L_b$)- is;

(i) a bond, or

(ii) a divalent substituted or unsubstituted chain of from 1 to 15 atoms;

A is an acidic group linked to the five membered ring by linking group -($L_a$)-, where -($L_a$)- is;

(i) a bond, or

(ii) a divalent substituted or unsubstituted chain of from 1 to 15 atoms;

n is an integer selected from 1 to 5;

$(R_1)_n$ are organic radicals attached to one or more of the atoms $X_1$, $X_2$, and $X_3$ of the five membered ring and where $R_1$ are independently selected from A, A-($L_a$)-, hydrogen, alkyl, halosubstituted alkyl, hydroxylalkyl, alkenyl, alkynyl, cycloalkyl, aryl, aryloxy, aralkyl, hydroxy, alkoxy, aralkoxy, carbamyl, carboxy, acyl, cyano, halo, nitro, sulfo, and when any of $X_1$, $X_2$, and $X_3$ have two sites for substitution, then $R_1$ may also be =O;

m is an integer selected from 1 to 7;

$(R_2)_m$ are organic radicals attached to one or more of the atoms $X_4$, $X_5$, $X_6$, and $X_7$ of the six membered ring and where $R_2$ are independently selected from B, B-($L_b$)-, hydrogen, alkyl, hydroxyalkyl, halosubstituted alkyl, alkenyl, alkynyl, cycloalkyl, aryl, aryloxy, aralkyl, hydroxy, alkoxy, aralkoxy, carbamyl, amino, substituted amino, acyl, cyano, halo, nitro, sulfo, and when any of $X_4$, $X_6$, $X_6$, and $X_7$ have two sites for substitution, then $R_2$ may also be =O.

**2.** The compound of Claim 1 wherein the acidic group A is selected from the group consisting of:

- 5-tetrazolyl,
- $SO_3H$,
- carboxyl,

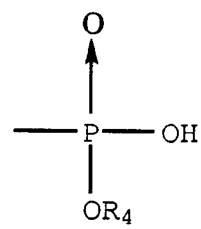
,

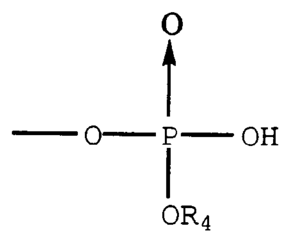
,

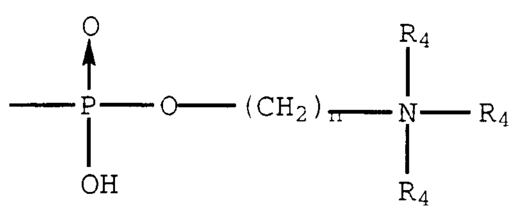
,

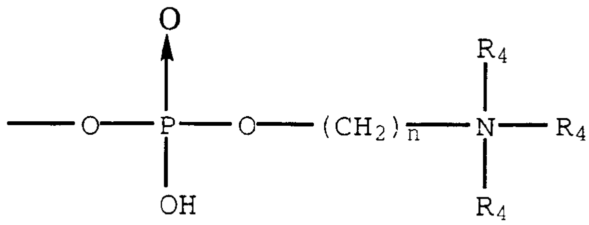,

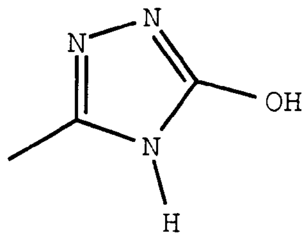,

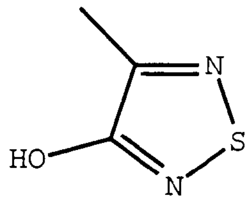;

and where n is an integer from 1 to 12 and $R_4$ is independently selected from:

—H ,

—($C_1$-$C_9$ alkyl) ,

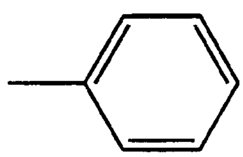,

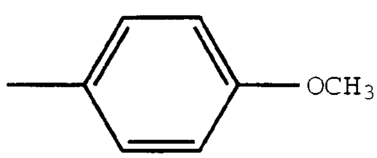,

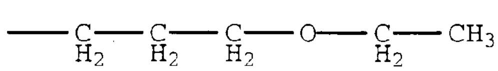,

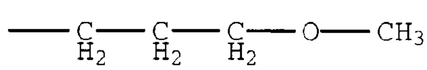,

—CONR' ,

—(pyridinyl) ,

and

—$SO_2R'$ ,

and where R' is H or $C_1$-$C_{12}$ alkyl.

**3.** The compound of Claim 1 wherein the acidic carboxyl group with linking group A-($L_a$)- is selected from groups represented by the formulae:

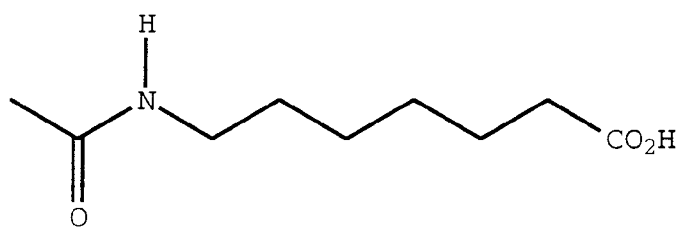

,

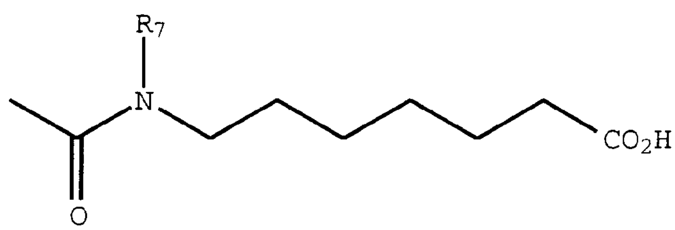

,

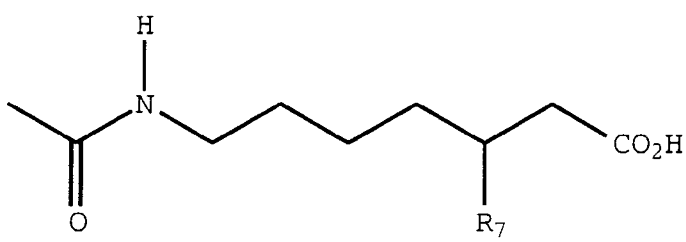

,

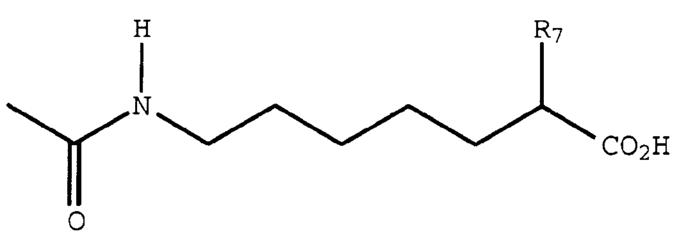

,

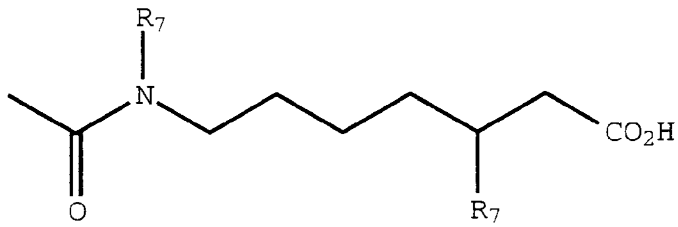

,

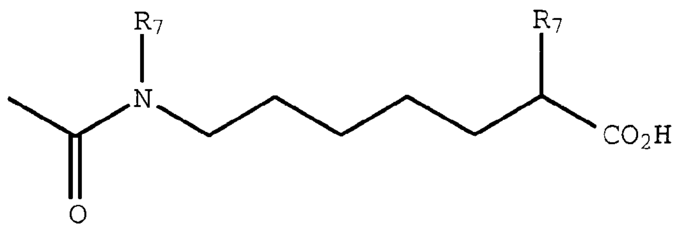

,

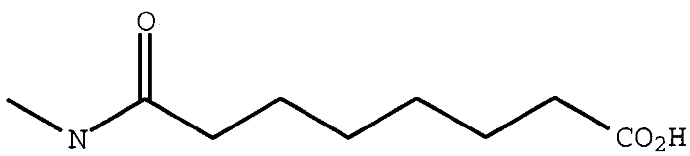

,

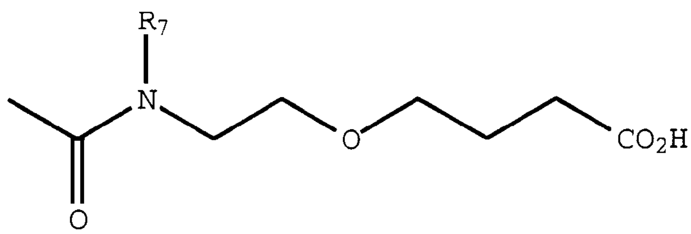

,

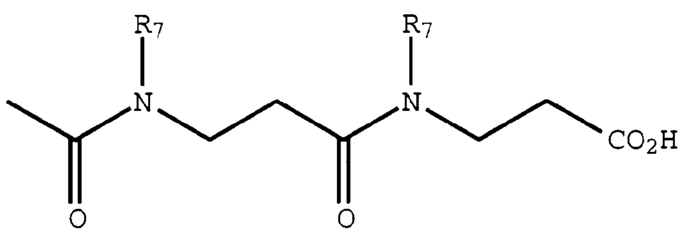

,

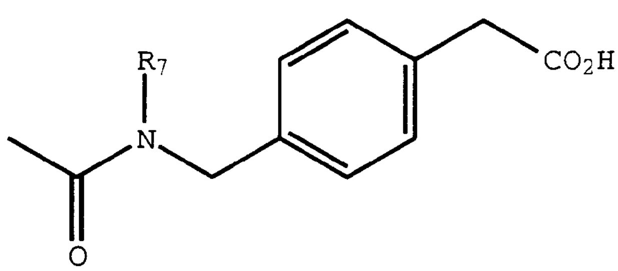

,

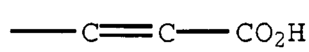

,

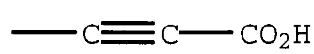

,

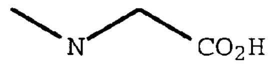

,

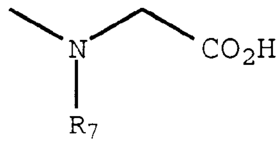

,

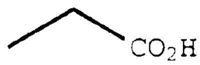

,

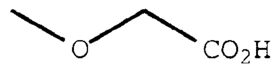

,

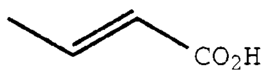

,

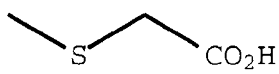

,

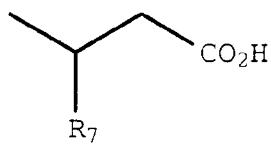

,

and where $R_7$ is selected from hydrogen and $C_1$-$C_{12}$ alkyl, phenyl, $C_1$-$C_{12}$ alkenyl, $C_1$-$C_{12}$ alkynyl, and $C_4$-$C_8$ cycloalkyl.

4. The compound of Claim 1 wherein the basic group with linking group B is selected from the substituents selected from the group of the following twelve formulae:

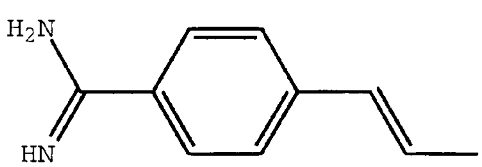

,

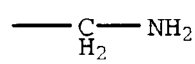

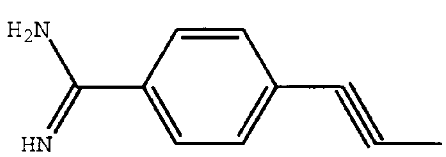

,

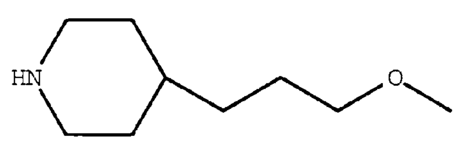

,

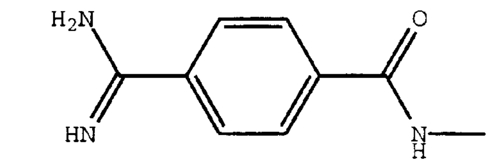 , 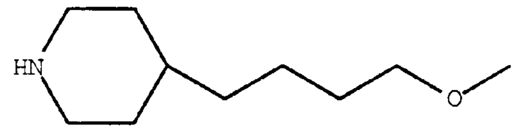 ,

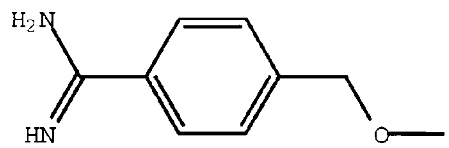 , 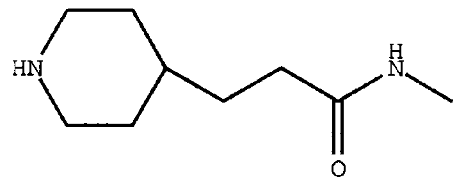 ,

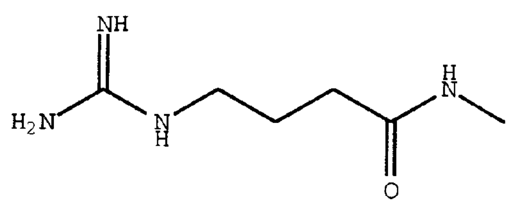 , 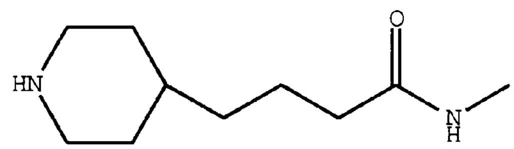 ,

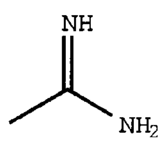 , and 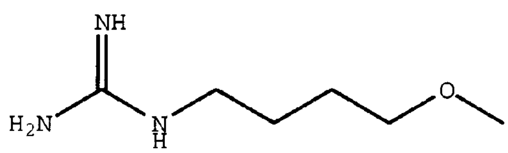 .

5. The compound of Claim 1 where the sum of the chain atoms in -(Lb)- and -(La)- of formula II is from 6 to 16.

6. A 5,6 bicyclic indole-type compound represented by the formula (III), and all pharmaceutically acceptable salts, solvates, and prodrug derivatives thereof:

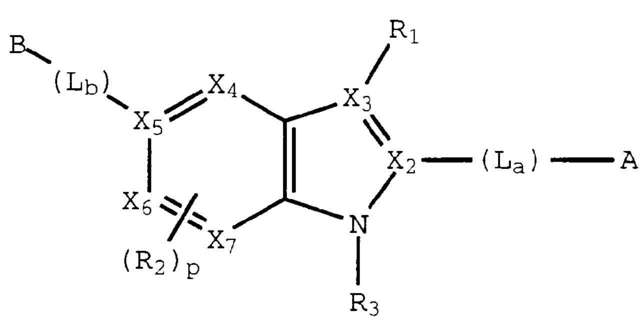

(III)

wherein:

$X_2$ and $X_5$ are carbon;

$X_3$ is independently selected from carbon and nitrogen;

$X_4$, $X_6$, and $X_7$ are independently selected from carbon and nitrogen, with the proviso that at least two of $X_4$, $X_6$, and $X_7$ are carbon;

B is a basic group linked to the six membered ring by linking group -($L_b$)-, where -($L_b$)- is;

(i) a bond, or

(ii) a divalent substituted or unsubstituted chain of from 1 to 15 atoms;

A is an acidic group linked to the five membered ring by linking group -($L_a$)-, where -($L_a$)- is;

(i) a bond, or

(ii) a divalent substituted or unsubstituted chain of from 1 to 15 atoms;

$R_1$ is selected from A, A-($L_a$)-, hydrogen, alkyl, hydroxyalkyl, halosubstituted alkyl, alkenyl, alkynyl, cycloalkyl, aryl, aryloxy, aralkyl, hydroxy, alkoxy, aralkoxy, carbamyl, carboxy, acyl, cyano, halo, nitro, sulfo;

p is an integer selected from 1 to 3;

($R_2$)$_p$ are organic radicals attached to one or more of the atoms $X_4$, $X_6$, and $X_7$ of the six membered ring and where $R_2$ are independently selected from B, B-($L_b$)-, hydrogen, alkyl, halosubstituted alkyl, hydroxyalkyl, alkenyl, alkynyl, cycloalkyl, aryl, aryloxy, aralkyl, hydroxy, alkoxy, aralkoxy, carbamyl, amino, substituted amino, acyl, cyano, halo, nitro, and sulfo; and

$R_3$ is hydrogen, halo, $C_1$-$C_4$ alkyl, $C_2$-$C_4$ alkenyl, $C_2$-$C_4$ alkynyl, $C_1$-$C_4$ alkoxy, $C_1$-$C_4$ haloalkyl, aralkyl, alkaryl, hydroxyalkyl and acyl.

**7.** A 5,6 bicyclic benzofuran-type compound represented by the formula (V), and all pharmaceutically acceptable salts, solvates, and prodrug derivatives thereof:

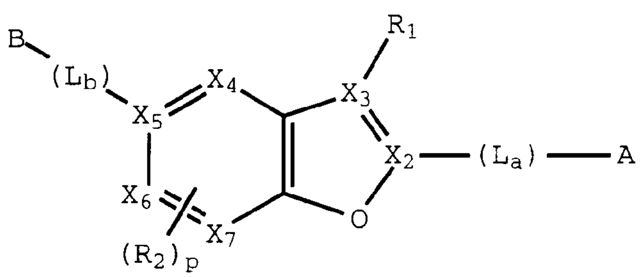

(V)

wherein:

$X_2$ and $X_5$ are carbon;

$X_3$ is independently selected from carbon and nitrogen;

$X_4$, $X_6$, and $X_7$ are independently selected from carbon and nitrogen, with the proviso that at least two of $X_4$, $X_6$, and $X_7$ are carbon;

B is a basic group linked to the six membered ring by linking group -($L_b$)-, where -($L_b$)- is;

(i) a bond, or

(ii) a divalent substituted or unsubstituted chain of from 1 to 15 atoms;

A is an acidic group linked to the five membered ring by linking group -($L_a$)-, where -($L_a$)- is;

(i) a bond, or

(ii) a divalent substituted or unsubstituted chain of from 1 to 15 atoms;

$R_1$ is selected from A, A-($L_a$)-, hydrogen, alkyl, hydroxyalkyl, halosubstituted alkyl, alkenyl, alkynyl, cycloalkyl, aryl, aryloxy, aralkyl, hydroxy, alkoxy, aralkoxy, carbamyl, carboxy, acyl, cyano, halo, nitro, sulfo;

p is an integer selected from 1 to 3;

($R_2$)$_p$ are organic radicals attached to one or more of the atoms $X_4$, $X_6$, and $X_7$ of the six membered ring and where $R_2$ are independently selected from B, B-($L_b$)-, hydrogen, alkyl, halosubstituted alkyl, hydroxyalkyl, alkenyl, alkynyl, cycloalkyl, aryl, aryloxy, aralkyl, hydroxy, alkoxy, aralkoxy, carbamyl, amino, substituted amino, acyl, cyano, halo, nitro, and sulfo.

**8.** A 5,6 bicyclic benzothiophene-type compound represented by the formula (VI), and all pharmaceutically acceptable salts, solvates, and prodrug derivatives thereof:

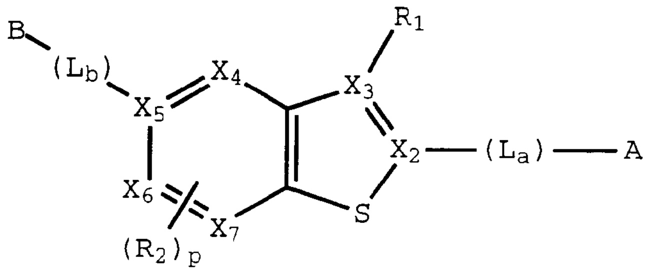

(VI)

wherein:

$X_2$ and $X_5$ are carbon;

$X_3$ is independently selected from carbon and nitrogen;

$X_4$, $X_6$, and $X_7$ are independently selected from carbon and nitrogen, with the proviso that at least two of $X_4$, $X_6$, and $X_7$ are carbon;

B is a basic group linked to the six membered ring by linking group $-(L_b)-$, where $-(L_b)-$ is;

(i) a bond, or

(ii) a divalent substituted or unsubstituted chain of from 1 to 15 atoms;

A is an acidic group linked to the five membered ring by linking group $-(L_a)-$, where $-(L_a)-$ is;

(i) a bond, or

(ii) a divalent substituted or unsubstituted chain of from 1 to 15 atoms;

$R_1$ is selected from A, A-$(L_a)$-, hydrogen, alkyl, hydroxyalkyl, halosubstituted alkyl, alkenyl, alkynyl, cycloalkyl, aryl, aryloxy, aralkyl, hydroxy, alkoxy, aralkoxy, carbamyl, carboxy, acyl, cyano, halo, nitro, sulfo;

p is an integer selected from 1 to 3;

$(R_2)_p$ are organic radicals attached to one or more of the atoms $X_4$, $X_6$, and $X_7$ of the six membered ring and where $R_2$ are independently selected from B, B-$(L_b)$-, hydrogen, alkyl, halosubstituted alkyl, hydroxyalkyl, alkenyl, alkynyl, cycloalkyl, aryl, aryloxy, aralkyl, hydroxy, alkoxy, aralkoxy, carbamyl, amino, substituted amino, acyl, cyano, halo, nitro, and sulfo.

**9.** A pharmaceutical formulation comprising as an active ingredient, a compound of Claim 1 together with a pharmaceutically acceptable carrier or diluent therefor.

**10.** A method of effecting inhibition of platelet aggregation which comprises administering to a mammal an effective amount of the compound of Claim 1.